# EUROPÄISCHE PATENTSCHRIFT

(11) **EP 1 723 234 B1**
(45) Veröffentlichungstag und Bekanntmachung des Hinweises auf die Patenterteilung: **16.06.2010**
(21) Anmeldenummer: 05707623.4
(22) Anmeldetag: 24.02.2005
(51) Int. Cl.: C12N 15/11, A61K 31/7088, C12Q 1/68

(54) **VGLUT-SPEZIFISCHE DSRNA-VERBINDUNGEN**
VGLUT-SPECIFIC DSRNA COMPOUNDS
COMPOSES ARN DOUBLE BRIN SPECIFIQUES DE VGLUT

(30) Priorität: 10.03.2004 DE 102004011687
(43) Veröffentlichungstag der Anmeldung: 22.11.2006
(73) Patentinhaber: Grünenthal GmbH, 52078 Aachen (DE)
(72) Erfinder: GILLEN, Clemens, 52076 Aachen (DE); BAHRENBERG, Gregor, 52074 Aachen (DE); CHRISTOPH, Thomas, 52080 Aachen (DE); WEIHE, Eberhard, 35037 Marburg (DE); SCHÄFER, K.-H., Martin, 35041 Marburg (DE); BENDER, Florian, 97072 Würzburg (DE)
(74) Vertreter: Hiebl, Inge Elisabeth
(86) Internationale Anmeldenummer: PCT/EP2005/001970
(87) Internationale Veröffentlichungsnummer: WO 2005/090571

(56) Entgegenhaltungen:
- WO-A-02/08384
- AGAMI R.: "RNAi and related mechanisms and their potential use for therapy" CURRENT OPINION IN CHEMICAL BIOLOGY, Bd. 6, Nr. 6, Dezember 2002 (2002-12), Seiten 829-834, XP002239988 ISSN: 1367-5931
- CAPLEN N.J. AND SPYRO M.: "Short interfering RNA (siRNA)-mediated RNA interference (RNAi) in human cells" ANNALS OF THE NEW YORK ACADEMY OF SCIENCES, Bd. 1002, Dezember 2003 (2003-12), Seiten 56-62, XP009048218 ISSN: 0077-8923
- SHUEY D.J. ET AL.: "RNAi: gene-silencing in therapeutic intervention" DRUG DISCOVERY TODAY, Bd. 7, Nr. 20, 15. Oktober 2002 (2002-10-15), Seiten 1040-1046, XP002331644
- SCHERER L. AND ROSSI J.J.: "Recent applications of RNA interference (RNAi) in mammalian systems" LETTERS IN PEPTIDE SCIENCE, Bd. 10, Nr. 3-4, 2003, Seiten 255-267, XP002331645

### Sequenzliste

<110> Grünenthal GmbH
<120> VGLUT-spezifische dsRNA-Verbindungen
<130> GR01P011WO
<160> 112
<170> PatentIn version 3.1
<210> 1
   <211> 17
   <212> RNA
   <213> Artificial Sequence
<220>
   <223> Erfindungsgemaesse dsRNA (S. 6 und 9 der Beschreibung). Allgemeine Struktur: N(17-25), bevorzugt N(19-25), staerker bevorzugt N(19-24), noch staerker bevorzugt N(21-23)
<220>
   <221> misc_feature
   <222> (1)..(17)
   <223> n = a, t/u, g, c, unbekannt oder sonstige
<400> 1
   nnnnnnnnnn nnnnnnn 17
<210> 2
   <211> 18
   <212> RNA
   <213> Artificial Sequence
<220>
   <223> Erfindungsgemaesse dsRNA (S. 6 und 9 der Beschreibung). Allgemeine Struktur: N(17-25), bevorzugt N(19-25), staerker bevorzugt N(19-24), noch staerker bevorzugt N(21-23)
<220>
   <221> misc_feature
   <222> (1)..(18)
   <223> n = a, t/u, g, c, unbekannt oder sonstige
<400> 2
   nnnnnnnnnn nnnnnnnn 18
<210> 3
   <211> 19
   <212> RNA
   <213> Artificial Sequence
<220>
   <223> Erfindungsgemaesse dsRNA (S. 6 und 9 der Beschreibung).
<110> Grünenthal GmbH
<120> VGLUT-spezifische dsRNA-Verbindungen
<130> GR01P011WO
<160> 112
<170> PatentIn version 3.1
<210> 1
   <211> 17
   <212> RNA
   <213> Artificial Sequence
<220>
   <223> Erfindungsgemaesse dsRNA (S. 6 und 9 der Beschreibung). Allgemeine Struktur: N(17-25), bevorzugt N(19-25), staerker bevorzugt N(19-24), noch staerker bevorzugt N(21-23)
<220>
   <221> misc_feature
   <222> (1)..(17)
   <223> n = a, t/u, g, c, unbekannt oder sonstige
<400> 1
   nnnnnnnnnn nnnnnnn 17
<210> 2
   <211> 18
   <212> RNA
   <213> Artificial Sequence
<220>
   <223> Erfindungsgemaesse dsRNA (S. 6 und 9 der Beschreibung). Allgemeine Struktur: N(17-25), bevorzugt N(19-25), staerker bevorzugt N(19-24), noch staerker bevorzugt N(21-23)
<220>
   <221> misc_feature
   <222> (1)..(18)
   <223> n = a, t/u, g, c, unbekannt oder sonstige
<400> 2
   nnnnnnnnnn nnnnnnnn 18
<210> 3
   <211> 19
   <212> RNA
   <213> Artificial Sequence
<220>
   <223> Erfindungsgemaesse dsRNA (S. 6 und 9 der Beschreibung). Allgemeine Struktur: N(17-25), bevorzugt N (19-25), staerker bevorzugt N(19-24), noch staerker bevorzugt N(21-23)
<220>
   <221> misc_feature
   <222> (1)..(19)
   <223> n = a, t/u, g, c, unbekannt oder sonstige
<400> 3
   nnnnnnnnnn nnnnnnnnn 19
<210> 4
   <211> 20
   <212> RNA
   <213> Artificial Sequence
<220>
   <223> Erfindungsgemaesse dsRNA (S. 6 und 9 der Beschreibung). Allgemeine Struktur: N(17-25), bevorzugt N(L9-25), staerker bevorzugt N(19-24), noch staerker bevorzugt N(21-23)
<220>
   <221> misc_feature
   <222> (1)..(20)
   <223> n = a, t/u, g, c, unbekannt oder sonstige
<400> 4
   nnnnnnnnnn nnnnnnnnnn 20
<210> 5
   <211> 21
   <212> RNA
   <213> Artificial Sequence
<220>
   <223> Erfindungsgemaesse dsRNA (S. 6 und 9 der Beschreibung). Allgemeine Struktur: N(17-25), bevorzugt N(19-25), staerker bevorzugt N(19-24), noch staerker bevorzugt N(21-23)
<220>
   <221> misc_feature
   <222> (1)..(21)
   <223> n = a, t/u, g, c, unbekannt oder sonstige
<400> 5
   nnnnnnnnnn nnnnnnnnnn n 21
<210> 6
   <211> 22
   <212> RNA
   <213> Artificial Sequence
<220>
   <223> Erfindungsgemaesse dsRNA (S. 6 und 9 der Beschreibung). Allgemeine Struktur: N(17-25), bevorzugt N(19-25), staerker bevorzugt N(19-24), noch staerker bevorzugt N (21-23)
<220>
   <221> misc_feature
   <222> (1)..(22)
   <223> n = a, t/u, g, c, unbekannt oder sonstige
<400> 6
   nnnnnnnnnn nnnnnnnnnn nn 22
<210> 7
   <211> 23
   <212> RNA
   <213> Artificial Sequence
<220>
   <223> Erfindungsgemaesse dsRNA (S. 6 und 9 der Beschreibung). Allgemeine Struktur: N(17-25), bevor zugt N(19-25), staerker bevorzugt N(19-24), noch staerker bevorzugt N(21-23)
<220>
   <221> misc_feature
   <222> (1)..(23)
   <223> n = a, t/u, g, c, unbekannt oder sonstige
<400> 7
   nnnnnnnnnn nnnnnnnnnn nnn 23
<210> 8
   <211> 24
   <212> RNA
   <213> Artificial Sequence
<220>
   <223> Erfindungsgemaesse dsRNA (S. 6 und 9 der Beschreibung). Allgemeine Struktur: N(17-25), bevorzugt N(19-25), staerker bevorzugt N(19-24), noch staerker bevorzugt N(21-23)
<220>
   <221> misc_feature
   <222> (1)..(24)
   <223> n = a, t/u, g, c, unbekannt oder sonstige
<400> 8
   nnnnnnnnnn nnnnnnnnnn nnnn 24
<210> 9
   <211> 25
   <212> RNA
   <213> Artificial Sequence
<220>
   <223> Erfindungsgemaesse dsRNA (S. 6 und 9 der Beschreibung). Allgemeine Struktur: N(17-25), bevorzugt N(19-25), staerker bevorzugt N(19-24), noch staerker bevorzugt N(21-23)
<220>
   <221> misc_feature
   <222> (1) .. (25)
   <223> n = a, t/u, g, c, unbekannt oder sonstige
<400> 9
   nnnnnnnnnn nnnnnnnnnn nnnnn 25
<210> 10
   <211> 23
   <212> DNA
   <213> Artificial Sequence
<220>
   <223> Sequenzmotiv einer bevorzugten erfindungsgemaessen VGLUT-dsRNA (S. 9 der Beschreibung).
   Das Sequenzmotiv lautet AAN(19)TT
<220>
   <221> misc_feature
   <222> (3) .. (21)
   <223> n = a, t/u, g, c, unbekannt oder sonstige
<400> 10
   aannnnnnnn nnnnnnnnnn ntt 23
<210> 11
   <211> 23
   <212> DNA
   <213> Artificial Sequence
<220>
   <223> Sequenzmotiv einer bevorzugten erfindungsgemaessen VGLUT-dsRNA (S. 9 der Beschreibung).
   Das Sequenzmotiv lautet NAN(19)NN.
<220>
   <221> misc_feature
   <222> (1)..(5)
   <223> n = a, t/u, g, c, unbekannt oder sonstige
<400> 11
   nannnnnnnn nnnnnnnnnn nnn 23
<210> 12
   <211> 23
   <212> DNA
   <213> Artificial Sequence
<220>
   <223> Sequenzmotiv einer bevorzugten erfindungsgemaessen VGLUT-dsRNA (S. 9 der Beschreibung).
   Das Sequenzmotiv lautet NARN(17)YNN.
<220>
   <221> misc_feature
   <222> (1)..(23)
   <223> n = a, t/u, g, c, unbekannt oder sonstige; y = c oder t; r = a oder g
<400> 12
   narnnnnnnn nnnnnnnnnn ynn 23
<210> 13
   <211> 23
   <212> DNA
   <213> Artificial Sequence
<220>
   <223> Sequenzmotiv einer bevorzugten erfindungsgemaessen VGLUT-dsRNA (S. 9 der Beschreibung).
   Das Sequenzmotiv lautet NANN(17)YNN.
<220>
   <221> misc_feature
   <222> (1)..(23)
   <223> n = a, t/u, g, c, unbekannt oder sonstige; y = c oder t.
<400> 13
   nannnnnnnn nnnnnnnnnn ynn 23
<210> 14
   <211> 21
   <212> DNA
   <213> Homo sapiens
<220>
   <221> misc_feature
   <222> (1)..(21)
   <223> Eine DNA-Zielsequenz von VGLUT1 (Fig. 26).
<400> 14
   aacgtgcgca agttgatgaa c 21
<210> 15
   <211> 21
   <212> DNA
   <213> Homo sapiens
<220>
   <221> misc_feature
   <222> (1) .. (21)
   <223> Eine DNA-Zielsequenz von VGLUT1 (Fig. 26).
<400> 15
   aagttgatga actgcggagg c 21
<210> 16
   <211> 21
   <212> DNA
   <213> Homo sapiens
<220>
   <221> misc_feature
   <222> (1)..(21)
   <223> Eine DNA-Zielsequenz von VGLUT2 (Fig. 26).
<400> 16
   aatgccttta gctggcattc t 21
<210> 17
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 17
   aatggtctgg tacatgtttt g 21
<210> 18
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 18
   aaagtcctgc aaagcatcct a 21
<210> 19
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 19
   aagtcctgca aagcatccta c 21
<210> 20
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 20
   aagaacgtag gtacatagaa g 21
<210> 21
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 21
   aattgttgca aacttctgca g 21
<210> 22
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 22
   aaattagcaa ggttggtatg c 21
<210> 23
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 23
   aattagcaag gttggtatgc t 21
<210> 24
   <211> 21
   <212> DNA ,
   <213> Homo sapiens
<220>
   <221> misc_feature
   <222> (1)..(21)
   <223> Eine der DNA-Zielsequenzen von VGLUT2 (Fig. 26).
<400> 24
   aaggttggta tgctatctgc t 21
<210> 25
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 25
   aagcaagcag attctttcaa c 21
<210> 26
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 26
   aaccacttgg atatcgctcc a 21
<210> 27
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 27
   aa tgggcatt tcgaatggtg t 21
<210> 28
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 28
   aataagtcac gtgaagagtg g 21
<210> 29
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 29
   aagtcacgt g aagagtggca g 21
<210> 30
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 30
   aagagtggca gtatgtcttc c 21
<210> 31
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 31
   aatatttgcc tcaggagaga a 21
<210> 32
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 32
   aagtcttatg gtgccacaac a 21
<210> 33
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 33
   aatggaggtt ggcctagtgg t 21
<210> 34
   <211> 21
   <212> DNA
   <213> Homo sapiens
<220>
   <221> misc_feature
   <222> (1)..(21)
   <223> Eine der DNA-Zielsequenzen von VGLUT2 (Fig. 26).
<400> 34
   aagactcaca tagctataag g 21
<210> 35
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 35
   aatcttggag ttgccattgt g 21
<210> 36
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 36
   aaccggaaat tcagacagca c 21
<210> 37
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 37
   aaacagtggg ccttatccat g 21
<210> 38
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 38
   aattccaggt ggtttcattt c 21
<210> 39
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 39
   aacatcgact ctgaacatgt t 21
<210> 40
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 40
   aaggtttagt ggagggtgtg a 21
<210> 41
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 41
   aagaggtctt tggatttgca a 21
<210> 42
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 42
   aataagtaag gtgggtctct t 21
<210> 43
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 43
   aagtaaggtg ggtctcttgt c 21
<210> 44
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 44
   aaggtgggtc tcttgtcagc a 21
<210> 45
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 45
   aatcgttgta cctattggag g 21
<210> 46
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 46
   aagacccgtg aagaatggca g 21
<210> 47
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 47
   aagaatggca gaatgtgttc c 21
<210> 48
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 48
   aatcattgac caggacgaat t 21
<210> 49
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 49
   aactcaacca tgagagtttt g 21
<210> 50
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 50
   aaagaagatg tcttatggag c 21
<210> 51
   <211> 21
   <212> DNA
   <213> Homo sapiens
<220>
   <221> misc_feature
   <222> (1)..(21)
   <223> Eine der DNA-Zielsequenzen von VGLUT3 (Fig. 26).
<400> 51
   aagagctgac atcctaccag a 21
<210> 52
   <211> 17
   <212> DNA
   <213> Artificial Sequence
<220>
   <223> Sequenzfolge einer dsRNA, deren Zielsequenz am 5'-Ende die Nukleotidfolge AA aufweist (S.12 der Beschreibung).
   Sequenzmotiv 5'AAN(15-23)
<220>
   <221> misc_feature
   <222> (3)..(17)
   <223> n = a, t/u, g, c, unbekannt oder sonstige.
<400> 52
   aannnnnnnn nnnnnnn 17
<210> 53
   <211> 18
   <212> DNA
   <213> Artificial Sequence
<220>
   <223> Sequenzfolge einer dsRNA, deren Zielsequenz am 5'-Ende die Nukleotidfolge AA aufweist (S .12 der Beschreibung).
   Sequenzmotiv 5'AAN(15-23)
<220>
   <221> misc_feature
   <222> (3)..(18)
   <223> n = a, t/u, g, c, unbekannt oder sonstige.
<400> 53
   aannnnnnnn nnnnnnnn 18
<210> 54
   <211> 19
   <212> DNA
   <213> Artificial Sequence
<220>
   <223> Sequenzfolge einer dsRNA, deren Zielsequenz am 5'-Ende die Nukleotidfolge AA aufweist (S. 12 der Beschreibung).
   Sequenzmotiv 5'AAN(15-23)
<220>
   <221> misc_feature
   <222> (3)..(19)
   <223> n = a, t/u, g, c, unbekannt oder sonstige.
<400> 54
   aannnnnnnn nnnnnnnnn 19
<210> 55
   <211> 20
   <212> DNA
   <213> Artificial Sequence
<220>
   <223> Sequenzfolge einer dsRNA, deren Zielsequenz am 5'-Ende die Nukleotidfolge AA aufweist (S.12 der Beschreibung).
   Sequenzmotiv 5'AAN(15-23)
<220>
   <221> misc_feature
   <222> (3)..(20)
   <223> n = a, t/u, g, c, unbekannt oder sonstige
<400> 55
   aannnnnnnn nnnnnnnnnn 20
<210> 56
   <211> 21
   <2127> DNA
   <213> Artificial Sequence
<220>
   <223> Sequenzfolge einer dsRNA, deren Zielsequenz am 5'-Ende die Nukleotidfolge AA aufweist (S.12 der Beschreibung).
   Sequenzmotiv 5'AAN (15-23)
<220>
   <221> misc_feature
   <222> (3)..(21)
   <223> n = a, t/u, g, c, unbekannt oder sonstige
<400> 56
   aannnnnnnn nnnnnnnnnn n 21
<210> 57
   <211> 22
   <212> DNA
   <213> Artificial Sequence
<220>
   <223> Sequenzfolge einer dsRNA, deren Zielsequenz am 5'-Ende die Nukleotidfolge AA aufweist (S.12 der Beschreibung).
   Sequenzmotiv 5'AAN(15-23)
<220>
   <221> misc_feature
   <222> (3)..(22)
   <223> n = a, t/u, g, c, unbekannt oder sonstige
<400> 57
   aannnnnnnn nnnnnnnnnn nn 22
<210> 58
   <211> 23
   <212> DNA
   <213> Artificial Sequence
<220>
   <223> Sequenzfolge einer dsRNA, deren Zielsequenz am 5'-Ende die Nukleotidfolge AA aufweist (S.12 der Beschreibung).
   Sequenzmotiv 5'AAN(15-23)
<220>
   <221> misc_feature
   <222> (3)..(23)
   <223> n = a, t/u, g, c, unbekannt oder sonstige
<400> 58
   aannnnnnnn nnnnnnnnnn nnn 23
<210> 59
   <211> 24
   <212> DNA
   <213> Artificial Sequence
<220>
   <223> Sequenzfolge einer dsRNA, deren Zielsequenz am 5'-Ende die Nukleotidfolge AA aufweist (S.12 der Beschreibung).
   Sequenzmotiv 5'AAN(15-23)
<220>
   <221> misc_feature
   <222> (3)..(24)
   <223> n = a, t/u, g, c, unbekannt oder sonstige
<400> 59
   aannnnnnnn nnnnnnnnnn nnnn 24
<210> 60
   <211> 25
   <212> DNA
   <213> Artificial Sequence
<220>
   <223> Sequenzfolge einer dsRNA, deren Zielsequenz am 5'-Ende die Nukleotidfolge AA aufweist (S.12 der Beschreibung).
   Sequenzmotiv 5'AAN(15-23)
<220>
   <221> misc_feature
   <222> (3)..(25)
   <223> n = a, t/u, g, c, unbekannt oder sonstige
<400> 60
   aannnnnnnn nnnnnnnnnn nnnnn 25
<210> 61
   <211> 17
   <212> DNA
   <213> Artificial Sequence
<220>
   <223> Sequenzfolge einer dsRNA, deren Zielsequenz am 3'-Ende die Nukleotidfolge TT aufweist (S.12 der Beschreibung).
   Sequenzmotiv 3'TTN(15-23)
<220>
   <221> misc_feature
   <222> (1)..(15)
   <223> n = a, t/u, g, c, unbekannt oder sonstige
<400> 61
   nnnnnnnnnn nnnnntt 17
<210> 62
   <211> 18
   <212> DNA
   <213> Artificial Sequence
<220>
   <223> Sequenzfolge einer dsRNA, deren Zielsequenz am 3'-Ende die Nukleotidfolge TT aufweist (S.12 der Beschreibung).
   Sequenzmotiv 3'TTN(15-23)
<220>
   <221> misc_feature
   <222> (1)..(16)
   <223> n = a, t/u, g, c, unbekannt oder sonstige
<400> 62
   nnnnnnnnnn nnnnnntt 18
<210> 63
   <211> 19
   <212> DNA
   <213> Artificial Sequence
<220>
   <223> Sequenzfolge einer dsRNA, deren Zielsequenz am 3'-Ende die Nukleotidfolge TT aufweist (S.12 der Beschreibung).
   Sequenzmotiv 3'TTN(15-23)
<220>
   <221> misc_feature
   <222> (1)..(17)
   <223> n = a, t/u, g, c, unbekannt oder sonstige
<400> 63
   nnnnnnnnnn nnnnnnntt 19
<210> 64
   <211> 20
   <212> DNA
   <213> Artificial Sequence
<220>
   <223> Sequenzfolge einer dsRNA, deren Zielsequenz am 3'-Ende die Nukleotidfolge TT aufweist (S.12 der Beschreibung).
   Sequenzmotiv 3'TTN(15-23)
<220>
   <221> misc_feature
   <222> (1)..(18)
   <223> n = a, t/u, g, c, unbekannt oder sonstige
<400> 64
   nnnnnnnnnn nnnnnnnntt 20
<210> 65
   <211> 21
   <212> DNA
   <213> Artificial Sequence
<220>
   <223> Sequenzfolge einer dsRNA, deren Zielsequenz am 3'-Ende die Nukleotidfolge TT aufweist (S.12 der Beschreibung).
   Sequenzmotiv 3'TTN(15-23)
<220>
   <221> misc_feature
   <222> (1)..(19)
   <223> n = a, t/u, g, c, unbekannt oder sonstige
<400> 65
   nnnnnnnnnn nnnnnnnnnt t 21
<210> 66
   <211> 22
   <212> DNA
   <213> Artificial Sequence
<220>
   <223> Sequenzfolge einer dsRNA, deren Zielsequenz am 3'-Ende die Nukleotidfolge TT aufweist (S.12 der Beschreibung).
   Sequenzmotiv 3'TTN(15-23)
<220>
   <221> misc_feature
   <222> (1)..(20)
   <223> n = a, t/u, g, c, unbekannt oder sonstige
<400> 66
   nnnnnnnnnn nnnnnnnnnn tt 22
<210> 67
   <211> 23
   <212> DNA
   <213> Artificial Sequence
<220>
   <223> Sequenzfolge einer dsRNA, deren Zielsequenz am 3'-Ende die Nukleotidfolge TT aufweist (S.12 der Beschreibung).
   Sequenzmotiv 3'TTN(15-23)
<220>
   <221> misc_feature
   <222> (1)..(21)
   <223> n = a, t/u, g, c, unbekannt oder sonstige
<400> 67
   nnnnnnnnnn nnnnnnnnnn ntt 23
<210> 68
   <211> 24
   <212> DNA
   <213> Artificial Sequence
<220>
   <223> Sequenzfolge einer dsRNA, deren Zielsequenz am 3'-Ende die Nukleotidfolge TT aufweist (S.12 der Beschreibung).
   Sequenzmotiv 3'TTN(15-23)
<220>
   <221> misc_feature
   <222> (1)..(22)
   <223> n = a, t/u, g, c, unbekannt oder sonstige
<400> 68
   nnnnnnnnnn nnnnnnnnnn nntt 24
<210> 69
   <211> 25
   <212> DNA
   <213> Artificial Sequence
<220>
   <223> Sequenzfolge einer dsRNA, deren Zielsequenz am 3'-Ende die Nukleotidfolge TT aufweist (S.12 der Beschreibung).
   Sequenzmotiv 3'TTN(15-23)
<220>
   <221> misc_feature
   <222> (1)..(23)
   <223> n = a, t/u, g, c, unbekannt oder sonstige
<400> 69
   nnnnnnnnnn nnnnnnnnnn nnntt 25
<210> 70
   <211> 21
   <212> RNA
   <213> Rattus norvegicus
<220>
   <221> misc_feature
   <222> (1)..(21)
   <223> Zielsequenz der VGLUT2-mRNA (S. 17 der Beschreibung).
<400> 70
   aaggcuccgc uaugcgacug u 21
<210> 71
   <211> 21
   <212> DNA
   <213> Artificial Sequence
<220>
   <223> Sense-Strang der dsRNA, die gegen die vorstehende Zielsequenz der VGLUT2-mRNA der Ratte gerichtet ist (mit einem ueberhaengenden TT am 3'-Terminus). (S. 17 der Beschreibung).
<400> 71
   ggcuccgcua ugcgacugut t 21
<210> 72
   <211> 21
   <212> DNA
   <213> Artificial Sequence
<220>
   <223> Anti-Sense-Strang der dsRNA, die gegen die vorstehende Zielsequenz der VGLUT2-mRNA der Ratte gerichtet ist (mit einem ueberhaengendem TT am 3'-Terminus). (S. 17 der Beschreibung).
<400> 72
   acagucgcau agcggagcct t 21
<210> 73
   <211> 21
   <212> DNA
   <213> Artificial Sequence
<220>
   <223> si-rVGLUT1 739-759 EGT (S. 36 der Beschreibung).
<400> 73
   agcgccaagc ucaugaacct t 21
<210> 74
   <211> 21
   <212> DNA
   <213> Artificial Sequence
<220>
   <223> si-rVGLUT1 739-759 EGT (S. 36 der Beschreibung)
<400> 74
   gguucaugag cuuggcgcut t 21
<210> 75
   <211> 21
   <212> DNA
   <213> Artificial Sequence
<220>
   <223> si-rVGLUT2 100-120 EGT (aktive siRNA) (S. 36 der Beschreibung)
<400> 75
   gcaggauaac cgagagacct t 21
<210> 76
   <211> 21
   <212> DNA
   <213> Artificial Sequence
<220>
   <223> si-rVGLUT2 100-120 EGT (aktive siRNA) (S. 36 der Beschreibung)
<400> 76
   ggucucucgg uuauccugct t 21
<210> 77
   <211> 21
   <212> DNA
   <213> Artificial Sequence
<220>
   <223> si-rVGLUT3 220-240 EGT (S. 36 der Beschreibung)
<400> 77
   gcgguacauc aucgcuguct t 21
<210> 78
   <211> 21
   <212> DNA
   <213> Artificial Sequence
<220>
   <223> si-rVGLUT3 220-240 EGT (S. 36 der Beschreibung)
<400> 78
   gacagcgaug auguaccgct t 21
<210> 79
   <211> 21
   <212> DNA
   <213> Artificial Sequence
<220>
   <223> si-VGLUT2 MM EGT (Kontroll-siRNA) (S. 36 der Beschreibung)
<400> 79
   ggacuagcaa agcgagccat t 21
<210> 80
   <211> 21
   <212> DNA
   <213> Artificial Sequence
<220>
   <223> si-VGLUT2 MM EGT (Kontroll-siRNA) (S. 36 der Beschreibung)
<400> 80
   uggcucgcuu ugcuagucct t 21
<210> 81
   <211> 21
   <212> DNA
   <213> Artificial Sequence
<220>
   <223> si-rVGLUT2 100-120 AMB (aktive siRNA) (S. 36 der Beschreibung)
<400> 81
   gcaggauaac cgagagacct t 21
<210> 82
   <211> 21
   <212> DNA
   <213> Artificial Sequence
<220>
   <223> si-rVGLUT2 100-120 AMB (aktive siRNA) (S. 36 der Beschreibung)
<400> 82
   ggucucucgg uuauccugct t 21
<210> 83
   <211> 21
   <212> DNA
   <213> Artificial Sequence
<220>
   <223> si-rVGLUT2 166-186 AMB (S. 36 der Beschreibung)
<400> 83
   ggcuccgcua ugcgacugut t 21
<210> 84
   <211> 21
   <212> DNA
   <213> Artificial Sequence
<220>
   <223> si-rVGLUT2 166-186 AMB (S. 37 der Beschreibung)
<400> 84
   acagucgcau agcggagcct t 21
<210> 85
   <211> 21
   <212> DNA
   <213> Rattus norvegicus
<220>
   <221> misc_feature
   <222> (1)..(21)
   <223> Targetsequenz von VGLUT2 der Ratte (S. 18 der Beschreibung).
<400> 85
   aagcaggata accgagagac c 21
<210> 86
   <211> 21
   <212> RNA
   <213> Rattus norvegicus
<220>
   <221> misc_feature
   <222> (1)..(21)
   <223> Zielsequenz der VGLUT2-mRNA der Ratte (S. 48 der Beschreibung).
<400> 86
   aagcaggaua accgagagac c 21
<210> 87
   <211> 21
   <212> DNA
   <213> Artificial Sequence
<220>
   <223> Sense-Strang der siRNA, die gegen die vorstehende Targetsequenz von VGLUT2 bzw. die Zielsequenz von VGLUT2-mRNA gerichtet ist. (S. 18 u. 48 der Beschreibung).
<220>
   <221> misc_feature
   <222> (20)..(20)
   <223> T = dT (desoxy Thymidin)
<220>
   <221> misc_feature
   <222> (21)..(21)
   <223> T = dT (desoxy Thymidin)
<400> 87
   gcaggauaac cgagagacct t 21
<210> 88
   <211> 21
   <212> DNA
   <213> Artificial Sequence
<220>
   <223> Antisense-Strang der siRNA, die gegen die vorstehende Targetsequenz von VGLUT2 bzw. die Zielsequenz von VGLUT2-mRNA gerichtet ist. (S. 18 u. 48 der Beschreibung).
<220>
   <221> misc_feature
   <222> (20)..(20)
   <223> T = dT (desoxy Thymidin)
<220>
   <221> misc_feature
   <222> (21)..(21)
   <223> T = dT (desoxy Thymidin)
<400> 88
   ggucucucgg uuauccugct t 21
<210> 89
   <211> 21
   <212> DNA
   <213> Artificial sequence
<220>
   <223> Targetsequenz (keine VGLUT2-Sequenz) der Kontroll-siRNA in Ausfuehrungsbeispiel 4. (S. 48 der Beschreibung).
<400> 89
   aaggactagc aaagcgagcc a 21
<210> 90
   <211> 21
   <212> DNA
   <213> Artificial Sequence
<220>
   <223> Sense-Strang der doppelstraengigen Kontroll-siRNA, die gegen die vorstehende Targetsequenz gerichtet ist. (S. 48 der Beschreibung).
<220>
   <221> misc_feature
   <222> (20)..(20)
   <223> T = dT (desoxy Thymidin)
<220>
   <221> misc_feature
   <222> (21)..(21)
   <223> T = dT (desoxy Thymidin)
<400> 90
   ggacuagcaa agcgagccat t 21
<210> 91
   <211> 21
   <212> DNA
   <213> Artificial Sequence
<220>
   <223> Antisense-Strang der doppelstraengigen Kontroll-siRNA, die gegen die vorstehende Targetsequenz gerichtet ist. (S. 48 der Beschreibung).
<220>
   <221> misc_feature
   <222> (20)..(20)
   <223> T = dT (desoxy Thymidin)
<220>
   <221> misc_feature
   <222> (21)..(21)
   <223> T = dT (desoxy Thymidin)
<400> 91
   uggcucgcuu ugcuagucct t 21
<210> 92
   <211> 19
   <212> DNA
   <213> Artificial Sequence
<220>
   <223> PCR-Primer: rVGluT1(2_4)F; (S. 56 der Beschreibung).
<400> 92
   tctgggtttc tgcatcagc 19
<210> 93
   <211> 20
   <212> DNA
   <213> Artificial Sequence
<220>
   <223> PCR-Primer: rVGluT1(2_4)R; (S. 56 der Beschreibung).
<400> 93
   ccatgtatga ggccgacagt 20
<210> 94
   <211> 20
   <212> DNA
   <213> Artificial Sequence
<220>
   <223> PCR-Primer: rVGluT2(8_9)F; (S. 56 der Beschreibung).
<400> 94
   aagaccccat ggaggaagtt 20
<210> 95
   <211> 20
   <212> DNA
   <213> Artificial Sequence
<220>
   <223> PCR-Primer: rVGluT2(8_9)R; (S. 56 der Beschreibung).
<400> 95
   attgtcatga ccaggtgtgg 20
<210> 96
   <211> 20
   <212> DNA
   <213> Artificial Sequence
<220>
   <223> PCR-Primer: rVGluT3(4_5)F; (S. 56 der Beschreibung).
<400> 96
   atccagagac ggtgggtctt 20
<210> 97
   <211> 20
   <212> DNA
   <213> Artificial Sequence
<220>
   <223> PCR-Primer: rVGluT3(4_5)R; (S. 56 der Beschreibung).
<400> 97
   atgacacagc cgtaatgcac 20
<210> 98
   <211> 20
   <212> DNA
   <213> Artificial Sequence
<220>
   <223> PCR-Primer: rGAPDH(7_8)F; (S. 56 der Beschreibung).
<400> 98
   atcctgggct acactgagga 20
<210> 99
   <211> 20
   <212> DNA
   <213> Artificial Sequence
<220>
   <223> PCR-Primer: rGAPDH(7_8)R; (S. 56 der Beschreibung).
<400> 99
   atgtaggcca tgaggtccac 20
<210> 100
   <211> 29
   <212> DNA
   <213> Artificial Sequence
<220>
   <223> Primer rVGluT2 TS10 as; (S. 56 der Beschreibung).
<400> 100
   aagcaggata accgagagac ccctgtctc 29
<210> 101
   <211> 29
   <212> DNA
   <213> Artificial Sequence
<220>
   <223> Primer rVGluT2 TS10 s; (S. 56 der Beschreibung).
<400> 101
   aaggtctctc ggttatcctg ccctgtctc 29
<210> 102
   <211> 29
   <212> DNA
   <213> Artificial Sequence
<220>
   <223> Primer rVGluT2 TS14 as; (S. 57 der Beschreibung).
<400> 102
   aaggctccgc tatgcgactg tcctgtctc 29
<210> 103
   <211> 29
   <212> DNA
   <213> Artificial Sequence
<220>
   <223> Primer rVGluT2 TS14 s; (S. 57 der Beschreibung).
<400> 103
   aaacagtcgc atagcggagc ccctgtctc 29
<210> 104
   <211> 1683
   <212> DNA
   <213> Unknown
<220>
   <223> Codierende Sequence einer VGLUT1 mit der Genbank Accession-Nummer U07609 (Fig. 5).
<400> 104
   atggagttcc ggcaggagga gtttcggaag ctggcggggc gcgccctggg gaggctgcac    60
   cggttactgg agaagcggca ggaaggcgcg gagacattgg agctgagcgc cgacgggcgc   120
   ccagtgacca cacacacgcg ggacccgccg gtggtggact gcacttgctt tggcctccct   180
   cgccgctaca tcatcgcgat catgagcggt ctgggtttct gcatcagctt tggcatccgc   240
   tgcaacctgg gcgtggccat cgtatccatg gtcaacaaca gtacaaccca ccgtgggggc   300
   cacgtggtgg tgcagaaagc ccagttcaac tgggatccag agactgtcgg cctcatacat   360
   ggctcctttt tctgggggta cattgtcact cagattcctg gaggatttat ctgccaaaaa   420
   ttcgcagcca acagggtctt tggctttgcc attgtggcta cctccaccct aaatatgttg   480
   atcccttcag cagcccgtgt tcactatggc tgtgtcatct tcgtgaggat ccttcaggga   540
   ttggtggagg gggtcacata ccctgcttgc catggcatct ggagcaaatg ggcccctccc   600
   ttagaacgga gtcggctggc gacgacagcc ttttgcggtt cctatgccgg ggcagtggtt   660
   gccatgcctc tggctggggt cctggtacag tattcaggat ggagttctgt cttctatgtc   720
   tatggcagct tcgggatctt ttggtacctg ttctggttgc ttgtctccta cgagtcacct   780
   gcactacacc ccagcatctc cgaggaggag cgcaaataca ttgaggatgc catcggagaa   840
   agcgccaagc tcatgaaccc tgttacgaag tttaacacac cctggaggcg cttctttacc   900
   tccatgccgg tctatgccat cattgtcgcc aacttttgcc gcagctggac tttctacctg   960
   ctcctcatct cccagcccgc ctactttgaa gaagtgttcg gctttgagat cagcaaggtg  1020
   ggactggtgt cggcactgcc tcaccttgtc atgactatca tcgtacccat cggaggccag  1080
   atcgccgact tcctgcgcag tcgtcatata atgtccacga ccaatgtgcg aaagctgatg  1140
   aactgcgggg gtttcgggat ggaagctacg ctgctgctgg tggtcggata ctcacactcc  1200
   aagggcgtgg ccatctcctt cctggtcctg gctgtgggct tcagtggctt tgctatctct  1260
   gggtttaacg tgaaccactt ggacatcgcc cctcgatatg ccagcatctt gatgggcatt  1320
   tccaatggcg tgggcacact gtctgggatg gtgtgcccca tcatcgtggg tgcaatgacc  1380
   aagcacaaga cgcgggagga gtggcagtac gtgttcctca tagcctccct ggtgcactat  1440
   ggaggtgtca tcttctatgg ggtctttgct tcgggagaga aacagccgtg ggcagagccg  1500
   gaggagatga gcgaggagaa gtgtggcttt gttggccacg accagctggc tggcagtgac  1560
   gaaagtgaaa tggaagacga ggttgagccc ccgggggcac cccccgcacc tccgccttcc   1620
   tacggggcca cacacagcac agttcagcct ccaaggcccc caccccctgt ccgggactac   1680
   tga                                                                 1683
<210> 105
   <211> 1749
   <212> DNA
   <213> Rattus norvegicus
<220>
   <221> misc_feature
   <222> (1)..(1749)
   <223> Codierende Sequenz von VGLUT2,; Genbank Accession-Nummer NM_053427 (Figur 6).
<400> 105
   atggagtcgg taaaacaaag gattttggcc ccggggaaag aggggataaa gaattttgct 60
   ggaaaatccc tcggacagat ctacagggtg ctggagaaga agcaggataa ccgagagacc 120
   atcgagctga cagaggacgg caagcccctg gaggtgcctg agaagaaggc tccgctatgc 180
   gactgtacgt gcttcggcct gccgcgccgc tacatcatag ccatcatgag cggcctcggc 240
   ttctgcatct cctttggtat ccgctgtaac ctgggtgtgg ccattgtgga catggtcaac 300
   aacagcacca tccaccgggg tggcaaagtt atcaaggaga aagccaagtt taactgggac 360
   cccgagactg tggggatgat tcacgggtcg ttcttctggg gctatatcat cacgcagatt 420
   ccgggcggat acatcgcatc gcgactggct gctaaccggg tctttggggc tgccatactg 480
   cttacctcta ccctcaatat gctgatccca tctgcagcca gagtgcatta tggatgcgtc 540
   atctttgtta gaatattgca aggacttgtg gagggcgtca cctacccagc ctgtcacggg 600
   atatggagca agtgggcccc tcctttggag aggagtaggt tggctaccac ctccttctgt 660
   ggttcctatg ctggagcagt cattgcaatg cccctagctg gtatcctggt gcagtacact 720
   ggatggtctt cagtatttta cgtatatgga agctttggta tggtctggta tatgttctgg 780
   cttctggtgt cttacgagag ccccgcaaag catccaacca taacagacga agaacgtagg 840
   tacatagaag agagcatcgg ggagagcgca aatctgttag gagcaatgga gaaattcaag 900
   accccatgga ggaagttttt cacatccatg cccgtctatg cgataattgt tgcaaacttc 960
   tgcaggagtt ggacttttta tttactgctc atcagtcaac cagcttattt cgaggaggtt 1020
   tttggatttg aaatcagcaa ggttggcatg ttgtctgcgg tcccacacct ggtcatgaca 1080
   atcattgtgc ctatcggggg gcaaattgca gactttctaa ggagcaagca aattctttca 1140
   acaactacag tgcgaaagat catgaactgc gggggttttg gcatggaagc cacactgctt 1200
   ctggttgttg gctactctca tactagaggg gtggccatct ccttcttggt gcttgcagtg 1260
   ggattcagtg gatttgctat ctctggtttc aatgtgaacc acttggatat tgccccgaga 1320
   tatgccagta tcttaatggg catttcaaat ggtgttggca cgctgtcggg aatggtctgc   1380
   ccgatcattg ttggtgcaat gacgaagaac aagtcccgtg aagaatggca gtatgtcttc   1440
   ctcatcgctg cactggtcca ctatggtgga gtcatatttt atgcactatt tgcctcagga   1500
   gagaagcaac cttgggcaga ccctgaggaa acaagcgaag aaaagtgtgg cttcattcat   1560
   gaagatgaac tggatgaaga aacgggggac atcactcaga attacataaa ttacggtacc   1620
   accaaatcct acggcgccac ctcacaggag aacggaggct ggcctaacgg ctgggagaaa   1680
   aaggaagaat ttgtgcaaga aagtgcgcaa gacgcgtact cctataagga ccgagatgat   1740
   tattcataa                                                           1749
<210> 106
   <211> 1767
   <212> DNA
   <213> Unknown
<220>
   <223> Codierende Sequenz einer VGLUT3 mit der Genbank Accession-Nummer AJ491795 (Fig. 7).
<400> 106
   atgccattta acgcatttga taccttcaaa gaaaaaattt tgaaacccgg gaaggaagga     60
   gtgaagaatg ccgtgggaga ttcgctgggg atcttacaaa gaaaactcga tgggaccaac    120
   gaggagggag atgccattga gctgagtgag gaaggaaggc ccgtgcagac atccagagcc    180
   cgagcccctg tgtgcgactg cagctgctgt ggcatcccca agcggtacat catcgctgtc    240
   atgagtggcc tgggattctg catttccttt gggattcggt gcaaccttgg agtggccatt    300
   gtggaaatgg tcaacaatag cactgtgtat gtggatggga aaccggaaat ccagacagca    360
   cagtttaact gggatccaga gacggtgggt cttatccatg gatctttttt ctggggttat    420
   attgtgacac aaattcccgg tggcttcatt tcaaacaagt ttgctgctaa cagggtcttt    480
   ggagctgcca tcttcttgac gtcaaccctg aacatgttca tcccttccgc ggccagggtg    540
   cattacggct gtgtcatgtg tgtgaggatt ttgcagggtc tggtggaggg tgtgacctac    600
   ccagcctgcc acgggatgtg gagtaagtgg gcacctcccc tggagagaag tcgtctagcc    660
   acaacctctt tttgtggttc ctatgccggg gcagtcgttg ctatgcccct tgcaggagta    720
   ttggtgcagt acattggctg ggcctctgtc ttttatattt acgggatgtt tggaattatt    780
   tggtacatgt tttggctgct gcaggcttat gagtgtccag cagttcaccc aacaatatcc    840
   aatgaagaac ggacctacat agagacaagt ataggagaag gcgccaactt ggccagtctg    900
   agcaaattca acacaccatg gagaaggttt ttcacatcct tgcctgtcta tgccattatt    960
   gtggcaaact tttgtagaag ctggaccttc tatttgctct taataagtca gcctgcttac   1020
   tttgaagagg tctttgggtt tgcaataagt aaggtgggtc tcttgtcagc tgtcccacac   1080
   atggtgatga caatcgtggt acccattgga ggacaactgg ctgattattt aagaagccga    1140
   aagattttga ccacaactgc tgtcagaaag atcatgaatt gtggaggctt tggcatggag    1200
   gcaaccttgc tcctggtggt tgggttttcc cataccaaag gagtggctat ctccttcctg    1260
   gtgcttgctg taggatttag tggctttgca atttcaggtt tcaatgtcaa ccacctggac    1320
   attgctccac gatatgccag catcctcatg gggatctcaa atggcgtggg aaccctctct    1380
   ggaatggttt gtcccctcat tgttggtgca atgacaaagc acaagacccg ggaagaatgg    1440
   cagaatgtgt tcctcatagc agccctggtg cactacagtg gagtcatctt ctacggggtc    1500
   tttgcttctg gggaaaaaca ggactgggct gatccagaga atctctctga ggagaaatgt    1560
   ggaatcattg accaagatga attagccgag gaaacagaac tcaaccacga ggctttcgta    1620
   agtcccagaa agaagatgtc ttatggagcc accacccaga attgtgaggt ccagaagacg    1680
   gatcggagac aacagagaga atccgccttc gagggggagg agccattatc ctaccagaat    1740
   gaagaggact tttcagaaac atcttaa                                        1767
<210> 107
   <211> 2959
   <212> DNA
   <213> Homo sapiens
<220>
   <221> misc_feature
   <222> (1)..(2959)
   <223> VGLUT1 (Fig. 27A)
<400> 107
   acttgcagcc tccttccccc cgagcggagc tgcggggccg gccgggccgg ggcggacccc      60
   gggaacccgg acgcggccgc ccgggcccgc gggcgggggg atcggcgggg gggacccgcg     120
   gggtgaccgg cggcaggagc cgccaccatg gagttccgcc aggaggagtt tcggaagcta     180
   gcgggtcgtg ctctcgggaa gctgcaccgc cttctggaga agcggcagga aggcgcggag     240
   acgctggagc tgagtgcgga tgggcgcccg gtgaccacgc agacccggga cccgccggtg     300
   gtggactgca cctgcttcgg cctccctcgc cgctacatta tcgccatcat gagtggtctg     360
   ggcttctgca tcagctttgg catccgctgc aacctgggcg tggccatcgt ctccatggtc     420
   aataacagca cgacccaccg cgggggccac gtggtggtgc agaaagccca gttcagctgg     480
   gatccagaga ctgtcggcct catacacggc tcctttttct ggggctacat tgtcactcag     540
   attccaggag gatttatctg tcaaaaattt gcagccaaca gagttttcgg ctttgctatt     600
   gtggcaacat ccactctaaa catgctgatc ccctcagctg cccgcgtcca ctatggctgt     660
   gtcatcttcg tgaggatcct gcaggggttg gtagaggggg tcacataccc cgcctgccat     720
   gggatctgga gcaaatgggc cccaccctta gaacggagtc gcctggcgac gacagccttt     780
   tgtggttcct atgctggggc ggtggtcgcg atgcccctcg ccggggtcct tgtgcagtac 840
   tcaggatgga gctctgtttt ctacgtctac ggcagcttcg ggatcttctg gtacctgttc 900
   tggctgctcg tctcctacga gtcccccgcg ctgcacccaa gcatctcgga ggaggagcgc 960
   aagtacatcg aggacgccat cggagagagc gcgaaactca tgaaccccct cacgaagttt 1020
   agcactccct ggcggcgctt cttcacgtct atgccagtct atgccatcat cgtggccaac 1080
   ttctgccgca gctggacgtt ctacctgctg ctcatctccc agcccgccta cttcgaagaa 1140
   gtgttcggct tcgagatcag caaggtaggc ctggtgtccg cgctgcccca cctggtcatg 1200
   accatcatcg tgcccatcgg cggccagatc gcggacttcc tgcggagccg ccgcatcatg 1260
   tccaccacca acgtgcgcaa gttgatgaac tgcggaggct tcggcatgga agccacgctg 1320
   ctgttggtgg tcggctactc gcactccaag ggcgtggcca tctccttcct ggtcctagcc 1380
   gtgggcttca gcggcttcgc catctctggg ttcaacgtga accacctgga catagcccgg 1440
   cgctacgcca gcatcctcat gggcatctcc aacggcgtgg gcacactgtc gggcatggtg 1500
   tgccccatca tcgtgggggc catgactaag cacaagactc gggaggagtg gcagtacgtg 1560
   ttcctaattg cctccctggt gcactatgga ggtgtcatct tctacggggt ctttgcttct 1620
   ggagagaagc agccgtgggc agagcctgag gagatgagcg aggagaagtg tggcttcgtt 1680
   ggccatgacc agctggctgg cagtgacgac agcgaaatgg aggatgaggc tgagcccccg 1740
   ggggcacccc ctgcaccccc gccctcctat ggggccacac acagcacatt tcagcccccc 1800
   aggcccccac cccctgtccg ggactactga ccatgtgcct cccactgaat ggcagtttcc 1860
   aggacctcca ttccactcat ctctggcctg agtgacagtg tcaaggaacc ctgctcctct 1920
   ctgtcctgcc tcaggcctaa gaagcactct cccttgttcc cagtgctgtc aaatcctctt 1980
   tccttcccaa ttgcctctca ggggtagtga agctgcagac tgacagtttc aaggataccc 2040
   aaattcccct aaaggttccc tctccacccg ttctgcctca gtggtttcaa atctctcctt 2100
   tcagggcttt atttgaatgg acagttcgac ctcttactct ctcttgtggt tttgaggcac 2160
   ccacaccccc cgctttcctt tatctccagg gactctcagg ctaacctttg agatcactca 2220
   gctcccatct cctttcagaa aaattcaagg tcctcctcta gaagtttcaa atctctccca 2280
   actctgttct gcatcttcca gattggttta accaattact tgtccccgcc attccaggga 2340
   ttgattctca ccagcgtttc tgatggaaaa tggcggtttc aagtccccga ttccgtgccc 2400
   acttcacatc tcccctacca gcagattctg cgaaagcacc aaatttctca agaccctctt 2460
   ctccctagct tagcataatg tctggggaaa caaccaaaat cgcaatttta acaatatgcc 2520
   tctctacccc cgtgcacttt ttctgacatg gttttcaggt ctaaatagtg gctgctccag 2580
   tccatgaact caaaggtttg aagctaccac cattgaactc ccccatggtg gtttcatgat 2640
   gccccctccc caattcctcg cactttattc tcctgggtgg tttcgaacta ccctgtttct 2700
   cagtggccat ttgttgtgtc cctcaggggc ttaatgactc aaaatctggg atccttcccc 2760
   tctcagacac ccctctttca gcttagaatt tggggaacct atagagaagt cacagaatcc 2820
   catgaaaggg aatggggcag gagacagggg tgttttttcc cagtgcaggg ttgtgtcttt 2880
   gtgtctctgt ctctgtgact gtaaatcctg ccctgcccca ctcccaataa aagctttggt 2940
   gtataggcaa aaaaaaaaa 2959
<210> 108
   <211> 3946
   <212> DNA
   <213> Homo sapiens
<220>
   <221> misc_feature
   <222> (1)..(3946)
   <223> VGLUT2 (Fig. 27B)
<400> 108
   cgtttaaaag ccatcagatt tgagagcaat aagtcttcaa aaccgggaat ttacattgtt 60
   tttcagctga ccgacttcca ggaaaaggac tcaaccgcat ctacccaaat accgtggcac 120
   tgcttgcgct ctttgccacc ggatactccc cttccaatga gactttctga ttgtgtctac 180
   caactctcct attaggaaac ccgtgggttg catgcagcta ttctgttgta ttctcattct 240
   cactctccct cccttctctc actctcactc ttgctggagg cgagccacta ccattctgct 300
   gagaaggaaa agcccgcaac tactttaaga gattaagaca atatgcgcaa tcctcgcctt 360
   tcctagcaat cactatttaa atctggcaag aactgacaac agtctttgca agaatggaat 420
   ccgtaaaaca aaggattttg gccccaggaa aagaggggct aaagaatttt gctggaaaat 480
   cactcggcca gatctacagg gtgctggaga agaagcaaga caccggggag acaatcgagc 540
   tgacggagga tgggaagccc ctagaggtgc ccgagaggaa ggcgccgctg tgcgactgca 600
   cgtgcttcgg cctgccccgc cgctacatta tcgccatcat gagcggcctg ggcttctgca 660
   tctccttcgg tatccgctgc aacctgggcg tggccattgt ggacatggtc aacaacagca 720
   ccatccaccg cgggggcaag gtcatcaagg agaaagccaa attcaactgg gacccggaaa 780
   ccgtggggat gatccacggt tccttctttt ggggctacat catcactcag attccgggag 840
   gctacatcgc gtctcggctg gcagccaaca gggttttcgg agctgccata cttcttacct 900
   ctaccctaaa tatgctaatt ccatcagcag ccagagtgca ttatggatgt gtcatctttg 960
   tcagaatact gcagggactt gttgagggtg tgacctaccc agcatgtcat gggatatgga 1020
   gcaaatgggc cccacctcta gagaggagta gactggcaac cacctccttt tgtggttcct 1080
   atgccggagc tgtgattgca atgcctttag ctggcattct tgtgcagtac actggctggt 1140
   cttcagtgtt ttatgtctac ggaagctttg gaatggtctg gtacatgttt tggcttttgg 1200
   tgtcttatga aagtcctgca aagcatccta ctattacaga tgaagaacgt aggtacatag 1260
   aagaaagcat tggagagagt gcaaatcttt taggtgcaat ggaaaaattc aagactccat 1320
   ggaggaagtt ttttacatcc atgccagtct atgcaataat tgttgcaaac ttctgcagaa 1380
   gctggacttt ttatttattg cttattagtc agccagcata ttttgaggaa gtctttggat 1440
   ttgaaattag caaggttggt atgctatctg ctgtgccaca cttagtaatg acaattattg 1500
   tgcctattgg gggacaaatt gcagattttc taagaagcaa gcagattctt tcaactacga 1560
   cagtgagaaa gatcatgaat tgtggtggtt ttggcatgga agccacactg ctcctggtcg 1620
   ttggctattc tcatactaga ggggtagcaa tctcattctt ggtacttgca gtgggattca 1680
   gtggatttgc tatatctggt ttcaatgtta accacttgga tatcgctcca agatatgcca 1740
   gtatcttaat gggcatttcg aatggtgttg gcacattgtc aggaatggtt tgtcctatca 1800
   ttgttggtgc aatgacaaag aataagtcac gtgaagagtg gcagtatgtc ttcctgatcg 1860
   ctgccctagt ccactatggt ggagttatat tttatgcaat atttgcctca ggagagaaac 1920
   aaccctgggc agacccggag gaaacaagtg aagaaaaatg tggatttatt catgaagatg 1980
   aactcgatga agaaacaggg gacattactc aaaattatat aaattatggt accaccaagt 2040
   cttatggtgc cacaacacag gccaatggag gttggcctag tggttgggaa aagaaagagg 2100
   aatttgtaca aggagaagta caagactcac atagctataa ggaccgagtt gattattcat 2160
   aacaaaacta attactggat ttatttttag tgtttgtgat taaattcatt gtgattgcac 2220
   aaaaatttta aaaacacgtg atgtaaactt gcaagcatat caaccaggca agtcttgctg 2280
   taaaaatgaa aacaaaacaa acccatgagg ttaccatcaa gtgcaatctg taaaattgtg 2340
   aagttccatc atttccattc aagtcatcca ttcttgcatt tgtgacttaa aggttgactg 2400
   gtcaaaattg tagaaacaag tagttaccca ttggattcat atgagctaaa actcatcact 2460
   atttactaaa gcacaacatc tcatcctaca aaagttaaga agccaaagct acttgatcat 2520
   gcaaaatgca cttatatatt tgttacactg tattgcaaga tagcacacag aagttggctg 2580
   cgtcaagtag aggcgacatt tattaagtga aaatcatgga gttgggatat ctctcaatta 2640
   aagaaataca ttgtgaacta tcagctacaa agttgtactg aataactatt agaattgcat 2700
   aatgtgagat attttgttag tcctcaaaag gaatatcttg cagtgttttc tatgaaatgc 2760
   ttgggcacaa acacttattt ctgtgaaaga gaacatgtaa gttgaggggt atgcttcatg 2820
   ttcttccatc catttaccta atagtatgaa acagttcaca tttcaataaa atcaaacttt 2880
   tcatgtagcg tatcacataa cttttttgca aaaaatataa aaagaaataa acttcaatgt 2940
   attttttatt acaactttgt actggttgta acttgcatta gaaaaaaaaa agagatatat 3000
   aaaccacaaa gaatctaata agaaatttat tatggagata tagcccttaa aatgcaatat 3060
   taagaacaaa gaaatagaaa atggtttaga tatctttctt ccttcataat taaatactat 3120
   atgaaacttg tgccacagag ctatatgtaa tatgaaaaga ttaacttcat agagatattg 3180
   taagtaggta attttattat ttaaagtcct attaagaaat atttgtctta aatatatagg 3240
   acaatacatt atattaaaat ggtctctctc tatatatatc tgtatatctt atacatgtcc 3300
   atacacagaa acataataaa caatcttcac acgaaaccaa aaatagcata cacctaatgt 3360
   tgggttaggg aattgcaatt tctactttca tagagtcata gaattttagg tggggaagag 3420
   gcattttgct tgtcatttct taatataact caacaagaat tgcaacattt gtgtaccaag 3480
   caataagtgc aatgcataaa atttcctgtc tgtatattac cttcattttg cttgtagtag 3540
   ctgtttgggt ggttggaata attttatttt tcttttaaaa aagctaacat cagacccctt 3600
   tataatgtcc taaaattatg ataatacatt tcccaattca actcaaaata ttattggtgt 3660
   attttgtcta ttctggatat ttgatctgtt taatgtactg tgctagtgac tggaggccct 3720
   gctactgcaa atataaaacc taaagtttgt ttaaaaaaat gcaaatcatt ctttacctta 3780
   agaaaaaaaa aatacccttt gctttgtgcc tcaaagtgat gtaatgtgat cacagctttt 3840
   gttgtgttga atgaaaatat gtggactgtc attttgttgc agcaaaaaag tgttaataaa 3900
   atgctctatt tatccttttt taaaaaaaaa aaaaaaaaaa aaaaaa 3946
<210> 109
   <211> 3838
   <212> DNA
   <213> Homo sapiens
<220>
   <221> misc_feature
   <222> (1)..(3838)
   <223> VGLUT3 (Fig. 27C)
<400> 109
   tcttggaaga tccgagctgg gtttcatctc ctttttgatt ttgagtagtt ccctccacga     60
   gaactgactt ccaggtgttc accaagggaa acaaggtggt tctcacactg gaaatgagga    120
   aggatgacag tttttgagac tgactgttaa cggctcagag gtgcccctca ttcaaaatgc    180
   cttttaaagc atttgatacc ttcaaagaaa aaattctgaa acctgggaag gaaggagtga    240
   agaacgccgt gggagattct ttgggaattt tacaaagaaa aatcgatggg acaactgagg    300
   aagaagataa cattgagctg aatgaagaag gaaggccggt gcagacgtcc aggccaagcc    360
   ccccactctg cgactgccac tgctgcggcc tccccaagcg ttacatcatt gctatcatga    420
   gtgggctggg attctgcatt tcctttggga tccggtgcaa tcttggagtt gccattgtgg    480
   aaatggtcaa caatagcacc gtatatgttg atggaaaacc ggaaattcag acagcacagt    540
   ttaactggga tccagaaaca gtgggcctta tccatggatc tttttctggg ggctatatta    600
   tgacacaaat tccaggtggt ttcatttcaa acaagtttgc tgctaacagg gtctttggag 660
   ctgccatctt cttaacatcg actctgaaca tgtttattcc ctctgcagcc agagtgcatt 720
   acggatgcgt catgtgtgtc agaattctgc aaggtttagt ggagggtgtg acctacccag 780
   cctgccatgg gatgtggagt aagtgggcac cacctttgga gagaagccga ctggccacaa 840
   cctctttttg tggttcctat gcaggggcag tggttgccat gcccctggct ggggtgttgg 900
   tgcagtacat tggatggtcc tctgtctttt atatttatgg catgtttggg attatttggt 960
   acatgttttg gctgttgcag gcctatgagt gcccagcagc tcatccaaca atatccaatg 1020
   aggagaagac ctatatagag acaagcatag gagagggggc caacgtggtt agtctaagta 1080
   aatttagtac cccatggaaa agatttttca catctttgcc ggtttacgca atcattgtgg 1140
   caaatttttg cagaagctgg accttttatt tgctcctcat aagtcagcct gcttattttg 1200
   aagaggtctt tggatttgca ataagtaagg tgggtctctt gtcagcagtc ccacacatgg 1260
   ttatgacaat cgttgtacct attggaggac aattggctga ttatttaaga agcagacaaa 1320
   ttttaaccac aactgctgtc agaaaaatca tgaactgtgg aggttttggc atggaggcaa 1380
   ccttactcct ggtggttggc ttttcgcata ccaaaggggt ggctatctcc tttctggtac 1440
   ttgctgtagg atttagtggc ttcgctattt caggttttaa tgtcaaccac ctggacattg 1500
   ccccacgcta tgccagcatt ctcatgggga tctcaaacgg agtgggaacc ctctctggaa 1560
   tggtctgtcc cctcattgtc ggtgcaatga ccaggcacaa gacccgtgaa gaatggcaga 1620
   atgtgttcct catagctgcc ctggtgcatt acagtggtgt gatcttctat ggggtctttg 1680
   cttctgggga gaaacaggag tgggctgacc cagagaatct ctctgaggag aaatgtggaa 1740
   tcattgacca ggacgaatta gctgaggaga tagaactcaa ccatgagagt tttgcgagtc 1800
   ccaaaaagaa gatgtcttat ggagccacct cccagaattg tgaagtccag aagaaggaat 1860
   ggaaaggaca gagaggagcg acccttgatg aggaagagct gacatcctac cagaatgaag 1920
   agagaaactt ctcaactata tcctaatgtc tgagaggcac ttctgtcttc tccttacttt 1980
   agaaacagaa agtatccata cctattgcct ttcttgtagc ccagcttgcc agaggtccaa 2040
   atattgggag gggagaagat ctaaccagca acagggaaaa gagaaatatt atctttcaat 2100
   gacatgtata ggtaaggagc tgcgctcagt tgataacata gttgataata catatttttt 2160
   gaattgacag ttgacccttc tctcaaagag ctaaacttat tcagaaagga atgactagaa 2220
   gaaaaaggag acaataccat gttgttcaaa gaaacattga aggaaattgg gatgtttggc 2280
   cagaaggaat gtaaacagta gtagtagctg ccaccacatc tctagggtag ccatgcagag 2340
   gagggcttca tattcccaat aaaccccacg ttgtggcagg tgctttataa acactcttat 2400
   ttaatctcca cacctttatg acacacattt cttatcccca ttttacaacc aaggcatcta 2460
   aagcaacaag aaatgaactt gcccaaggtc atctgccagg gtcagtgctg agactgttga 2520
   agctctcaat aggtggcagt tttagggaag atttccattc agtgtaggga agacatttgt 2580
   aataatgaaa actgaaaatg gagtaattgt gagtaactca ccactttagc aggtgttggg 2640
   gaagggaaac atttgggttg atgaggcaga ggggattcaa atgtgtgaga ggctagattc 2700
   aaagaccctc agtgttctat gttatctgaa gagtcaaatg gttttgtgac tccatagttt 2760
   ttaaagtaat aagggtcaaa gactacatca gagattcaaa taggttttta aagaaaagct 2820
   aagcaagaga gccaaatttt tagaaatctg atggtcaaaa tagctgaaag cagtaaacaa 2880
   gagattggct attaaatttc aactttccat aatattaaga atgtagctaa atgatgtccc 2940
   aaactactta caaactttta agacatttaa taatttaaga agtaggttca tgtgttttct 3000
   taggtaaagt tcttctgaaa gaattttcta tttttaaaaa atgtatctct ttagcctttt 3060
   ctgctggaga ttatattagg aagtttcatc agattgtata aaattatgat tttgtatcaa 3120
   aagtattcat gatgactcta tttggaatga tattcaggga aatcacaata atatagcagt 3180
   agttatacag agaaatacta caatgaaaac atttggggca attagaccta cagttactgt 3240
   tgaaaaattc acctttgatt gcataaggca attacatgga tacttttaga tatatttaaa 3300
   attttaacat tggcatctaa agtgttattt gaaaataaaa ttattttcct gttcattgat 3360
   tttaaacatt ttattcctac tttcagaaga aaaatataat acggaaaaaa ttatagattt 3420
   acttgtagct tattattgta aagtgttttt tttttttttc taatttctcc cacatgtatt 3480
   tctggtcccc agtgatacta gctgagttgt agtgtatttt ataaatggaa taatcttggg 3540
   gaaaaattgc gattcttcat taaataatat tctttatgtc actagcatac aatttatgtt 3600
   agtagacatc tttaaatctc tttaatgagt gaatccatgc aagccccata aaacagttcc 3660
   tagcatgcag aaaatgccca cgtaaatagc tgtcatcatc attatctttt aacattttgg 3720
   gggactttcc agttgaaaag aaaacatgct atgtcatttt tatccattat ccctggaact 3780
   tattgtgaaa gttgtgctgt tttctaagta aaataaaaaa taaaaaatta gcaattta 3838
<210> 110
   <211> 21
   <212> RNA
   <213> Unknown
<220>
   <223> Zur dsRNA-Sequenz der Erfindung komplementaere Zielsequenz einer m(RNA) eines Mitglieds der VGLUT-Familie (Patentanspruch 21).
<400> 110
   aaguguacuu uaggcaaagg g 21
<210> 111
   <211> 21
   <212> DNA
   <213> Artificial Sequence
<220> <223> Sense-Strang der dsRNA, die zu der vorstehenden Zielsequenz komplementaer ist (Anspruch 22).
<220>
   <221> misc_feature
   <222> (20)..(20)
   <223> T = dT (desoxy Thymidin)
<220>
   <221> misc_feature
   <222> (21)..(21)
   <223> T = dT (desoxy Thymidin)
<400> 111
   guguacuuua ggcaaagggt t 21
<210> 112
   <211> 21
   <212> DNA
   <213> Artificial Sequence
<220> <223> Antisense-Strang der dsRNA, die zu der vorstehenden Zielsequenz komplementaer ist (Anspruch 22).
<220>
   <221> misc_feature
   <222> (20)..(20)
   <223> T = dT (desoxy Thymidin)
<220>
   <221> misc_feature
   <222> (21)..(21)
   <223> T = dT (desoxy Thymidin)
<400> 112
   cccuuugccu aaaguacact t 21

## Beschreibung

Die Erfindung betrifft kleine, insbesondere Interferenz-auslösende, doppelsträngige RNA-Moleküle (dsRNA), die gegen Mitglieder der VGLUT-Familie gerichtet sind und erfindungsgemäße dsRNA enthaltende Wirtszellen. Erfindungsgemäße dsRNA und entsprechende Wirtszellen eignen sich als Arzneimittel bzw. zur Herstellung von Arzneimitteln, insbesondere zur Behandlung von Schmerzen und anderen mit VGLUT-Familienmitgliedern bzw. dessen unphysiologischer Expression zusammenhängenden krankhaften Zuständen.

Schmerz ist nach der Definition der Internationalen Gesellschaft zum Studium des Schmerzes (IASP) ein "unangenehmes Sinnes- und Gefühlserlebnis, das mit einer akuten oder potentiellen Gewebsschädigung verknüpft ist oder mit Begriffen solcher Schädigungen beschrieben wird" (Wall and Melzack, 1999). Der Organismus reagiert dabei auf einen schmerzhaften (nozizeptiven) Reiz mit einer komplexen Reaktion, an der sensorisch-diskriminative, kognitive, affektive, autonome und motorische Komponenten beteiligt sind. Während akuter Schmerz eine physiologische Schutzreaktion darstellt und für das Überleben eines Individuums von vitaler Bedeutung ist, hat chronischer Schmerz demgegenüber keine eindeutige biologische Funktion. Nozizeptiver Schmerz wird durch noxische Reize, wie Hitze, mechanische Stimulation, Protonen oder Kälte, an spezialisierten hochschwelligen sensorischen Apparaten, den Nozizeptoren, ausgelöst und in Form von elektrischer Aktivität durch unmyelinisierte C-Fasern oder schwach myelinisierte Aδ-Fasern ins Hinterhorn des Rückenmark geleitet. Die Nozizeptoren sind hierfür mit spezifischen Rezeptoren und Ionenkanälen ausgestattet (Scholz and Woolf, 2002).

Geschädigtes oder verletztes Gewebe, Entzündungen oder Tumorzellen können Signale für Nozizeptoren darstellen, sie führen zur Freisetzung chemischer Mediatoren aus Entzündungszellen, Blutgefäßen und aus den afferenten Terminalen, die entweder selbst zu einer Aktivierung der Nozizeptoren führen (z.B. durch Bradykinin), oder das Reiz-Antwort-Verhalten nozizeptiver Afferenzen, beispielsweise durch Erniedrigung der Aktivierungsschwelle, verändern (z.B. durch Prostaglandine, Interleukine, NGF) und so eine Sensitivierung der Nozizeptoren bewirkt (Scholz and Woolf, 2002).

Die Stärke des Schmerzes wird durch die Anzahl der Impulse pro Zeiteinheit kodiert. Bei nozizeptiver peripherer Reizung werden die raschen synaptischen Antwortkomponenten (5 - 20 msec) und monosynaptischen Reflexantworten im Rückenmark insbesondere, durch AMPA- und Kainat-Rezeptoren vermittelt, während NMDA- und metabotrope Glutamatrezeptoren eine besondere Beteiligung an späten, längeranhaltenden (20 - 150 msec) und polysynaptisch vermittelten Antwortkomponenten besitzen (Tölle, 1997).

Bei der Entstehung chronischer Schmerzen spielt die Glutamatfreisetzung im Hinterhorn des Rückenmarks eine entscheidende Rolle (Baranauskas und Nistri, 1998; Zhuo, 2001). Die Freisetzung von Glutamat führt zu einer Aktivierung der Glutamatrezeptoren (AMPA, Kainat, mGlu-R, NMDA). Proteine, die an der Glutamat-Freisetzung beteiligt sind, stellen somit für die Schmerzforschung interessante Targets dar.

Glutamat gehört zu einer der wichtigste n exzitatorischen Neurotransmitter im Nervensystem der Wirbeltiere. Die nicht-essentielle Aminosäure Glutamat kann die Blut-Hirn-Schranke nicht überwinden und wird daher im Gehirn aus Glukose und einer Vielzahl anderer Vorstufen synthetisiert.

Die Bildung von Glutamat in den exzitatorischen Nervenendigungen wird durch das Enzym Phosphat-aktivierte Glutaminase (PAG) aus Glutamin katalysiert. Der vesikuläre Glutamat-Transporter (VGLUT) verpackt das Glutamat in Vesikel und setzt das Glutamat nach Ankunft eines depolarisierten Aktionspotentials durch Kalziumeinstrom aus den Vesikeln in den synaptischen Spalt frei. Vom synaptischen Spalt wird das Glutamat teilweise durch einen Plasmamembrantransporter für exzitatorische Aminosäuren (EAATs) zurück in die exzitatorischen Terminalen transportiert, wo es erneut in Vesikeln verpackt wird.

Demnach sind am Transport von Glutamat Transporterproteine aus 2 Superfamilien beteiligt: Plasmamembrantransporter und der vesikuläre Membrantransporter (Disbrow et. al., 1982; Shioi et al., 1989; Tabb et al., 1992).

Drei vesikuläre Glutamattransporter VGLUT1 (SLC17A6), VGLUT2 (SLC17A7) und VGLUT3 (SLC17A8) wurden als Mitglieder der SLC-17 Transporterfamilie (Typ I Phosphat-/Vesikulärer Glutamattransporter) identifiziert, die den Transport organischer Anionen vermittelt und zunächst als Na⁺-abhängige Phosphat-Transporter identifiziert wurden. VGLUT1, VGLUT2 und VGLUT3 tragen die Sammelbezeichnung VGLUT. Bei den Proteinen der SLC17-Familie handelt es sich um mehrspannige Transmembranproteine mit 6 bis 12 hypothetischen Transmembrandomänen, wobei die drei vorgenannten Glutamattransporter als Subfamilie der SLC17-Familie auftreten. Die drei vorgenannten VGLUTs weisen in ihrer Aminosäuresequenz untereinander eine sehr hohe Homologie auf (Takamori et. al., 2002). Die cDNA-Sequenz von humaner VGLUT2 findet sich in den Datenbanken unter Genbank Accession-Nummer NM_020346. Die Aminosäure-Sequenz von humaner VGLUT2, findet sich in den Datenbanken unter NP_065079. Die cDNA-Sequenz von VGLUT2, Ratte, findet sich in den Datenbanken unter NM_053427. Die Aminosäure-Sequenz von VGLUT2, Ratte, findet sich in den Datenbanken unter NP_445879. Die cDNA-Sequenz von VGLUT2, Maus, findet sich in den Datenbanken unter AN: BC038375. die Aminosäure-Sequenz von VGLUT2, Maus, findet sich in den Datenbanken unter AAH38375.

Die kortikalen Schichten des Großhirns weisen einen starken mRNA-Expressionspiegel für VGLUT1 auf, wohingegen VGLUT2 mRNA insbesondere in der Schicht IV des Cortex detektiert werden konnte. Die VGLUT3 Expression ist beispielweise in den inhibitorischen Zellen in der Schicht II des parietalen Kortex, oder in GAD-positiven Interneuronen im Stratum radiatum von CA1-CA3 des Hippokampus lokalisiert. Weiterhin konnte VGLUT1 und VGLUT2 nur in den Nervenendigungen nachgewiesen, während VGLUT3 nicht nur in den synaptischen Vesikeln, sondern auch in vesikulären Strukturen von Astrozyten und neuronalen Dendriten nachgewiesen wurde (Fremeau et. al., 2002).

VGLUT1 und VGLUT2 werden in zwei getrennten Populationen im Spinalganglion exprimiert, wobei eine dritte Subpopulation eine Koexpression für VGLUT1 und VGLUT2 aufweist. VGLUT2-mRNA wird dabei überwiegend von kleinen und mittleren, VGLUT1-mRNA dagegen eher von mittleren und großen DRG-Neuronen (Hinterwurzelganglion) exprimiert. Vereinzelt kommen auch VGLUT3-mRNA exprimierende Neurone im Spinalganglion vor (Oliveira et. al., 2003; Todd et. al., 2003).

Sowohl VGLUT1, als auch VGLUT2 lassen sich auf Proteinebene in der grauen Substanz des Rückenmarks nachweisen (Varoqui et. al., 2002). Die Dominanz von VGLUT2 im oberflächlichen Hinterhorn spricht für eine prominente Rolle in der Schmerzübertragung. Die Dominanz von VGLUT1 im tiefen Hinterhorn spricht für eine Rolle in der Propriozeption. Damit ist insbesondere VGLUT2 aber auch VGLUT1 ein Schmerztarget (Varoqui et al, 2002).

Die effektive Behandlung von Schmerz ist eine große Herausforderung für die molekulare Medizin. Akuter und transitorischer Schmerz ist ein wichtiges Signal des Körpers, um den Menschen vor schwerem Schaden durch die Umgebung oder Überbelastung des Körpers zu bewahren. Im Gegensatz dazu hat der chronische Schmerz, der länger als die Ursache des Schmerzes und der erwartungsgemäße Zeitrahmen der Heilung ist, keine bekannte biologische Funktion und betrifft Hunderte von Millionen Menschen weltweit. Rund 7,5 Mio. Menschen leiden allein in der Bundesrepublik Deutschland an chronischen Schmerzen. Unglücklicherweise ist die pharmakologische Behandlung des chronischen Schmerzes noch unbefriedigend und bleibt daher eine Herausforderung für die aktuelle medizinische Forschung. Die derzeit existierenden Analgetika sind häufig nicht ausreichend wirksam und haben z. T. schwere Nebenwirkungen.

Aufgrund der Funktion und des Expressionsprofils stellen die VGLUT-Proteine im allgemeinen, VGLUT2 aber im besonderen, einen interessanten Ansatzpunkt als Target für neue Schmerzmedikamente (Varoqui et al, 2002) dar.

Bekannt sind beispielsweise aus der Schmerzmittelforschung etliche Substanzen, die die Aktivität oder Expression von VGLUT modulieren können (Carrigan et al., 2002; Roseth et al., 1995; Roseth et al., 1998). Allerdings wirken diese nicht subtypspezifisch und es würden therapeutische Ansätze sowohl durch die Verfügbarkeit der Inhibitoren im Nervensystem und an den synaptischen Vesikeln, als auch durch die unspezifische Wirkung an allen drei VGLUTs limitiert.

Die Aufgabe der vorliegenden Erfindung ist es, weitere Substanzen zur Verfügung zu stellen, die die Wirkung der VGLUTs, also bspw. VGLUT1, VGLUT2 und VGLUT3 selektiv und effizient modulieren können, und ggf. Zellpermeabilität besitzen. Weiterhin ist es eine Aufgabe der vorliegenden Erfindungen, derartige Substanzen für therapeutische Zwecke nutzbar zu machen, insbesondere für die Schmerzbehandlung.

Die vorstehend genannte Aufgabe wird durch die in den Ansprüchen gekennzeichneten Gegenstände der vorliegenden Erfindung gelöst. Erfindungsgemäß werden zur Lösung der Aufgabe VGLUT-spezifische dsRNAs zur Verfügung gestellt, die das Phänomen der RNA-Interferenz auszulösen vermögen.

Die Erfindung betrifft VGLUT-spezifische si-RNA-Moleküle mit einer Sequenz, die ausgewählt ist aus und

Die allgemeine Struktur besteht aus einer doppelsträngigen RNA mit einem aus Ribonucleotiden aufgebauten Makromolekül, wobei das Ribonukleotid aus einer Pentose (Ribose), einer organischen Base und einem Phosphat besteht. Hierbei bestehen die organischen Basen in der RNA aus den Purinbasen Adenin (A) und Guanin (G) sowie den Pyrimidinbasen Cytosin (C) und Uracil (U). Die dsRNA enthält Nukleotide mit einer gerichteten Struktur mit Überhängen. Derartige doppelsträngige erfindungsgemäße RNAs können das Phänomen der RNA-Interferenz auslösen (siRNAs).

Auf das Phänomen der RNA-Interferenz als immunologisches Abwehrsystem wurde man im Zuge der immunologischen Forschung bei höheren Eukaryonten aufmerksam.

Das System wurde ursprünglich unabhängig voneinander in verschiedenen Spezies, zuerst in C. elegans (Fire et. al., 1998), beschrieben, ehe man die zugrundeliegenden Mechanismen auf bestimmten Ebenen der Prozesse als identisch identifizieren konnte: RNA-vermittelte Virus-Resistenz in Pflanzen (Lindbo and Dougherty, 1992), PTGS (posttranscriptional gene silencing) bei Pflanzen (Napoli et. al., 1990) und RNA-Interferenz bei Eukaryonten basieren demnach auf einer gemeinsamen Funktionsweise (Plasterk, 2002).

Die *in-vitro*-Technik der RNA-Interferenz (RNAi) beruht auf doppelsträngigen RNA-Molekülen (dsRNA), welche die sequenzspezifische Suppression der Genexpression auslösen (Zamore (2001) Nat. Struct. Biol. 9: 746-750; Sharp (2001) Genes Dev. 5:485-490: Hannon (2002) Nature 41: 244-251). Die Aktivierung von Proteinkinase R und RNaseL bewirkte bei der Transfektion von Säugerzellen mit langer dsRNA unspezifische Effekte, wie z.B. eine Interferon-Antwort (Stark et. al. (1998) Annu. Rev. Biochem. 67: 227-264; He und Katze (2002) Viral Immunol. 15: 95-119). Diese unspezifischen Effekte werden bei Verwendung von kürzerer, bspw. 21- bis 23-merer, sog. dsRNA (engl. "small interfering RNA") umgangen, da unspezifische Effekte durch dsRNA, die kürzer als 30 bp ist, nicht ausgelöst werden (Elbashir et. al. (2001) Nature 411: 494-498). Kürzlich wurden dsRNA-Moleküle auch *in-vivo* zur Anwendung gebracht (McCaffrey et. al. (2002), Nature 418: 38-39; XIa et. al. (2002), Nature Biotech. 20: 1006-1010; Brummelkamp et. al. (2002), Cancer Cell 2: 243-247.

Im Rahmen der vorliegenden Erfindung werden dsRNAs (si-RNA-Moleküle), gerichtet gegen definierte Mitglieder der VGLUT-Familie offenbart. Bei diesen dsRNAs handelt es sich erfindungsgemäß um die vorstehend definierten Sequenzen. DsRNA kann in Form (i) einer siRNA, (ii) einer langen dsRNA, die eine oder mehr identische oder verschiedene siRNA(s) in der langen dsRNA-Sequenz enthält, oder (iii) einer siRNA-basierten Haarnadel RNA oder (iv) einer miRNA-basierten Haarnadel-siRNA vorliegen.

Während (i) siRNA (typischerweise chemisch synthetisiert und dann intrazellulär unter Umgehung des Dicing-Schritts in den RISC-Komplex inkorporiert, wodurch eine sequenzspezifische mRNA-Degradation (der Zielsequenz) einsetzt) im folgenden näher beschrieben ist, handelt es sich bei (ii), also einer langen dsRNA (ds: doppelsträngig), um eine Vorstufe der siRNA (gemäß (i), die erst durch einen Dicing-Schritt (Enzym: Dicer) in reife siRNA prozessiert wird. Auch diese Vorstufe der siRNA, die typischerweise erst Intrazellulär in reife siRNA umgesetzt wird, erfüllt die Voraussetzungen für den Einsatz als bspw. Arzneimittel bzw. zur Herstellung eines Arzneimittels zur Behandlung der in der vorliegenden Anmeldung genannten Indikationen.

Es entstehen diesbezüglich also aus größeren VGLUT-dsRNA-Molekülen, insbesondere VGLUT1-, -2- oder -3-spezifischen dsRNA-Molekülen (bevorzugt >30 bp, stärker bevorzugt >40 bp und noch stärker bevorzugt >50 bp), nach der Dicer-Prozessierung mehrere verschiedene siRNAs, die eine unterschiedliche Effizienz aufweisen können. Lange (ggf. haarnadelförmige) dsRNA-Moleküle, die intrazellulär nach Dicer-Prozessierung zu verschiedenen siRNAs umgewandelt werden, können auch (abgesehen von chemischer Synthese) vektorbasierend unter Kontrolle eines Pol II Promotors in einer Zelle exprimiert werden. Der Pol II Promotor erlaubt hierbei eine induzierbare, gewebe- oder zelltypspezifische Expression (Kennerdell and Carthew, 2000). Somit ermöglicht diese Anwendungsform eine induzierbare und transiente, simultane Expression einer großen Anzahl von siRNAs, die von einer Vorläufer-dsRNA abstammen.

Derartige ds-RNA-Moleküle können durch genetische Manipulationstechniken, wie die homologe Rekombination von Stammzellen, einen bestimmten Phänotyp ausbilden.

Auch Modifikationen von siRNA gemäß (i), nämlich siRNA-basierte Haamadel-Schleifen (iii) können eingesetzt werden. Derartige Haarnadeln können vorzugsweise an einem, ggf. aber auch an beiden Enden des siRNA-Doppelstrangs auftreten. Hierbei handelt es sich typischerweise um mindestens 5, stärker bevorzugt um mindestens 8 und noch stärker bevorzugt mindestens 10 Nukleotide, die auf Grund von Modifikationen und/oder In Ermangelung entsprechender Komplementarität keine Doppelstrang-Interaktionen ausbilden, sondern eine Schleife ausbilden, die die beiden Stränge zunächst kovalent miteinander verbindet. Durch entsprechende Enzyme (bspw. Dicer), bspw. intrazellulär, kann eine derartige siRNA-basierte Haarnadel-RNA in aktive siRNA weiterprozessiert werden. Schließlich sind auch (iv) miRNA-basierte Haarnadel-RNAs, gerichtet gegen Sequenzen der VGLUT-Familie, möglich.

Hierbei handelt es sich um nicht-perfekt komplementäre siRNA, vorzugsweise mit mindestens einer Haarnadel am Terminus (an den Termini). Derartige nicht-perfekt komplementäre Duplex-Stränge von mRNA weisen mindestens eine Fehlpaarung, vorzugsweise zwischen 1 und 4 Fehlpaarungen im Duplexstrang auf.

Die Wirkung von miRNA-basierter Haarnadel-RNA beruht auf deren enzymatischer Prozessierung (bspw. durch Dicer) zu miRNA(s), nachfolgender Inkorporation derselben in miRNPs und zuletzt deren Translationsinhibition.

Die jeweilige Länge der Duplex-Stränge gemäß den Ausführungsformen (i), (iii) und (iv) unterscheidet sich nicht und alle Ausführungsformen können chemisch synthetisiert werden.

In einer eukaryontischen Zelle wird für die Herstellung einer mRNA das Gen in seiner gesamten Länge, sowohl Introns als auch Exons, in ein langes RNA-Molekül transkribiert, das Primärtranskript. Die Stabilität der zellulären mRNA wird durch Prozessierung des Primärtranskripts am 5'-Ende mit einer Addition eines untypischen Nukleotids mit einem methylierten Guanin und einer Polyadenylierung am 3'-Ende sichergestellt. Bevor die RNA den Zellkern verlässt, werden durch RNA-Spleißen die intronsequenzen entfernt und die Exons miteinander verbunden.

Zielsequenzen für erfindungsgemäße dsRNA können sowohl das Primärtranskript als auch die prozessierte mRNA sein. Das Primärtranskript und die mRNA werden im folgenden zusammenfassend als (m)RNA bezeichnet.

Die erfindungsgemäßen si-RNA-Moleküle sind gegen definierte Regionen im codierenden Bereich (m)RNA eines Mitglieds der VGLUT-Familie gerichtet.

Die erfindungsgemäßen (doppelsträngigen) siRNAs weisen daher am Terminus mindestens eines Strangs die Sequenz TT auf, vorzugsweise überhängend gegenüber dem Terminus des komplementären anderen Strangs. Der komplementäre andere Strang der erfindungsgemäßen siRNA entspricht in seiner Sequenz an einem Terminus dann typischerweise den bspw. oben genannten Sequenzen hinter AA (wobei T gegenüber den obigen Zielsequenzen durch U in der erfindungsgemäßen siRNA ersetzt wird) und am anderen Terminus weist er typischerweise ein überstehendes TT auf (s. auch Ausführungsbeispiel 4).

Vorzugsweise jedoch wird eine effektive Blockierung und Spaltung der (m)RNA eines Mitglieds der VGLUT-Familie besonders dadurch erreicht, dass für die Wahl der Zielsequenz von erfindungsgemäßen dsRNAs gewisse Auswahlregeln berücksichtigt werden.

So weisen die erfindungsgemäßen dsRNAs einen GC-Gehalt von mindestens 30%, insbesondere von 30% bis 70% und speziell von 40% to 60% auf.

Vorzugsweise können die Enden der doppelsträngigen RNA (dsRNA) modifiziert werden, um einem Abbau in der Zelle oder einer Dissoziation in die Einzelstränge entgegenzuwirken, insbesondere um einen vorzeitigen Abbau durch Nukleasen zu umgehen.

Eine regelmäßig nicht gewünschte Dissoziation der Einzelstränge von dsRNA tritt insbesondere bei Verwendung niedriger Konzentrationen oder kurzer Kettenlängen auf. Zur besonders wirksamen Hemmung der Dissoziation kann der durch die Nukleotidpaare bewirkte Zusammenhalt der doppelsträngigen Struktur von erfindungsgemäßer dsRNA durch mindestens eine, vorzugsweise mehrere, insbesondere 2 bis 5, chemische Verknüpfung/en erhöht werden. Eine erfindungsgemäße dsRNA, deren Dissoziation vermindert ist, weist eine höhere Stabilität gegen enzymatischen und chemischen Abbau in der Zelle bzw. im Organismus oder *ex-vivo* auf.

Die chemische Verknüpfung der Einzelstränge einer erfindungsgemäßen dsRNA wird zweckmäßigerweise durch eine kovalente oder ionische Bindung, Wasserstoffbrückenbindung, hydrophobe Wechselwirkung, vorzugsweise van-der-Waals oder Stapelungswechselwirkungen, oder durch Metall-ionenkoordination gebildet. Sie kann nach einem besonders vorteilhaften Ausgestaltungsmerkmal an mindestens einem, vorzugsweise an beiden, Ende/n hergestellt werden. Es hat sich weiter als vorteilhaft erwiesen, dass die chemische Verknüpfung mittels einer oder mehrerer Verbindungsgruppen gebildet wird, wobei die Verbindungsgruppen vorzugsweise Poly-(oxyphosphinicooxy-1,3-propan-diol)- und/oder Polyethylenglycol-Ketten sind. Die chemische Verknüpfung kann auch durch in der doppelsträngigen Struktur anstelle von Purinen benutzte Purinanaloga gebildet werden. Von Vorteil ist es ferner, dass die chemische Verknüpfung durch in der doppelsträngigen Struktur eingeführte Azabenzoleinheiten gebildet wird. Sie kann außerdem durch in der doppelsträngigen Struktur anstelle von Nukleotiden benutzte verzweigte Nukleotidanaloga gebildet werden.

Es hat sich als zweckmäßig erwiesen, dass zur Herstellung der chemischen Verknüpfung mindestens eine der folgenden Gruppen benutzt wird: Methylblau; bifunktionelle Gruppen, vorzugsweise Bis-(2-chlorethyl)-amin; N-acetyl-N'-(p-glyoxybenzoyl)-cystamin; 4-Thiouracil; Psoralen. Ferner kann die chemische Verknüpfung durch an den Enden des doppelsträngigen Bereichs angebrachte Thiophosphoryl-Gruppen gebildet werden. Vorzugsweise wird die chemische Verknüpfung an den Enden des doppelsträngigen Bereichs durch Tripelhelix-Bindungen hergestellt. Die chemische Verknüpfung kann zweckmäßigerweise durch ultraviolettes Licht induziert werden.

Die Modifizierung der Nukleotide der dsRNA führt zu einer Deaktivierung einer RNA(doppelsträngig)-abhängigen ("RNA-dependent") Proteinkinase (PKR) in der Zelle. Die PKR induziert Apoptose. Vorteilhafterweise ist mindestens eine 2'-Hydroxygruppe der Nukleotide der dsRNA in der doppelsträngigen Struktur durch eine chemische Gruppe, vorzugsweise eine 2'-Amino- oder eine 2'-Methylgruppe, ersetzt. Mindestens ein Nukleotid in mindestens einem Strang der doppelsträngigen Struktur kann auch ein sogenanntes "locked nucleotide" mit einem, vorzugsweise durch eine 2'-O, 4'-C-Methylenbrücke, chemisch modifizierten Zuckerring sein. Vorteilshafterweise sind mehrere Nukleotide "locked nucleotides".

Modifizierungen der Nukleotide von erfindungsgemäßen dsRNA betrifft vor allem die Dissoziation der Nukleotide durch Verstärkung der Wasserstoffbrückenbindung. Die Stabilität der Nukleotide wird erhöht und gegen einen Angriff von RNAsen geschützt.

Eine weitere Möglichkeit zur Verhinderung frühzeitiger Dissoziation erfindungsgemäßer dsRNA in der Zelle besteht durch die Ausbildung der Haarnadelschleife. In einer besonderen Ausführungsform weist eine erfindungsgemäße dsRNA eine Haarnadelstruktur auf, um die Dissoziations- kinetik zu verlangsamen. Bei einer derartigen Struktur ist vorzugsweise am 5'- und/oder 3'-Ende eine Loopstruktur ausgebildet.Eine derartige Loopstruktur weist keine Wasserstoffbrücken auf.

Darüber hinaus kann durch Modifizierung des Rückgrates der erfindungsgemäßen dsRNA ein vorzeitiger Abbau verhindert werden. Insbesondere bevorzugt ist dsRNA, die modifiziert ist (z.B. Phosphorthioat, 2'-O-Methyl-RNA, LNA, LNA/DNA-Gapmeren) und daher eine längere Halbwertszeit *in-vivo* aufweist.

Eine erfindungsgemäße dsRNA ist gegen die (m)RNA der VGLUT2-Familie gerichtet, insbesondere von Säugern, wie Mensch, Affe, Ratte, Hund, Katze, Maus, Kaninchen, Meerschweinchen, Hamster, Rind, Schwein, Schaf und Ziege.

Vorzugsweise unterdrückt ein erfindungsgemäßes si-RNA-Molekül die Expression von VGLUT2 in der Zelle mindestens zu 50%, 60%, 70%, ganz besonders bevorzugt mindestens zu 90%.

Die Messung der Unterdrückung kann über Northern-Blot, quantitative Real-Time PCR oder auf Proteinebene mit VGLUT2 spezifischen Antikörpern erfolgen.

Die dsRNA wird nach dem Fachmann bekannten Verfahren hergestellt, dabei werden Nukleotide, insbesondere auch Oligonukleotide, beispielsweise nach Art der Merryfield-Synthese, an einem unlöslichen Träger (H.G. Gassen, Chemical and Enzymatic Synthesis of Genfragments (Verlag Chemie, Weinheim 1982)) oder auf andere Art synthetisiert (Beyer/Walter, Lehrbuch der Organischen Chemie, 20. Auflage, (S. Hirzel Verlag, Stuttgart 1984), S. 816 ff.). Die Gewinnung der VGLUT-mRNA kann durch Hybridisierung mittels Genom- und cDNA-Datenbanken erreicht werden. Erfindungsgemäße dsRNA-Moleküle, insbesondere siRNA-Moleküle, können bspw. synthetisch hergestellt werden und ggf. auch von verschiedenen Anbietern, bspw. IBA GmbH (Göttingen, Deutschland), bezogen werden.

Erfindungsgemäße doppelsträngige RNA (dsRNA) kann in micellare Strukturen eingeschlossen sein, die die Separation von Substanzgruppen *In vitro* und *in vivo* beeinflussen. Hierbei liegt die dsRNA vorzugsweise in Liposomen vor. Die Liposomen sind künstliche, kugelig in sich geschlossene Membranen, aus Phospholipiden, in die sowohl hydrophile Substanzen in den wässrigen Innenraum verkapselt, als auch lipophile Substanzen in den Innenbereich der Lipidmembran inkorporiert werden können. Die Voraussetzung für die Verwendung von Liposomen für experimentelle oder therapeutische Zwecke ist deren Verträglichkeit mit Zellen und Geweben. Die dsRNA, die vorzugsweise in den Liposomen vorliegt, kann mit einer Peptidsequenz, vorzugsweise mit einer Lysin- und Arginin-reichen Sequenz, bspw. einer Sequenz aus dem viralen TAT-Protein (bspw. enthaltend AS 49-57) modifiziert sein, um dann als Transporterpeptid die Zellmembran leichter zu überwinden.

Die dsRNA kann gleichfalls in virale natürliche Kapside oder in auf chemischem oder enzymatischem Weg hergestellte künstliche Kapside oder davon abgeleitete Strukturen eingeschlossen sein. Die genannten Merkmale ermöglichen ein Einschleusen der dsRNA in vorgegebene Zielzellen.

Eine gegenüber siRNA alternative Ausgestaltung der VGLUT-dsRNAs, sind mikroRNAs (s.o.) mit mindestens einer Haarnadelschleife, durch die die beiden nicht-perfekt komplementären Strängen miteinander kovalent verbunden werden (miRNA-basierte Haarnadel-RNA)(s, auch Schwarz et. al., 2002). Diese VGLUT-miRNAs werden von den Zellen selbst transkribiert und führen nach sequenzspezifischer Bindung an die mRNA, nicht zu einer mRNA-Degradation, sondern induzieren lediglich eine Translationsrepression. Erfindungsgemäße VGLUT-miRNAs werden als mindestens 50, bevorzugt zwischen 60 und 80, ganz besonders bevorzugt zwischen 65 und 75 Nukleotid-lange Vorläufer transkribiert und bilden eine charakteristische "Haarnadelstruktur" aus. Das Enzym Dicer schneidet in der Zelle aus diesen Vorläufern einen 21 bis 23 Nukleotid-langen doppelsträngigen Bereich heraus, der in weiteren Schritten entwunden wird. Hierdurch kann bspw. die reife miRNA in ein miRNP-Partikel inkorporiert werden. Diese Partikel können dann eine spezifische Translationsrepression der komplementären mRNA vermitteln. Der Grad der Komplementarität zur Ziel-mRNA entscheidet darüber, ob die gebildete DNA-Duplex als miRNA oder siRNA wirkt.

Erfindungsgemäß ermöglichen zahlreiche Vektorsysteme den Einsatz von miRNAs für eine nachfolgende stabile und regulierte Transkription der entsprechenden VGLUT-siRNAs. Die Transkription der miRNAs kann von Polymerase III Promotoren (z. B. H1- oder U6-Promotoren) und auch von Polymerase II Promotoren gesteuert werden (Brummelkamp et. al., 2002; Lee et. al., 2002; Miyagishi and Taira, 2002). Dabei können die Sense- und Antisense-Stränge von verschiedenen Promotoren abgelesen werden und sich in der Zelle zu 19-nt Duplizes mit 4-nt Überhängen zusammenlagern (B) (Lee et. al., 2002), oder die Expression von Haarnadelstrukturen wird benutzt (Brummelkamp et. al., 2002).

Vorzugsweise werden virale Vektoren, z. B. retrovirale oder Adenovirusabgeleitete Vektoren für diese Vektorsysteme benutzt. Virale Vektoren besitzen eine sehr effiziente und gezielte Transduktion spezifischer Zellen, einschließlich primärer Zellen und ermöglichen damit ein erweitertes Anwendungsspektrum, bspw. in der Schmerztherapie.

Nach einer weiteren besonders vorteilhaften Ausgestaltung ist vorgesehen, dass die dsRNA an mindestens ein von einem Virus stammendes, davon abgeleitetes oder ein synthetisch hergestelltes virales Hüllprotein gebunden, damit assoziiert oder davon umgeben wird. Das Hüllprotein kann vom Polyomavirus abgeleitet sein. Es kann sich also bspw. um das Virus-Protein 1 (VP1) und/oder das Virus-Protein 2 (VP2) vom Polyomavirus handeln. Die Verwendung derartiger Hüllproteine ist z.B. aus der DE 19618797 A1 bekannt. Die vorgenannten Merkmale erleichtern wesentlich das Einführen der dsRNA in die Zelle.

In einer bevorzugten Ausführungsform erfolgt die Expression der erfindungsgemäßen dsRNA dadurch, daß das 1. Template (Sense-dsRNA) und das 2. Template (Antisense-dsRNA) unter der Kontrolle von zwei identischen oder verschiedenen Promotoren stehen. Hierbei erfolgt die Expression *in vivo* und wird gentherapeutisch über Vektoren in die Zellen gebracht.

Ein weiterer Gegenstand der vorliegenden Erfindung ist ein Arzneimittel, enthaltend mindestens eine erfindungsgemäße dsRNA und/oder eine diese enthaltende Zelle, sowie gegebenenfalls geeignete Hilfs- und/oder Zusatzstoffe.

Arzneimittel: ein Stoff entsprechend der Definition im Artikel 1 §2 des deutschen Gesetzes über den Verkehr mit Arzneimitteln (AMG). Das heißt, Stoffe oder Zubereitungen aus Stoffen, die dazu bestimmt sind, durch Anwendungen am oder im menschlichen oder tierischen Körper
1. Krankheiten, Leiden, Körperschäden oder krankhafte Beschwerden zu heilen, lindern, zu verhüten oder zu erkennen,
2. die Beschaffenheit, den Zustand oder die Funktionen des Körpers oder seelische Zustände erkennen zu lassen.
3. vom menschlichen Körper erzeugte Wirkstoffe oder Körperflüssigkeiten zu ersetzen,
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Die erfindungsgemäßen Arzneimittel können als flüssige Arzneiformen in Form von Injektionslösungen, Tropfen oder Säften, als halbfeste Arzneiformen in Form von Granulaten, Tabletten, Pellets, Patches, Kapseln, Pflastern oder Aerosolen verabreicht werden und enthalten neben dem mindestens einen erfindungsgemäßen Gegenstand je nach galenischer Form gegebenenfalls Trägermaterialien, Füllstoffe, Lösungsmittel, Verdünnungsmittel, Farbstoffe und/oder Bindemittel. Die Auswahl der Hilfsstoffe sowie die einzusetzenden Mengen derselben hängt davon ab, ob das Arzneimittel oral, parenteral, intravenös, intraperitonal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation, Lösungen, Suspensionen, leicht rekonstituierbare Trockenzubereitungen sowie Sprays. Erfindungsgemäße Gegenstände in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Gegenstände verzögert freisetzen. Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 2 bis 500 mg/kg wenigstens eines erfindungsgemäßen Gegenstandes appliziert. Wenn das Arzneimittel insbesondere zur Gentherapie verwendet werden soll; empfehlen sich als geeignete Hilfs- oder Zusatzstoffe beispielsweise eine physiologische Kochsalzlösung, Stabilisatoren, Proteinase-, DNAse-Inhibitoren etc.

Ein weiterer Gegenstand der vorliegenden Erfindung betrifft Wirtszellen, ausgenommen humane Keimzellen und humane embryonale Stammzellen, die mit mindestens einem erfindungsgemäßen si-RNA-Molekül transformiert sind. Erfindungsgemäße dsRNA-Moleküle können in die jeweilige Wirtszelle über übliche Methoden, bspw. Transformation, Transfektion, Transduktion, Elektroporation oder Partikel-Gun eingebracht werden. Bei der Transformation werden mindestens zwei voneinander verschiedene si-RNA-Moleküle in die Zelle eingeführt, wobei ein Strang jedes si-RNA-Moleküls zumindest abschnittsweise komplementär zur (m)RNA eines Mitglieds der VGLUT2-Familie ist. Der komplementäre Bereich der dsRNA zur (m)RNA von VGLUT2 enthält weniger als 25 aufeinanderfolgende Nukleotidpaare.

Als Wirtszellen kommen alle Zellen pro- oder eukaryontischer Natur in Betracht, bspw. von Bakterien, Pilzen, Hefen, pflanzlichen oder tierische n Zellen. Bevorzugte Wirtszellen sind Bakterienzellen, wie *Escherichia coli, Streptomyces, Bacillus* oder *Pseudomonas,* eukaryontische Mikroorganismen, wie *Aspergillus* oder Sac*charomyces cerevisiae* oder die gewöhnliche Bäckerhefe (Stinchcomb et. al. (1997) Nature 282: 39).

In einer bevorzugten Ausführungsform werden jedoch zur Transformation mittels erfindungsgemäßer dsRNA-Konstrukte Zellen aus multizellulären Organismen gewählt. Im Prinzip ist jede höhere eukaryontische Zellkultur als Wirtszelle verfügbar, wenn auch Zellen von Säugern, bspw. Affen, Ratten, Hamstern, Mäusen oder Menschen, ganz besonders bevorzugt sind. Dem Fachmann ist eine Vielzahl von etablierten Zellinien bekannt. In einer keineswegs abschließenden Aufzählung werden die folgenden Zellinien genannt: 293T (Embryonennierenzellinie) (Graham et al., J. Gen. Virol. 36: 59 (1997), BHK (Babyharnsternierenzellen), CHO (Zellen aus den Harnsterovarien, Urlaub und Chasin, Proc. Natl. Accad. Sci. USA 77: 4216, (1980)), HeLa (humane Karzinomzellen) und weitere - insbesondere für den Laboreinsatz etablierte - Zellinien, bspw. HEK293-, SF9- oder COS-Zellen, wt-PC12 und DRG-Primärkulturen. Ganz besonders bevorzugt sind humane Zellen, insbesondere neuronale Stammzellen und Zellen des "Schmerz-Weges", vorzugsweise primäre sensorische Neuronen. Humane Zellen, insbesondere autologe Zellen eines Patienten, eignen sich nach (vor allem *ex-vivo)* Transformation mit erfindungsgemäßen dsRNA-Molekülen, ganz besonders als Arzneimittel für bspw. gentherapeutische Zwecke, also nach Durchführung einer Zellentnahme, ggf. *ex-vivo* Expansion, Transformation, Selektion und abschließender Retransplantation in den Patienten.

Ein weiterer bevorzugter Gegenstand ist auch die Verwendung mindestens eines erfindungsgemäßen si-RNA-Moleküls bzw.pharmazeutischen Zusammensetzung und/oder einer erfindungsgemäßen Zelle zur Herstellung eines Arznei- bzw. Schmerzmittels, zur Behandlung von Schmerz, insbesondere von chronischem Schmerz, taktiler Allodynie, thermisch ausgelöstem Schmerz, und/oder Entzündungsschmerz.

Die erfindungsgemäßen Gegenstände eignen sich als Arzneimittel, bspw. zur Inhibition der Nozizeption, bspw. aufgrund der Verminderung der Expression von mindestens einem Mitglied der VGLUT2-Familie mittels erfindungsgemäßen si-RNA-Molekülen.

Darüber hinaus wird die Verwendung mindestens eines erfindungsgemäßen si-RNA-Moleküls und/oder einer erfindungsgemäßen Zelle zur Herstellung eines Arzneimittels zur Behandlung von Haminkontinenz; auch von neurogenen Blasensymptomen; Pruritus, Tumoren, Entzündungen; insbesondere von VGLUT-assoziierten Entzündungen mit Symptomen wie Asthma; sowie von allen mit VGLUT-Familienmitgliedern zusammenhängenden Krankheitssymptomen offenbart.

Ein weiterer Gegenstand der Erfindung ist ein Verfahren zur Behandlung, insbesondere Schmerzbehandlung, eines nichthumanen Säugetieres oder Menschen, das oder der eine Behandlung von Schmerzen, insbesondere chronischer Schmerzen, benötigt, durch Verabreichung eines erfindungsgemäßen Arzneimittels, insbesondere solche enthaltend ein erfindungsgemäßes si-RNA-Molekül. Ein weiterer Gegenstand sind auch entsprechende Verfahren zur Behandlung von Pruritus und/oder Harninkontinenz.

Ein weiterer bevorzugter Gegenstand ist ebenso die Verwendung mindestens eines erfindungsgemäßen si-RNA-Moleküls, insbesondere siRNA, und/oder einer erfindungsgemäßen Zelle für die Gentherapie, vorzugsweise *in-vivo*- oder *in*-*vitro*-Gentherapie.

Ein weiterer bevorzugter Gegenstand ist ebenso die Verwendung mindestens eines erfindungsgemäßen si-RNA-Moleküls und/oder einer erfindungsgemäßen Zelle für die Gentherapie, vorzugsweise *in-vivo-* oder *in-vitro*-Gentherapie. Unter Gentherapie versteht man eine Therapieform, bei der durch die Einführung von Nukleinsäuren in Zellen bspw. ein Effektorgen, meist ein Protein, exprimiert wird, im vorliegenden Fall also insbesondere erfindungsgemäße dsRNA.

Man unterscheidet prinzipiell *in-vivo-* und *in-vitro*-Verfahren. Im Falle von *in-vitro-*Verfahren werden Zellen aus dem Organismus entfernt und *ex-vivo* mit Vektoren transfiziert, um anschließend wieder in denselben oder in einen anderen Organismus eingebracht zu werden. Bei der *in-vivo*-Gentherapie werd en Vektoren, beispielsweise zur Bekämpfung von Tumoren, systemisch (z.B. über die Blutbahn) oder direkt in den Tumor appliziert. Gemäß einer bevorzugten Ausführungsform wird ein Vektor verabreicht, der sowohl das Transkriptionselement für die Sense-dsRNA, als auch das Transkriptionselement für die Antisense-dsRNA unter der Kontrolle geeigneter Promotoren enthält. Gemäß einer weiteren bevorzugten Ausführungsform können die beiden Transkriptionselemente auf verschiedenen Vektoren liegen.

Ein weiterer bevorzugter Gegenstand ist auch ein Diagnostikum enthaltend mindestens eine dsRNA und/oder eine erfindungsgemäße Zelle sowie gegebenenfalls geeignete Zusatzstoffe. Diagnostikum bedeutet hierin eine Verbindung oder ein Verfahren, das verwendet werden kann, um eine Krankheit zu diagnostizieren.

Beschrieben ist auch ein Verfahren zur Identifizierung schmerzmodulierender Substanzen. In einem vorzugsweise vorgeschalteten Verfahrensschritt (a) erfolgt eine Überexpression von VGLUT, vorzugsweise VGLUT1, VGLUT2 oder VGLUT3, in einer Testzelle. Diese Überexpression in einer Testzelle stellt sicher, dass eine erhöhte Konzentration von VGLUT in diesen zur weiteren Untersuchung herangezogenen, manipulierten Testzellen vorliegt, so dass der Wirkungsgrad potentiell schmerzmodullerender Subtanzen durch Skalenerweiterung genauer bestimmt werden kann. Grundsätzlich können jedoch auch nicht dergestalt manipulierte Zellen, die aber gleichwohl VGLUT nativ exprimieren, für das Verfahren eingesetzt werden.

Die ggf. durch Vorschaltung von Verfahrensschritt (a) erhaltenen, vorzugsweise kultivierbaren Testzellen werden den (insbesondere parallelen) Verfahrensschritten (b) bzw. (b') unterzogen, nämlich (b) einer bevorzugt gentechnischen Manipulation mindestens einer Zelle (Testzelle) mit mindestens einer erfindungsgemäßen dsRNA und (b') einem (insbesondere parallelen) Vergleichversuch (Kontrollversuch) mit mindestens einer identischen Zelle (Kontrollzelle). Ein solcher Vergleichsversuch gemäß Verfahrensschritt (b') kann, abhängig vom gewünschten Erkenntnisgewinn, unterschiedliche Zielrichtungen verfolgen. Deswegen sind verschiedene Ausgestaltungsformen denkbar. So etwa kann der Vergleichsversuch mit Testzellen erfolgen, die - im Unterschied zu Verfahrensschritt (b) - ohne jede gentechnische Manipulation mit dsRNA eingesetzt werden. Alternativ können Kontrollzellen jedoch auch eine veränderte, bspw. nicht mehr erfindungsgemäße dsRNA aufweisen oder aber mit einer bestimmten dsRNA von bekannter Wirkung auf die VGLUT-Expression manipuliert sein. Schließlich kann Verfahrensschritt (b') ggf. auch unterbleiben. In einem Verfahrensschritt (c) erfolgt die Inkubation der Testzellen, die sowohl VGLUT exprimieren als auch gemäß Verfahrensschritt (b) die zu testende Substanz aufweisen, unter geeigneten Bedingungen. Die Inkubation der Testzellen aus Verfahrensschritt (b) und der Kontrollzellen gemäß Verfahrensschritt (b') erfolgt typischerweise parallel.

Daraufhin wird in einem Verfahrensschritt (d) bspw. die Bindung der Testsubstanz an die von den Zellen synthetisierte VGLUT-(m)RNA, vorzugsweise unter geeigneten Bedingungen, gemessen. Hierzu kann bspw. eine Präparation der mit der Testsubstanz manipulierten Testzellen erforderlich sein. Bevorzugt ist allerdings die Messung mindestens eines der durch die Bindung der Testsubstanz, typischerweise dsRNA, an bspw. die VGLUT-(m)RNA veränderten funktionellen Parameters. Dieser veränderte Parameter kann bspw. ein quantifizierbarer Phänotyp der inkubierten Zelle sein, der sich durch die Bindung der Testsubstanz an VGLUT-(m)RNA, bspw. auf Grund der durch die Bindung unterdrückten Expression des VGLUT-Proteins, einstellt. Die Messung kann auch bspw. über Immunfluoreszenzmethoden, wodurch die Konzentration von VGLUT in den Zielzellen bestimmt wird, geschehen. Ggf. kann das durch einen Verfahrensschritt (a) in der Testzelle überexprimierte VGLUT jedoch mit einer Reporterfunktion (zusätzlich) ausgestaltet sein. So wäre bspw. eine mit dem überexprimierten VGLUT durch ein entsprechendes Genkonstrukt verbundene Fluoreszenzeigenschaft (bzw. deren ggf. auftretende Unterdrückung durch die Zugabe einer positiv gestesteten Testsubstanz) direkt in der Zelle messbar. Die Identifizierung von potentiell schmerzmodulierenden Substanzen erfolgt bspw. dann über das Ausmaß der Differenz zwischen dem Meßwert bei der Testzelle und dem bei der Kontrollzelle in einem Verfahrensschritt (e).

Die gemäß Verfahrensschritt (b) bzw. (b') in Form genetischer Manipulation in die Testszellen transferierte dsRNA als typische Testsubstanz eines solchen Verfahrens kann auch durch jeden anderen alternativen Weg in die Testzellen transferiert werden. So kann bspw. die Zugabe exogen auf die Testzellen erfolgen, ggf. in Verbindung mit weiteren aus dem Stand der Technik bekannten chemischen oder physikalischen Maßnahmen, um die Aufnahme der dsRNA in die Zellen sicherzustellen, bspw. durch Elektroporation etc.. Sofern die auf die Testzellen exogen aufgebrachten dsRNA-Testsubstanzen nicht als solche zellmembrangängig sind, kann deren Zellmembrandurchtrittsvermögen auch durch entsprechende Formulierungen, bspw. in Liposomen oder durch Ankopplung von bekannten Verstärkern der Membranpenetration, bspw. entsprechenden Polymeren, bzw. Transfektionsreagenzien erhöht werden.

Dabei bezieht sich der Begriff schmerzmodulierend auf einen potentiellen regulierenden Einfluss auf das physiologische Schmerzgeschehen, insbesondere auf eine analgetische Wirkung. Der Begriff Substanz umfasst jede als Arzneimittel-Wirkstoff geeignete Verbindung, insbesondere also niedermolekulare Wirkstoffe, aber auch andere wie Nukleinsäuren, Fette, Zucker, Peptide oder Proteine wie Antikörper. Die Inkubation unter geeigneten Bedingungen ist hier so zu verstehen, dass die zu untersuchende Substanz mit der Zelle oder der entsprechenden Präparation in einem wässrigen Medium eine definierte Zeit vor der Messung reagieren kann. Dabei kann das wässrige Medium temperiert werden, beispielsweise zwischen 4°C und 40°C, vorzugsweise bei Raumtemperatur oder bei 37°C. Die Inkubationszeit kann zwischen wenigen Sekunden und mehreren Stunden variiert werden, je nach der Wechselwirkung der Substanz mit dem Protein. Bevorzugt sind aber Zeiten zwischen 1 min und 60 min. Das wässrige Medium kann geeignete Salze und/oder Puffersysteme enthalten, so dass bei der Inkubation beispielsweise ein pH zwischen 6 und 8, vorzugsweise pH 7,0 - 7,5 im Medium herrscht. Dem Medium können weiter geeignete Substanzen, wie Coenzyme, Nährstoffe etc. beigefügt werden. Die geeigneten Bedingungen können vom Fachmann in Abhängigkeit von der zu untersuchenden Wechselwirkung der Substanz mit dem Protein aufgrund seiner Erfahrung, der Literatur oder weniger, einfacher Vorversuche leicht festgelegt werden, um im Verfahren einen möglichst deutlichen Messwert zu erhalten. Eine Zelle, die ein Protein synthetisiert hat, ist eine Zelle, die dieses Protein bereits endogen exprimiert hat oder eine solche, die gentechnisch verändert wurde, so dass sie dieses Protein exprimiert und entsprechend von Beginn des beschriebenen Verfahrens das Protein enthält. Die Zellen können Zellen aus evt. immortalisierten Zellinien sein oder native aus Geweben stammende und aus diesen isolierte Zellen sein, wobei der Zellverband meist aufgelöst ist. Die Präparation aus diesen Zellen umfasst insbesondere Homogenate aus den Zellen, das Cytosol, eine Membranfraktion der Zellen mit Membranfragmenten, eine Suspension isolierter Zellorganellen etc.

Der Maßstab über den das Verfahren die Auffindung interessanter Substanzen erlaubt, ist entweder die Bindung an das Protein, die z.B. durch Verdrängung eines bekannten Liganden oder das Ausmaß gebundener Substanz nachgewiesen werden kann, oder die Veränderung eines funktionellen Parameters durch die Wechselwirkung der Substanz mit dem Protein. Diese Wechselwirkung kann insbesondere in einer Regulation, Hemmung und/oder Aktivierung von Rezeptoren, Ionenkanälen und/oder Enzymen liegen. Veränderte funktionelle Parameter können beispielsweise die Genexpression, das Ionenmilieu, der pH oder das Membranpotential bzw. die Veränderung der Enzymaktivität oder der Konzentration der 2^{nd} messenger sein. Dabei heißt:
- genetisch manipuliert: Manipulation von Zellen, Geweben oder Organismen derart, dass hier genetisches Material eingebracht wird.
- endogen exprimiert: Expression eines Proteins, die eine Zellinie unter geeigneten Kulturbedingungen aufweist, ohne das dieses entsprechende Protein durch gentechnische Manipulation zur Expression veranlasst wurde.

Eine weitere Ausführungsform dieses Verfahrens sieht vor, dass die Zelle bereits vor den Verfahrensschritten (b) und (b') gentechnisch manipuliert wird.

Eine weitere Ausführungsform dieses Verfahrens sieht vor, dass die gentechnische Manipulation die Messung mindestens eines der durch die Testsubstanz veränderten funktionellen Parameter erlaubt.

Eine weitere Ausführungsform dieses Verfahrens sieht vor, dass durch die gentechnische Manipulation eine in der Zelle nicht endogen exprimierte Form eines Mitglieds der VLGUT2-Familie exprimiert oder ein Reportergen eingeführt wird.

Eine weitere Ausführungsform dieses Verfahrens sieht vor, dass die Messung der Bindung über die Verdrängung eines bekannten markierten Liganden eines Mitglieds der VGLUT2-Familie erfolgt.

Eine weitere Ausführungsform dieses Verfahrens sieht vor, dass zwischen den parallelen Verfahrensschritten (b) und (b') und dem Verfahrensschritt (c) ≥ 8 h, vorzugsweise ≥ 12 h, insbesondere ≥ 24 h, vergehen.

Das Einbringen der erfindungsgemäßen Gegenstände in die Zelle kann dabei auf die vorstehend dargelegte Art und Weise erfolgen.

Die vorliegende Erfindung wird durch die nachfolgenden Figuren näher erläutert.

Die Figuren zeigen:

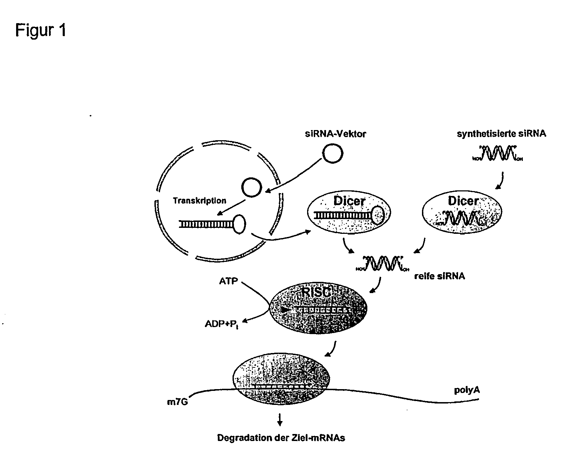

Figur 1 zeigt Strategien zur RNA-Interferenztechnik.Synthetische siRNA-Duplizes können direkt in Zellen transfiziert werden, wo sie über die zelluläre RNAi-Maschinerie die Ziel-mRNA-Degradation induzieren. Dagegen werden vektorkodierte siRNAs als haarnadelförmige Vorläufer im Zellkern gebildet und im Zytoplasma voh Dicer zur siRNA prozessiert.

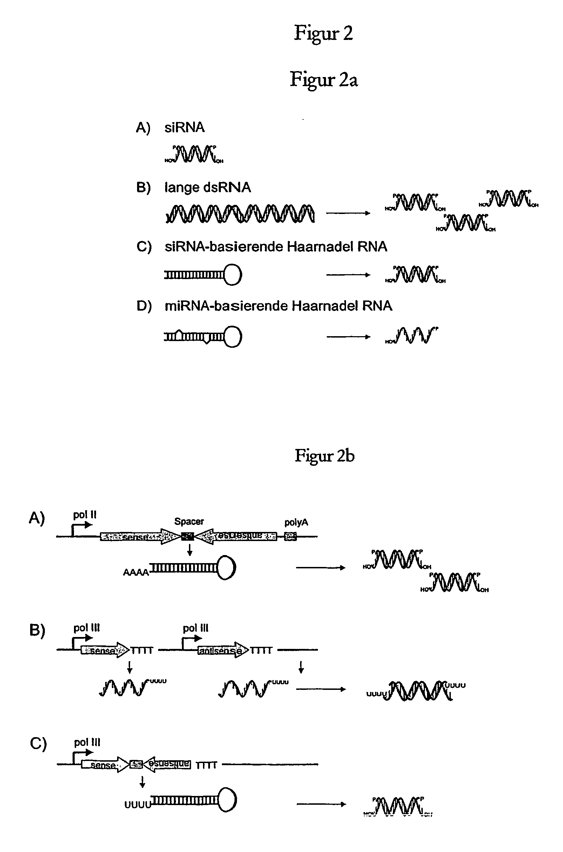

Figur 2a zeigt *In vitro* hergestellte siRNAs: Prinzipiell gibt es mehrere Möglichkeiten sich die RNAi-Technik nutzbar zu machen: Es gibt die Möglichkeit, chemisch synthetisierte siRNA einzusetzen (s. Figur 2A) oder auch durch molekularbiologische Methoden (bspw. Figur 2B).
   (A) Chemisch synthetisierte siRNA umgeht den Dicing-Schritt, inkorporiert in den RISC und führt zu einer sequenzspezifischen mRNA-Degradation. (B) Lange dsRNA wird durch Dicer in aktive siRNAs prozessiert. (C) Duplex Haarnadel-RNA kann durch Dicer in aktive siRNAs prozessiert werden. (D) Unvollkommene Duplex-Haarnadel RNA wird durch Dicer in miRNAs prozessiert, in miRNPs inkorporiert und führt zu einer Translationsinhibition. Lange, in Zellen transfizierte dsRNA-Moleküle (B) werden in kurze 19 bis 21 bp siRNA-Moleküle prozessiert, die zur Degradation von komplementären mRNA-Sequenzen führen. Die chemisch synthetisierbaren einzelsträngigen 21-mere mimen die *in vivo* gefundenen siRNAs nach und werden nach einer Duplexbildung für relativ kurze und transiente RNAi-Effekte *in vitro* und *in vivo* eingesetzt (Elbashir et al., 2001a; Holen et al., 2002; Yu et al., 2002). Damit sind vier Ausführungsformen für erfindungsgemäße dsRNA gegen VGLUT-Familienmitglieder beschrieben.
   Eine andere Möglichkeit bieten erfindungsgemäße dsRNA- (insbesondere siRNA-)-exprimierende Plasmide (Figur 2b), also *in vivo* generierte kurze RNA-Fragmente:
   (A) Lange Haarnadel-RNA durch RNA-Polymerase II exprimiert führt nach Dicer-Prozessierung zu mehreren siRNAs mit unterschiedlichsten Sequenzspezifitäten.
   (B) Tandem Pol III Promotoren sorgen für die Expression von individuellen Sense- und Antisense-Strängen, die sich in der Zelle zu aktiven siRNAs zusammenlagern. (C) Ein einzelner Pol III Promotor sorgt für die Expression einer kurzen haarnadelförmigen (sh)RNA, die durch Dicer zu aktiver siRNA prozessiert wird.

   Mit Hilfe von RNA-Polymerase III Promotoren wie U6 oder H1 ist es möglich, dsRNA und in der Folge siRNA-Moleküle intrazellulär zu exprimieren und damit stabile RNAi-Systeme in Säugetierzellen zu etablieren (Brummelkamp et al., 2002; Lee et al., 2002; Miyagishi and Taira, 2002). Dabei können entweder die Sense- und Antisense-Stränge von verschiedenen Promotoren abgelesen werden und sich in der Zelle zu 19-nt Duplizes mit 4-nt Überhängen zusammenlagern oder aber man greift auf die Expression von Haarnadelstrukturen zurück. In beiden Fällen wird durch den RISC-vermittelten RNAi-Prozess eine effektive und stabile Suppression der Genexpression erreicht. Die geringe Größe eines Transkriptes, das durch den Pol III Promotor exprimiert werden kann, ist zwar zunächst für die siRNA-Technologie nicht hinderlich, schränkt aber die Zahl der verschiedenen siRNAs, die von einem Transkript gebildet werden können, ein (Myslinski, 2001).

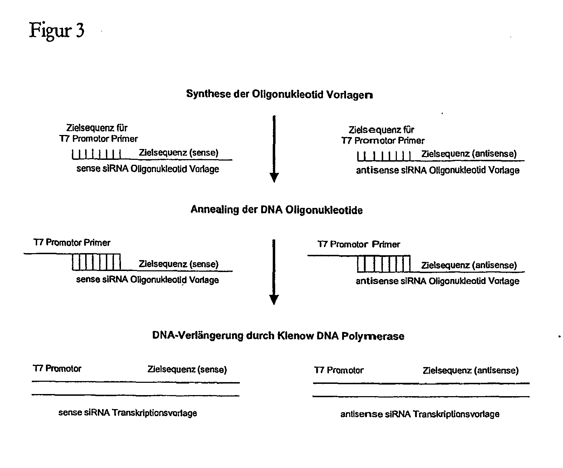

Figur 3 zeigt die Herstellung der DNA-Vorlagen für die siRNA-Synthese

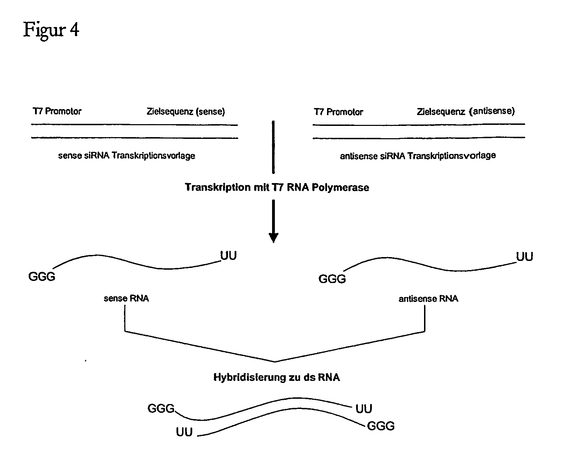

Figur 4 zeigt die Transkription und Hybridisierung der siRNA

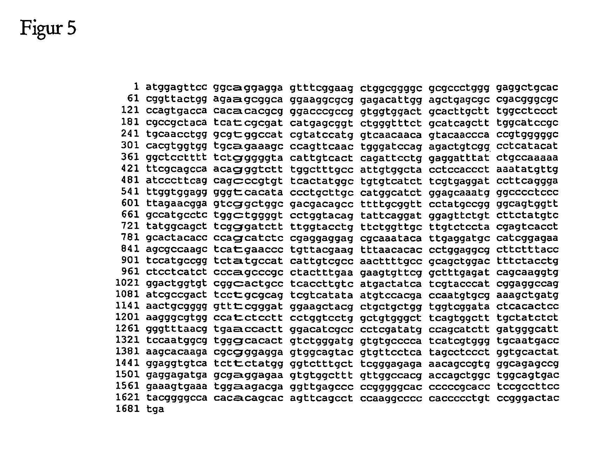

   Die Figur 5 zeigt VGLUT1 cDNA mit siRNA-Zielsequenz; Genbank Accession-Nummer U07609. Farbig unterlegt: Start-Kodon (gelb), siRNA si-rVGLUT1 739-759 EGT (rot), Primer rVGLUT1(2_4)F (hellgrau), Primer rVGLUT1(2_4)R (dunkelgrau).

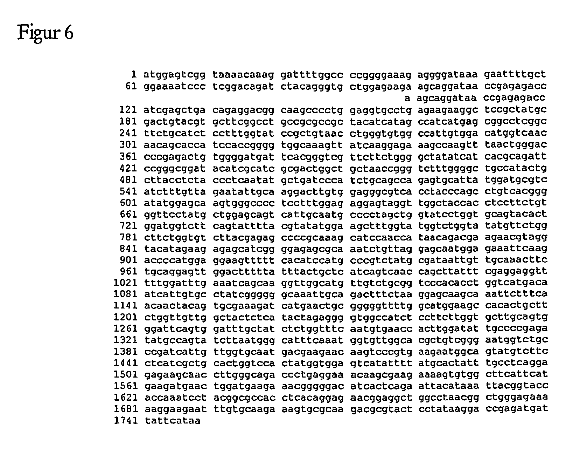

Figur 6 zeigt VGLUT2 cDNA mit siRNA-Zielsequenzen; Genbank Accession-Nummer NM_053427. Farbig unterlegt: Start-Kodon (gelb), siRNA si-rVGLUT2 100-120 EGT (rot), siRNA si-rVGLUT2 100-120 AMB (grün), siRNA si-rVGLUT2 166-186 AMB (blau), Primer rVGLUT2(8_9)F (hellgrau), Primer rVGLUT2(8_9)R (dunkelgrau).

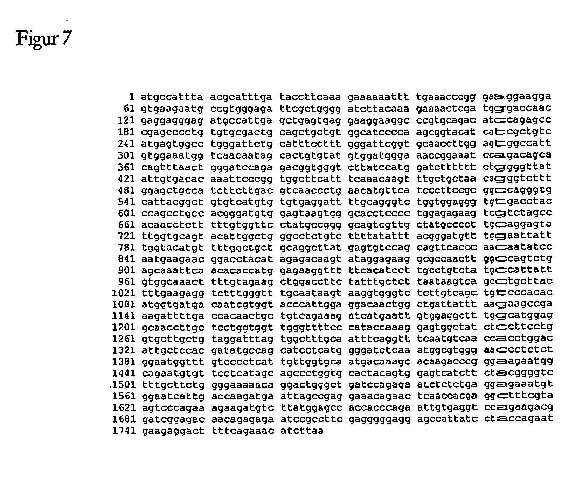

Figur 7 zeigt VGLUT3 cDNA mit siRNA-Zielsequenz; Genbank Accession-Nummer AJ491795. Farbig unterlegt: Start-Kodon (gelb), siRNA si-rVGLUT3 220-240 EGT (rot), Primer rVGLUT3(4_5)F (hellgrau), Primer rVGLUT3(4_5)R (dunkelgrau).

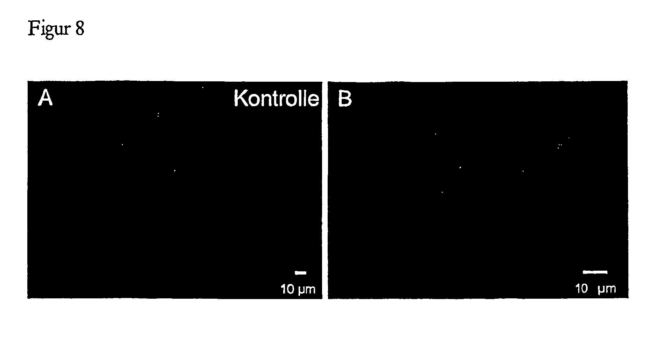

Figur 8 zeigt die Transfektion von PC12 MR-A Zellen mit Cy3-markierter siRNA; Cy3-markierte siRNA (100 pmol) wurde mittels LF2000 (1µl) in Zellen der PC12 MR-A Zelllinie transfiziert (B). Bei der Kontrolle (A) wurde auf LF2000 verzichtet. Die Zellen wurden 24 Stunden nach der Transfektion fixiert und die Zellkerne mit DAPI gegengefärbt.

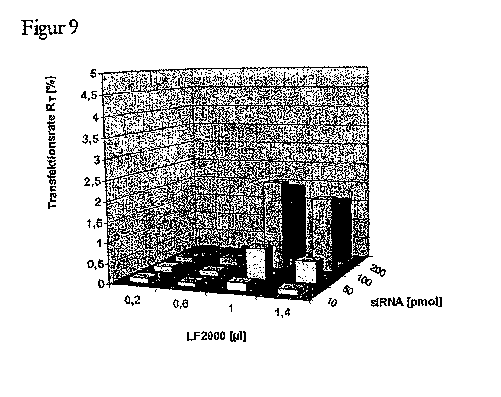

Figur 9 zeigt die Transfektionsrate bei DRG-Primärkulturen. Die Ermittlung der Transfektionsrate bei 250.000 Zellen einer DRG-Primärkultur (P0, 1 T.i.v.) wurde in einer 24-Loch-Platte unter Einsatz verschiedener Mengen Lipofectamine™ 2000 (0,2 - 1,4 µl), sowie unterschiedlicher Cy3-siRNA-Konzentrationen (10 - 200 pmol) 24 Stunden nach der Transfektion mittels Fluoreszenzmikroskopie durchgeführt.

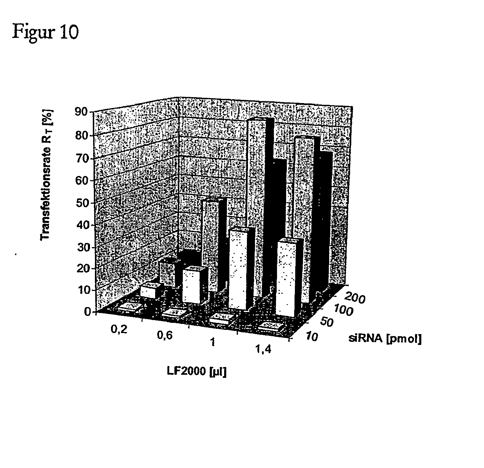

Figur 10 zeigt die Transfektionsrate bei PC12 MR-A Zellen. Die Transfektionsrate wurde bei 250.000 PC12 MR-A Zellen in einer 24-Loch-Platte unter Einsatz verschiedener Mengen LF 2000 (0,2 - 1,4 µl), sowie unterschiedlicher Cy3-siRNA-Konzentrationen (10 - 200 pmol) 24 Stunden nach der Transfektion mittels Fluoreszenzmikroskopie ermittelt.

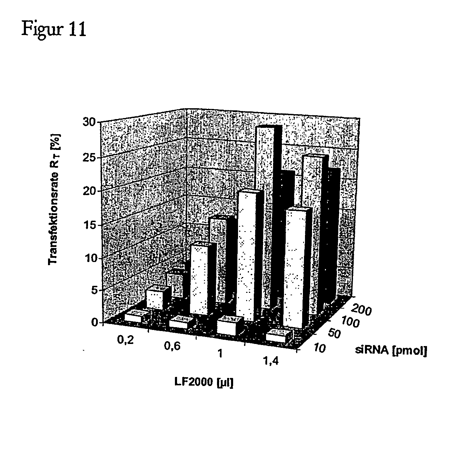

Figur 11 zeigt die Transfektionsrate bei wt-PC12-Zellen. Hierzu wurde die Transfektionsrate bei 250.000 wt-PC12-Zellen in einer 24-Loch-Platte unter Einsatz verschiedener Mengen LF2000 (0,2 - 1,4 µl), sowie unterschiedlicher Cy3-siRNA-Konzentrationen (10 - 200 pmol) 24 Stunden nach der Transfektion mittels Fluoreszenzmikroskopie ermittelt.

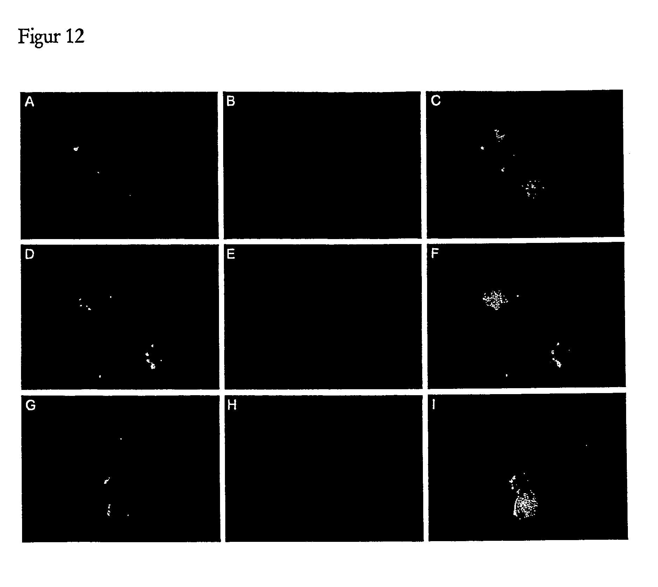

Figur 12 zeigt die Ergebnisse der Behandlung einer DRG-Primärkultur mit Cy3-markierter siRNA gegen VGLUT2. Mit Hilfe der Immunfluoreszenz wurde die Suppression der VGLUT2-Proteinexpression mittels Cy3-markierter siRNA in Neuronen einer DRG-Primärkultur (P0, 2 T.i.v.) 48 Stunden nach Transfektion mit LF2000 (1µl/Loch) in einer 24-Loch-Platte ermittelt. VGLUT2 (Meerschweinchen, 1:800, grün), Cy3-si-rVGLUT2 166 - 186 AMB (100 pmol, rot) und überlagerte Darstellung der VGLUT2 und Cy3-siRNA Signale mit DAPI-gefärbten Zellkernen (C, F, I).

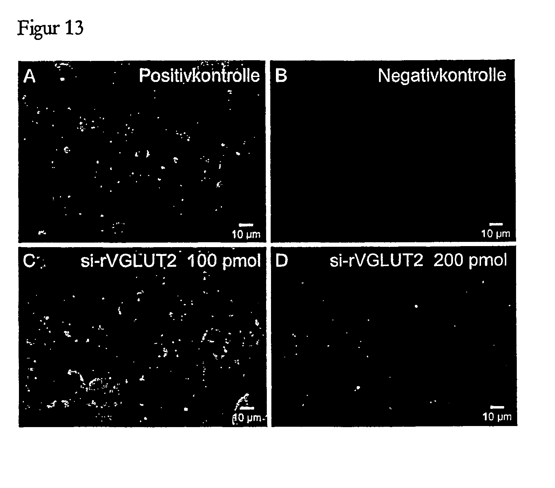

Figur 13 zeigt VGLUT2-Proteinexpression in PC12 MR-A Zellen und Proteinsuppression mittels siRNA. Der immuncytochemische Nachweis der VGLUT2-Proteinexpression wurde (A) in Zellen der Zelllinie PC12 MR-A und Reduktion der VGLUT2-Proteinspiegel mittels siRNA gegen VGLUT2 (si-rVGLUT2 100-120 EGT), 100 pmol siRNA (C), 200 pmol siRNA (D) und Negativkontrolle (B) ohne Primärantikörper gegen VGLUT2 (Kaninchen, 1:800) geführt.

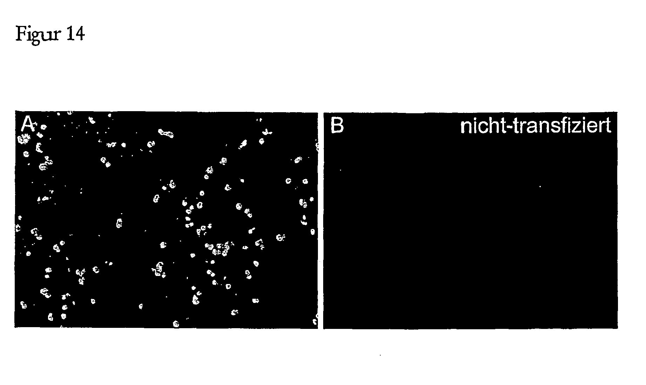

Figur 14 zeigt den Nachweis von VGLUT2 in transfizierten wt-PC12 Zellen. Immunzytochemisch wurde VGLUT2-Protein mit Primärantikörpern gegen VGLUT2 (Kaninchen, 1:800) 24 h nach Transfektion von rVGLUT2-Plasmiden mittels LF2000™ in wt-PC12 Zellen nachgewiesen: (A) VGLUT2-positive Zellen (A488-markiert, grün) in rVGLUT2-transfizierten Zellen; (B) keine VGLUT2-Immunreaktivität in nicht-transfizierten Zellen (Negativkontrolle).

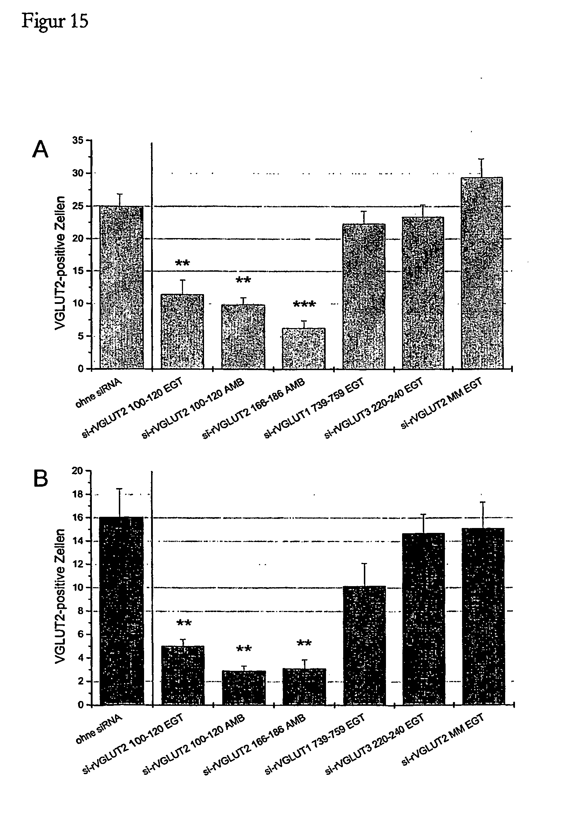

Figur 15 zeigt die siRNA-Behandlung von VGLUT2-kotransfizierten wt-PC12-Zellen. Die Auszählung der immunzytochemisch ma rkierten, VGLUT2-positiven wt-PC12-Zellen erfolgte 24 h nach Kotransfektionen von rVGLUT2-Plasmid mit siRNAs gegen VGLUT2 (si-rVGLUT2 100-120 EGT, si-rVGLUT2 100-120 AMB, si-rVGLUT2 166-186), gegen VGLUT1 (si-rVGLUT1 739-759 EGT) und gegen VGLUT3 (si-rVGLUT3 220-240 EGT) sowie mit einer mismatch siRNA (si-rVGLUT2 MM EGT). Darstellung der Mittelwerte ± S.E.M. für n = 6 pro Gruppe. * p<0,05; ** p<0,01 und *** p<0,001 im Vergleich zur Kontrollgruppe ohne siRNA-Behandlung (ANOVA, Bonferroni-Test). Gegenüberstellung zweier Auswertungsmethoden: (A) Handauszählung (B) Digitalauszählung.

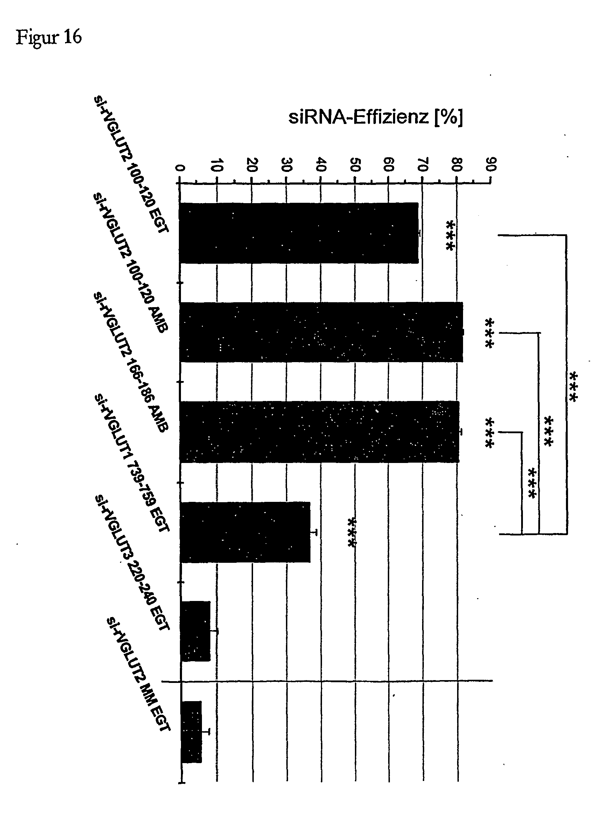

Figur 16 zeigt die Effizienz der siRNAs in wt-PC12 Zellen. Die siRNA-Effizienzen (Prozentsatz der Reduktion VGLUT2-positiver Zellen bezogen auf die VGLUT2-Expression ohne siRNA) wurden 24 h nach Kotransfektion von rVGLUT2-Plasmid und siRNA in wt-PC12-Zellen verglichen. Darstellung der Mittelwerte ± S.E.M. für n = 6 pro Gruppe. *** p<0,001 im Vergleich zur Behandlung mit mismatch-siRNA (ANOVA, Bonferroni-Test).

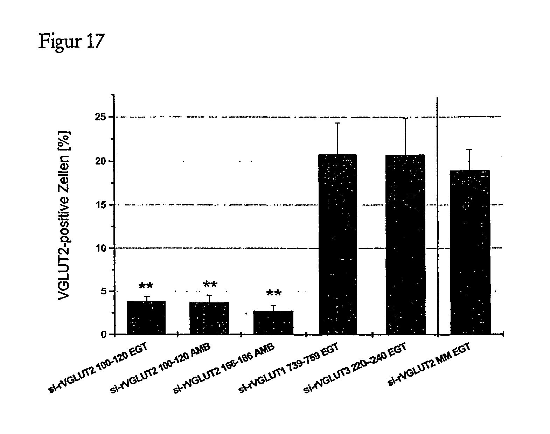

Figur 17 zeigt die siRNA-Behandlung von wt-PC12-Zellen 6 h vor Transfektion mit rVGLUT2. Hierbei wurden immunzytochemisch markierte VGLUT2-positive wt-PC12-Zellen 24 h nach Behandlung mit siRNAs gegen VGLUT2 (si-rVGLUT2 100-120 EGT, si-rVGLUT2 100-120 AMB, si-rVGLUT2 166-186), gegen VGLUT1 (si-rVGLUT1 739-759 EGT) und gegen VGLUT3 (si-rVGLUT3 220-240 EGT), sowie mit einer mismatch siRNA (si-rVGLUT2 MM EGT) ausgezählt. Die Zellen wurden 6 h nach siRNA Behandlung mit rVGLUT2-Plasmid transfiziert. Darstellung der Mittelwerte ± S.E.M. für n = 6 pro Gruppe. ** p<0,01 im Vergleich zur Behandlung mit mismatch-siRNA (ANOVA, Bonferroni-Test).

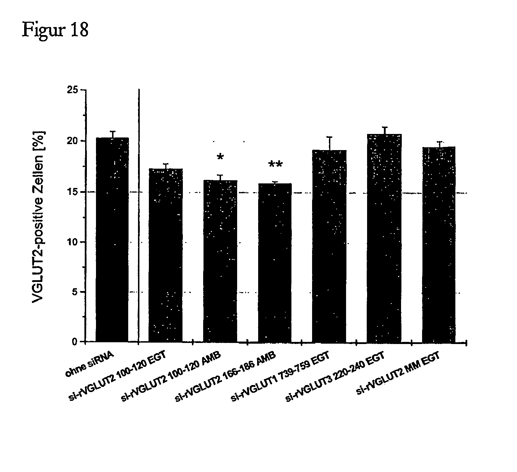

Figur 18 zeigt den Einfluss von siRNA-Behandlung 24h nach rVGLUT2-Transfektion. Immunzytochemisch markierte VGLUT2-positive wt-PC12-Zellen wurden 24 h nach Behandlung mit siRNAs gegen VGLUT2 (si-rVGLUT2 100-120 EGT, si-rVGLUT2 100-120 AMB, si-rVGLUT2 166-186), gegen VGLUT1 (si-rVGLUT1 739-759 EGT) und gegen VGLUT3 (si-rVGLUT3 220-240 EGT), sowie mit einer mismatch siRNA (si-rVGLUT2 MM EGT) ausgezählt. Die Zellen wurden 24 h vor der siRNA Behandlung mit rVGLUT2-Plasmid transfiziert. Darstellung der Mittelwerte ± S.E.M. für n = 6 pro Gruppe. * p<0,05 und ** p<0,01 im Vergleich zu unbehandelten Zellen (ANOVA, Bonferroni-Test).

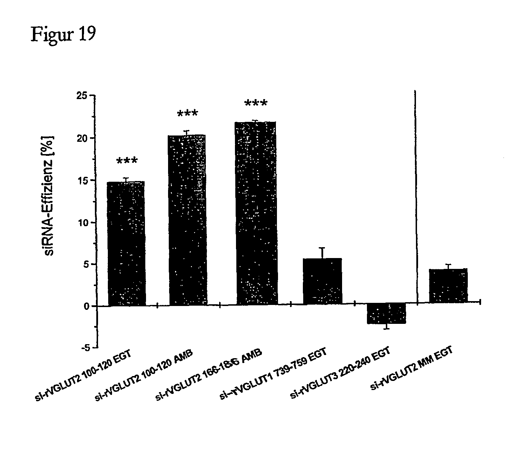

Figur 19 zeigt die Effizienz der siRNAs 24h nach rVGLUT2-Transfektion. Verglichen wurden die siRNA-Effizienzen (Anteil der VGLUT2-Suppression bezogen auf VGLUT2-Expression ohne siRNA) 24 h nach siRNA-Behandlung in wt-PC12-Zellen, die 24 h vor der siRNA Behandlung mit rVGLUT2-Plasmid transfiziert wurden. Darstellung der Mittelwerte ± S.E.M. für n = 6 pro Gruppe. *** p<0,001 im Vergleich zur Behandlung mit mismatch-siRNA (ANOVA, Bonferroni-Test).

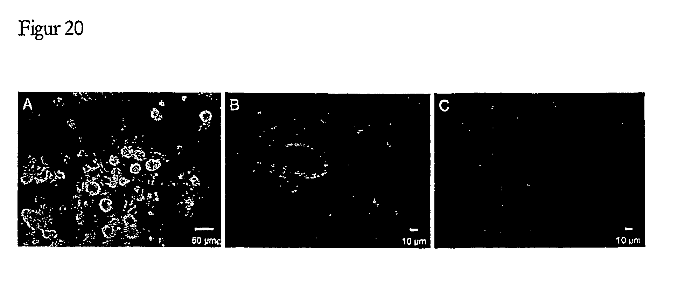

Figur 20 zeigt die Charakterisierung der Zellen in einer Primärkultur des Spinalganglions. Licht- und fluoreszenzmikroskopische Dokumentation unterschiedlicher Zelltypen in einer Primärkultur des Spinalganglion (postnatal Tag 2, 1-5 Tage *in vitro*) werden dargestellt: (A) Lichtmikroskopische Dokumentation (1 T.i.v.); (B, C) Immunzytochemischer Nachweis von (B) Neuronen (5 T.i.v.) mittels Primärantikörper gegen PGP9.5U (1:1500) und von (C) Schwannzellen (5 T.i.v.) mittels Primärantikörper gegen GFAP (1:5000). Die Zellkerne sind in (B) und (C) mit DAPI blau gegengefärbt.

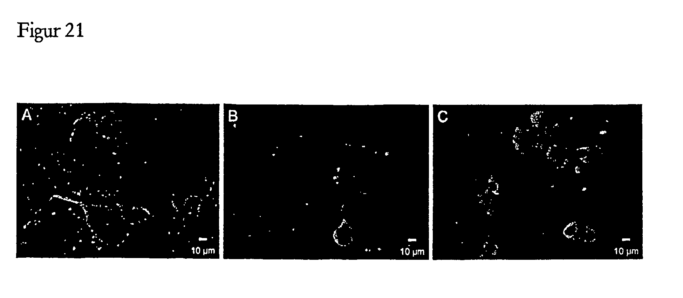

Figur 21 zeigt nozizeptive Neurone in DRG-Primärkulturen. Immunzytochemisch wurden nozizeptive Markerproteine in Zellen einer DRG-Primärkultur (P2, 8 T.i.v.) mittels Primärantikörpern gegen (A) CGRP (Kaninchen, 1:8000); (B) TRPV1 (Kaninchen, 1:250); (C) TRPV2 (Kaninchen, 1:400) nachgewiesen.

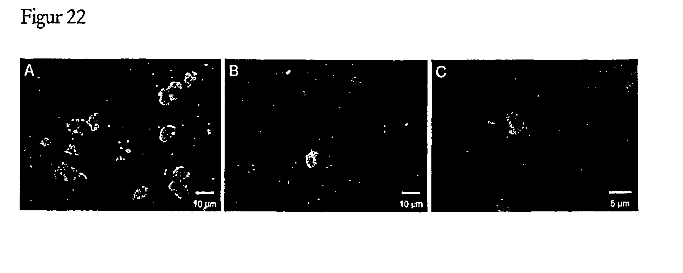

Figur 22 zeigt die VGLUT2-Proteinexpression in DRG-Primärkulturen. Hierzu wurde immunzytochemisch die VGLUT2-Proteinexpression in Primärkulturen von Spinalganglien charakterisiert: (A) VGLUT2 (Meerschweinchen, 1:800); (B) VGLUT2 (1:800, grün), PGP9.5 (Kaninchen, 1:1500, rot), Koexpression von VGLUT2 und PGP9.5 (gelb); (C) VGLUT2 (1:800, grün), GFAP (Kaninchen, 1:5000, rot). Alle Zellkerne wurden mit DAPI gegengefärbt.

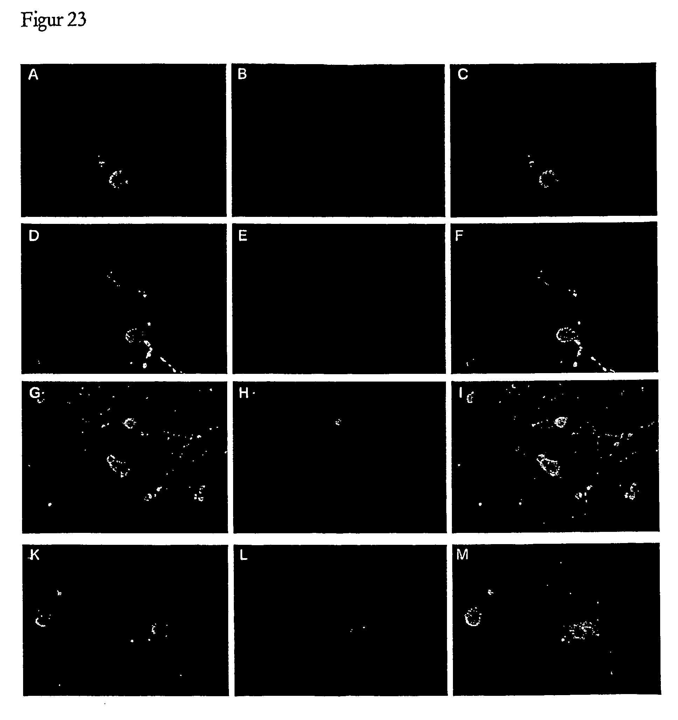

Figur 23 zeigt die VGLUT1 bzw. VGLUT2 Expression in peptidergen DRG-Neuronen. Doppelimmunfluoreszenz von VGLUT1 bzw. VGLUT2 mit CG RP in DRG-Neuronen (A-M) ist dargestellt: (A, C, D, F) VGLUT1 (Meerschweinchen, 1:800, grün); (B, C, E, F, H, I, L, M) CGRP (Kaninchen, 1:5000, rot); (G, I, K, M) VGLUT2 (Meerschweinchen, 1:800, grün). VGLUT1 und CGRP werden in verschiedenen Subpopulationen (C, F) exprimiert, da CGRP mit VGLUT2 koexistiert (I, M, gelbes Signal).

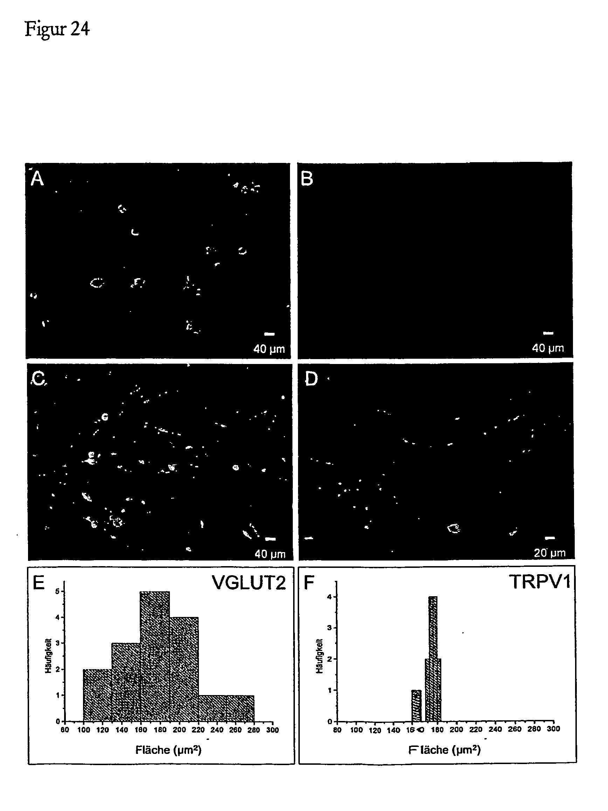

Figur 24 zeigt die Expression von VGLUT2 in TRPV1-positiven Neuronen. Immunzytochemisch wurde die VGLUT2-Expression in TRPV1-positiven Neuronen einer DRG-Primärkultur nachgewiesen: (A) VGLUT2 (Meerschweinchen, 1:800, grün); (B) TRPV1 (Kaninchen, 1:250, rot); (C) VGLUT2-Kolokalisation in TRPV1 - positiven Neuronen (gelbes Signal); (D) Ausschnittsvergrößerung von Bild C: Signale von VGLUT2 im Zellsoma (gelb) und im Axon (gelb, grün) des TRPV1 - positiven Neurons; (E, F) Häufigkeitsverteilung für die Zellflächen von VGLUT2- bzw. TRPV1-positiven Neuronen.

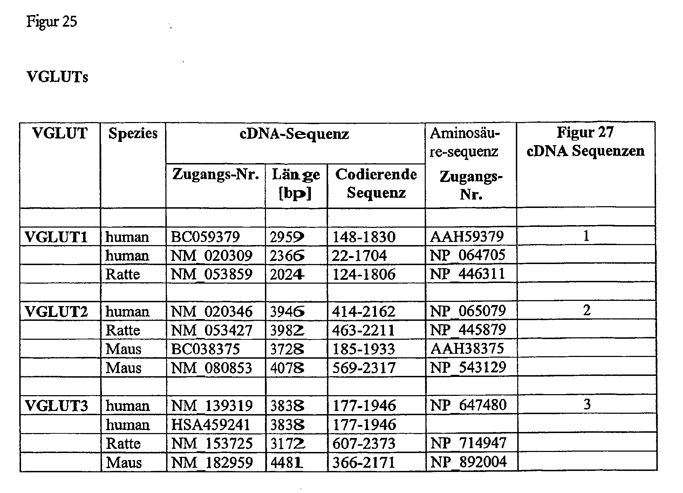

Figur 25 gibt einen Überblick über die verschiedenen VGLUT-Sequenzen (human, Ratte) (VGLUT1, VGLUT2, VGLUT3), einschließlich des jeweiligen Datenbank-Zugangscodes.

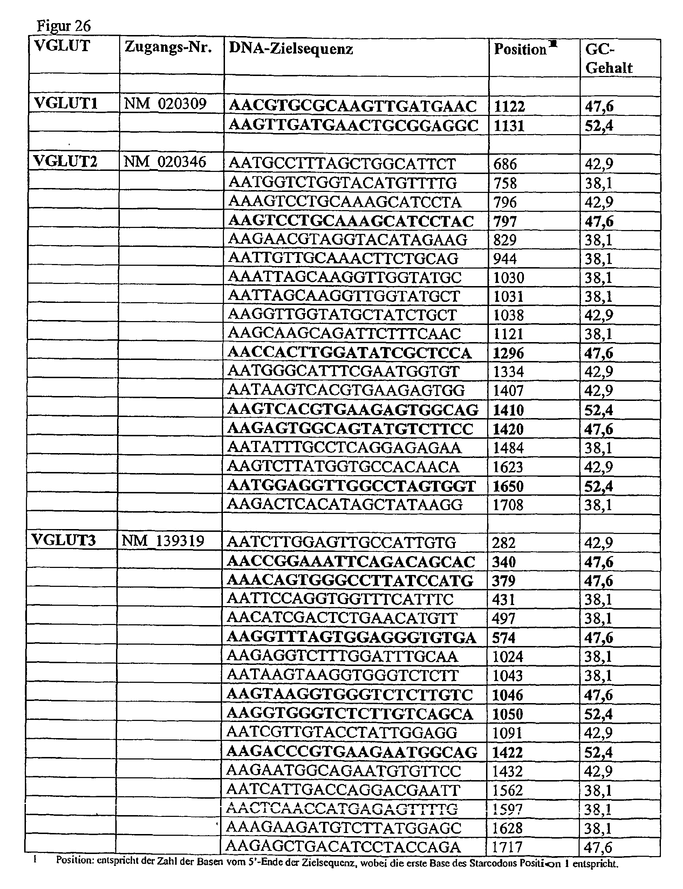

Figur 26 stellt DNA-Zielsequenzen von VGLUT-Isoform-spezifischen siRNAs dar. Im Fettdruck sind bevorzugte Sequenzen dargestellt. Homologien wurden mit dem Smith-Waterman-Algorithmus getestet.

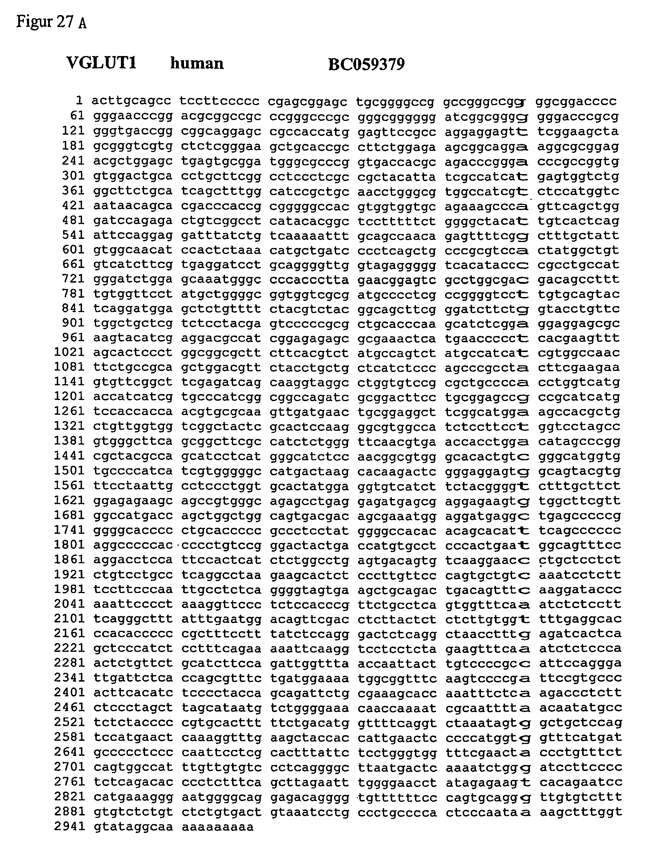

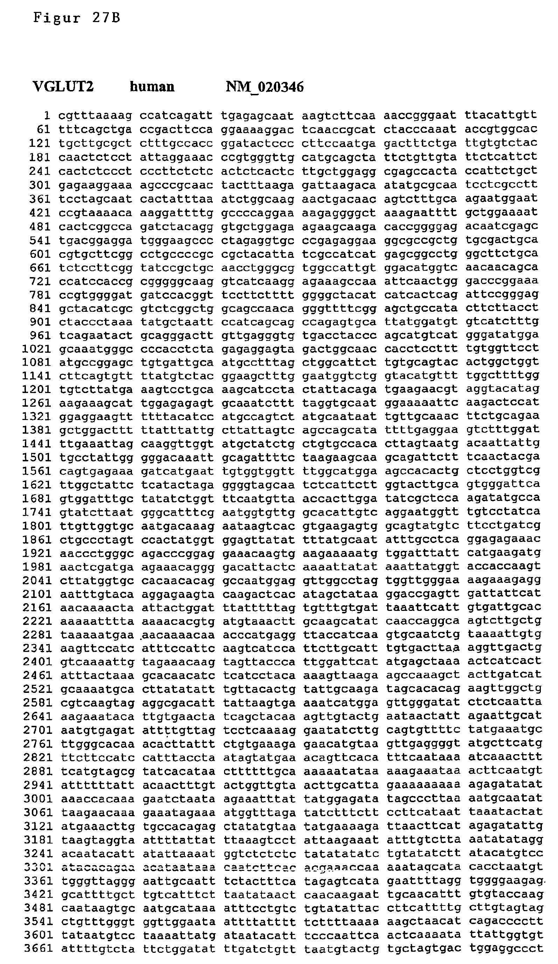

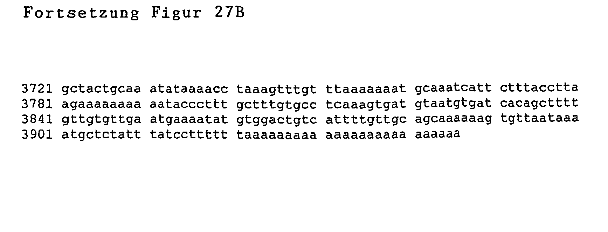

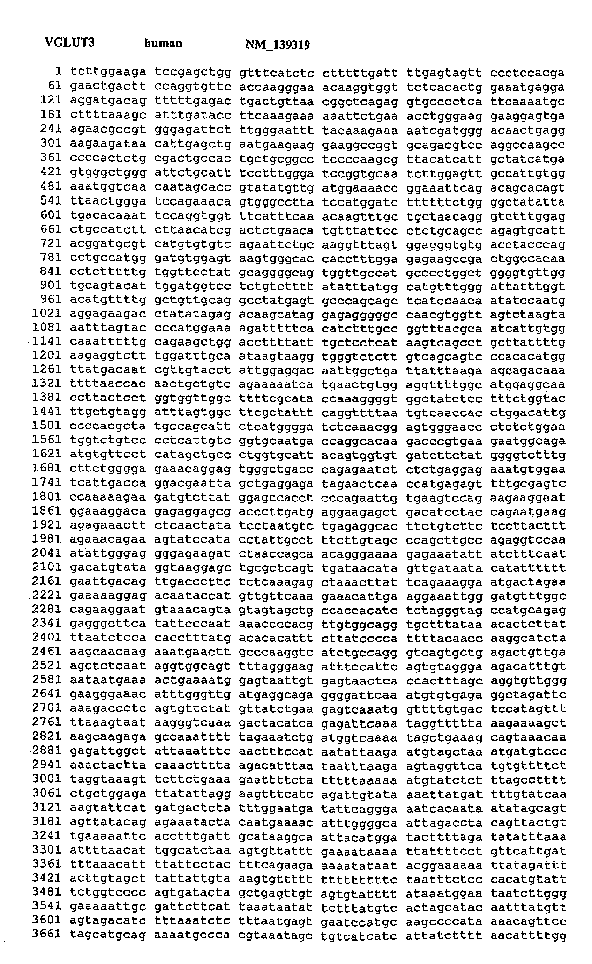

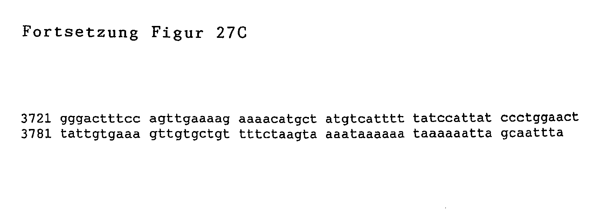

Figur 27 stellt die Nukleotidsequenzen von VGLUT1, VGLUT2 bzw. VGLUT3 dar (jeweils human).

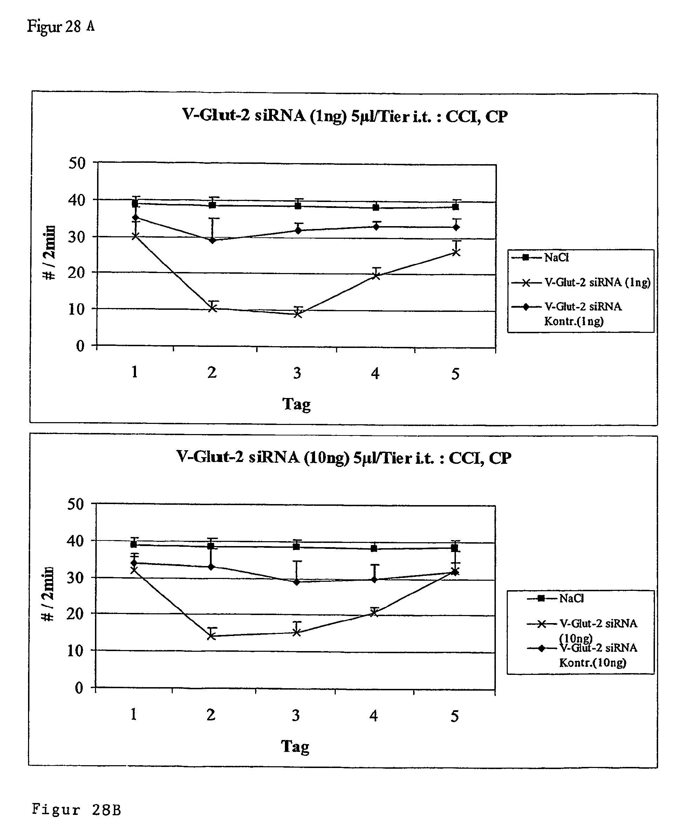

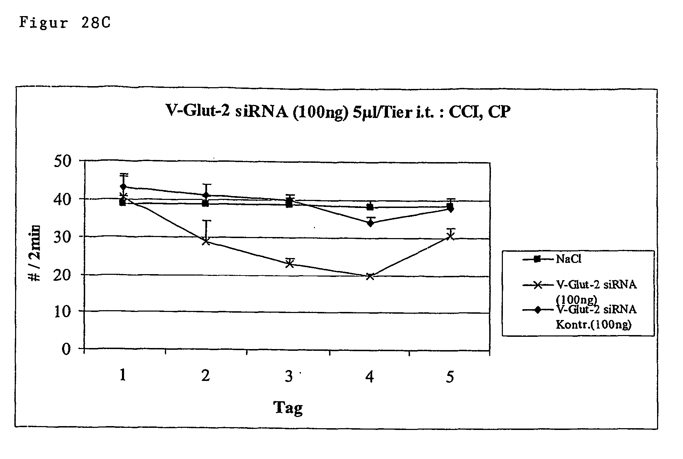

Figur 28 zeigt die Ergebnisse der *in-vivo*-Versuche mit Ratten nach dem Schmerzmodell von Bennett (s. Ausführungsbeispiel 4). Es wurde bei drei verschienen Dosierungen getestet. Die Figuren 28 A, 28 B bzw. 28C zeigen die Ergebnisse der Versuche bei Verwendung von 1ng, 10 ng bzw. 100 ng Testsubstanz (VGLUT2-siRNA) und entsprechenden Mengen an Kontroll-siRNA. Darüber hinaus wurde jedem Tier NaCl als weitere Kontrolle verabreicht. Die antiallodynische Wirkung von VGLUT2-siRNA (gekreuztes Symbol) ist insbesondere in Figur 28A deutlich erkennbar.

Die vorliegende Erfindung wird durch die nachfolgen den Ausführungsbeispiele näher charakterisiert.

### Ausführungsbeispiele

### Ausführungsbeispiel 1

### A) Design von siRNA-Molekülen

Das Design der verwendeten erfindungsgemäßen siRNA-Moleküle entspricht den erfindungsgemäßen Ausführungsformen. In Figur 5, Figur 6 und Figur 7 sind die kodierenden Sequenzen der drei verschiedenen vesikulären Glutamat-Transporter dargestellt. Farbig unterlegt wurde jeweils das Start-Kodon (gelb), sowie die für die (quantitative) Bestimmung verwendeten Primerpaare (hell- und dunkelgrau). Die Zielsequenzen der verschiedenen siRNAs sind ebenfalls farbig unterlegt (rot, grün, blau).

### B) Herstellung der siRNA

Die verwendeten siRNAs wurden zum einen bei der Firma Eurogentec (EGT) zur Synthese in Auftrag gegeben, andererseits mit dem siRNA-Construction-Kit der Firma Ambion (AMB) selbst hergestellt. Folgende siRNA-Moleküle wurden für die Synthese bei Eurogentec in Auftrag gegeben:

Mit dem Silencer^{™} siRNA-Construction Kit der Firma Ambion wurden folgende siRNA-Moleküle hergestellt:

Mit dem Silencer siRNA Labeling Kit der Firma Ambion wurde ein Teil der selbst hergestellten siRNAs mit dem Farbstoff Cy3 markiert. Die vorgenannten Sequenzen wurden für die nachfolgend beschriebenen *in-vitro*-Experimente eingesetzt.

### Herstellung von siRNA mittels Silencer^{™} siRNA-Construction Kit (Ambion)

Um effiziente Transkriptions-Vorlagen für die siRNA-Synthese herzustellen, müssen die Sense- und Antisense-Oligonukleotide in dsRNA mittels T7 Promotor am 5' Ende umgewandelt werden. Dies wird dadurch erreicht, dass die zwei Oligonukleotide mit dem T7 Promotor Primer hybridisiert und durch anschließende DNA-Polymerisations-Reaktion verlängert werden (s. auch Figur 3).

Die Sense- und Antisense-siRNA-Templates werden in getrennten Reaktionsansätzen für zwei Stunden transkribiert. Die Ansätze werden anschließend gemischt und der gemeinsame Reaktionsansatz über Nacht inkubiert. Die getrennten Transkriptionsansätze verhindern zwischen den Templates einen potentiellen Wettbewerb um die Transkriptionsreagenzien, was die Synthese von ei nem der beiden Stränge der siRNA limitieren könnte. Durch das Mischen der Transkriptionsansätze wird die Hybridisierung der beiden siRNA-Stränge erleichtert und so eine kontinuierliche RNA Synthese ermöglicht, was die Ausbeute der dsRNA erhöht. Die durch *in-vitro*-Transkription gewonnene siRNA besitzt am 5' En de überhängende "Leader-Sequenzen", die vor einer Transfektion entfernt werd en müssen. Diese "Leader-Sequenz" wird durch eine Einzelstrang-spezifische Ribonuklease verdaut. Im selben Reaktionsansatz wird das DNA-Template durch einen DNase-Verdau entfernt (s. auch Figur 4).

Die resultierende siRNA muss von der Mischung aus Nukleotiden, Enzymen, kurzen Oligomeren und Salzen mittels RNA-Säulen aufgereinigt werden.

Die so gereinigte siRNA wird in nukleasefreiem Wasser eluiert und steht anschließend für die Transfektion zur Verfügung.

### Durchführung

Aus der 200 µM Stocklösung wurde eine 100 µM Lösung von jedem siRNA ONV hergestellt. Für die Hybridisierung der siRNA ONV mit dem T7 Promotor Primer wurden für Sense und Antisense jeweils folgende Reaktionsansätze hergestellt:

| | |
|---|---|
| T7 Promotor Primer | 2 µl |
| DNA Hyb Puffer | 6 µl |
| Sense- / Antisense-siRNA ONV | 2 µl |

Die Ansätze wurden zuerst für 5 min auf 70°C erhitzt, dann für 5 min bei RT gehalten. Anschließend wurde den Reaktionsansätzen folgender Reaktionsmix zugeführt, vorsichtig vermischt und für 30 min bei 37°C inkubiert:

| | |
|---|---|
| 10X Klenow Reaktionspuffer | 2 µl |
| 10X dNTP Mix | 2 µl |
| Nukleasefreies Wasser | 4 µl |
| Exo-Klenow DNA Polymerase | 2 µl |

Für beide DNA-Ansätze wurde jeweils ein Transkriptions-Reaktionsansatz bei RT hergestellt, um die Sense- und Antisense-ssRNA-Stränge zu synthetisieren. Dazu wurden folgende Komponenten in der angegebenen Reihenfolge zusammengestellt, vorsichtig, ohne zu pipettieren, gemischt und für 2 h bei 37°C inkubiert:

| | |
|---|---|
| Sense- oder Antisense-DNA-Template | 2 µl |
| Nukleasefreies Wasser | 4 µl |
| 2X NTP Mix | 10 µl |
| 10X T7 Reaktionspuffer | 2 µl |
| T7 Enzym Mix | 2 µl |

Nach den 2 h wurden die beiden Transkriptionsansätze zusammenpipettiert und über Nacht bei 37°C inkubiert.

Für den Verdau der hybridisierten dsRNA mit RNase und DNase wurde folgender Reaktionsansatz zusammengestellt und zur dsRNA hinzupipettiert, vorsichtig gemischt und für 2 h bei 37°C inkubiert::

| | |
|---|---|
| Verdau Puffer | 6 µl |
| Nukleasefreies Wasser | 48,5 µl |
| RNase | 3 µl |

Zum Nukleaseverdau wurden anschließend 400 µl siRNA Bindepuffer zugeführt und 2 - 5 min bei RT inkubiert. In der Zwischenzeit wurde die mitgelieferte Filtermembran mit 100 µl siRNA Waschpuffer befeuchtet. Auf den befeuchteten Filter wurde die siRNA im siRNA Bindepuffer aufgetragen und für 1 min bei 10.000 U/min zentrifugiert. Der Durchfluss wurde verworfen und die Filtermembran 2 x mit jeweils 500 µl des siRNA Waschpuffers gewaschen und zentrifugiert (2 min bei 10.000 U/min). Die gereinigte siRNA wurde anschließend mit in 100 µl 75°C heißem nukleasefreiem Wasser eluiert und in ein sauberes Auffanggefäß abzentrifugiert (2 min bei 12.000 U/min). Die siRNA wurde bis zur Verwendung bei - 20°C oder -80°C gelagert.

### Synthese bei Eurogentec und siRNA-Duplex Bildung

Die von Eurogentec synthetisierten RNA-Oligonukleotide wurden mittels DEPCbehandeltem H₂O in eine 50 µM Lösung gebracht und aliquotiert. Jeweils 30 µl der zusammengehörenden RNA-Oligonukleotidlösungen wurden mit 15 µl 5X Annealing-Puffer gemischt Endkonzentrationen: 20 µM siRNA Duplex; 50 mM Tris pH 7,5 - 8,0; 100 mM NaCl in DEPC-H₂O). Die Lösung wurde 1 - 2 min im Wasserbad bei 90 - 95°C erhitzt und bei RT für 45 - 60 min abkühlen lassen. Die siRNA wurde bei -20°C bis zur Verwendung gelagert.

### Markierung der siRNA mit Cy3

Zur siRNA-Markierung mit dem Fluoreszenzfarbstoff Cy3 wurden sowohl die von Eurogentec synthetisierten, als auch die selbst hergestellten Duplex siRNAs verwendet. Um 5 µg siRNA zu markieren, wurde folgender Reaktionsansatz hergestellt und 1 h bei 37°C inkubiert:

| | |
|---|---|
| Nukleasefreies Wasser | 18,3 µl |
| 10x Labeling Mix | 5,0 µl |
| 21-mer Duplex siRNA (20 µM) | 19,2 µl |
| Cy3 Labeling Reagenz | 7,5 µl |

Die Cy3-markierte siRNA wurde mittels Ethanol Präzipitation aufgereinigt. Dazu wurde 0,1 Volumen 5 M NaCl und 2,5 Volumen 100% Ethanol zum Reaktionsansatz hinzugeführt, gründlich gemischt und für 60 min bei -80°C aufbewahrt. Das Präzipitat wurde durch 20 min Zentrifugation (> 8.000 x g) pelletiert, der Überstand vorsichtig, ohne das Pellet zu zerstören, abgenommen und schließlich mit 175 µl 70% Ethanol gewaschen. Nach Abzentrifugation (5 min bei > 8.000 x g) wurde der gesamte Überstand entfernt, das Pellet bei RT für 5 - 10 min getrocknet und abschließend in einer entsprechenden Menge nukleasefreiem Wasser (hier 19,2 µl) gelöst.

### Ausführungsbeispiel 2

### Einsatz der VGLUT-siRNAs in verschiedenen in-vitro-Modellen

Um die Effektivität der hergestellten siRNAs zu testen, wurden sie in verschiedenen *in-vitro*-Modellen eingesetzt und anschließend die Proteinexpression mittels Immunzytochemie ermittelt.

### A) Optimierung der Transfektionsbedingungen

Für eine hohe Effizienz der siRNA-Gensuppression ist neben der eigentlichen Wirksamkeit der siRNA eine hohe Transfektionsrate der jeweiligen Zellen notwendig. Dabei spielen die Zelldichte, die Menge der Transfektionsreagenz, sowie die Konzentration der siRNA eine wichtige Rolle. Unter Variation dieser verschiedenen Parameter wurde die Transfektionsrate (R_{T} = Anzahl transfizierter Zellen / Gesamtzellzahl) mittels Cy3-markierter siRNA ermittelt. Als Transfektionsreagenz diente Lipofectamine^{™} 2000.

Figur 8 zeigt beispielhaft die Ergebnisse der Lokalisation von Cy3-markierter siRNA 24 Stunden nach Transfektion mit LF2000^{™}. In den hier gezeigten PC12 MR-A Zellen akkumuliert die markierte siRNA im Zytoplasma vor allem in der Nähe des Zellkerns.

Die Transfektion von Spinalganglienzellen in einer Primärkultur erwies sich als extrem schwierig. Wie in Figur 9 gezeigt, erreicht man beim Transfizieren der Zellen mit Cy3-markierter siRNA mittels LF2000 lediglich eine Transfektionsrate von maximal ~2%. Dabei ließ sich keine Präferenz der Transfektion für einen bestimmten Zelltyp ausmachen, sondern neuronale, wie nicht-neuronale Zellen zeigten gleichermaßen ein Cy3-Fluoreszenzsignal. Da jedoch der Neuronenanteil maximal 60% betrug, war die Ausbeute siRNA-transfizierter Neurone sehr gering.

Die höchsten Transfektionsraten (R_{Tmax} ≈ 80%) wurden bei Zellen der Zelllinien PC12 MR-A und PC12 MR-B erzielt, wie in Figur 10 beispielhaft für die Zelllinie PC12 MR-A gezeigt.

Normale wt-PC12-Zellen lassen sich ebenfalls gut mit siRNA transfizieren. In der Figur 11 sind die unterschiedlichen Transfektionsraten für die verschiedenen Transfektionsbedingungen dargestellt. Die maximale Transfektionsrate betrug ~ 28%.

### B) Suppression der VGLUT-Expression mittels siRNA in verschiedenen in-vitro-Modellen

### 1. Suppression der endogenen VGLUT-Expression in DRG-Primärkulturen

Die Zellen der DRG-Primärkulturen wurden gleich nach der Dissoziation der Ganglien und Aufreinigung der Zellsuspension mit dem Transfektionsreagenz LF2000™ mit verschiedenen siRNAs transfiziert. Die transfizierten Zellen wurden entweder in normale Kulturschalen ausgesät, oder in einer 24-Loch-Platte auf Poly-L-Lysin beschichteten Deckgläschen kultiviert.

DRG-Primärkulturen, die mit Cy3-markierter siRNA (Cy3-si-rVGLUT2 166 - 186 AMB) transfiziert wurden, wurden 48 Stunden nach der Transfektion fixiert und immunzytochemisch bzgl. ihrer VGLUT2-Proteinexpression charakterisiert. Figur 12 zeigt das Ergebnis einer solchen siRNA-Behandlung.

Auf den mit siRNA-behandelten Deckgläschen waren ca. 2% transfizierte Zellen, die an ihrer Cy3-Färbung zu erkennen waren. Keine dieser siRNA-transfizierten Zellen zeigte nach immunzytochemischer Markierung des vesikulären Glutamat-Transporters VGLUT2 ein deutliches Proteinsignal für VGLUT2. Dagegen waren alle VGLUT2-positiven Zellen ohne Signale der Cy3-markierten siRNA.

### 2. Suppression der endogenen VGLUT-Expression in etablierten Zelllinien

Die ausdifferenzierten Zelllinien PC12 MR-A und PC12 MR-B exprimieren die vesikulären Glutamat-Transporter VGLUT1 und VGLUT2. Die Figur 13 zeigt die Expression des VGLUT2-Proteins in den PC12 MR-A Zellen.

Unter optimierten Bedingungen wurden diese Zellen mit siRNA gegen VGLUT2 (si-rVGLUT2 100-120 EGT) transfiziert. Wie in Figur 13 dargestellt, wird mit 100 pmol siRNA (C) bereits eine Reduktion der VGLUT2-Immunreaktivität erreicht, bei 200 pmol siRNA (D) sind die meisten Zellen ohne VGLUT-2 Immunreaktivität bzw. weisen nur noch geringe VGLUT2-Immunreaktivität auf.

### 3. Suppression der VGLUT2-Expression in transient transfizierten wt-PC12 Zellen

Zellen der normalen wt-PC12 Zelllinie exprimieren nur VGLUT1, jedoch kein VGLUT2. Aus diesem Grund eignen sich diese endokrinen, vesikelproduzierenden Zellen als Modellsystem für Suppressionsexperimente nach transienter VGLUT2-Transfektion. Zur Optimierung der DNA-Transfektionen wurde ein EGFP-Vektor eingesetzt, dessen Genprodukt, das grünfluoreszierende Protein (GFP) direkt durch Fluoreszenzmikroskopie nachgewiesen werden kann. Durch Optimierung der DNA-Transfektionsbedingungen wurde eine EGFP-Transfektionsrate von ~40% erreicht. Eine ähnliche hohe Transfektionsrate konnte auch bei Transfektionen mit rVGLUT2-Plasmiden erreicht werden (Figur 14).

Die siRNA-Suppressionsexperimente wurden unterschiedlich gestaltet:
- Kotransfektionen von siRNA und cDNA-Vektor
- siRNA-Transfektion 6 h vor cDNA-Transfektion
- cDNA-Transfektion 24 h vor siRNA-Transfektion

Zunächst wurden Kotransfektionen mit rVGLUT2-Plasmiden und verschiedenen siRNAs durchgeführt. Dabei wurden drei siRNAs gegen VGLUT2 verwendet, sowie siRNAs gegen VGLUT1, VGLUT3 und eine mismatch-siRNA zur Spezifitätskontrolle. Die Zellen wurden 24 h nach der Transfektion fixiert und die VGLUT2-Proteinexpression immunzytochemisch nachgewiesen. Die Auswertung erfolgte am Fluoreszenzmikroskop, wobei die VGLUT2-positiven Zellen sowohl per Hand, als auch mittels digitaler Bildanalyse (MCID) ausgezählt wurden.

Figur 15 zeigt einen Vergleich des Anteils VGLUT2-positiver Zellen nach rVGLUT2-Plasmid-Transfektionen ohne und mit siRNA-Kotransfektion, sowie eine Gegenüberstellung der beiden Auswertungsmethoden. Die Resultate der zeitsparenden Digitalauszählung (B) mit der Handauszählung (A) stimmen von ihren relativen Verhältnissen überein. Alle späteren Experimente wurden daher digital ausgewertet. Nach Behandlung mit spezifisch gegen VGLUT2-gerichteten siRNAs war der Anteil VGLUT2-positiver Zellen im Vergleich zu nicht-siRNA-behandelten Zellen (24,92 ± 1,9) signifikant reduziert (si-rVGLUT2 100-200 EGT 11,4 ± 2,2 p<0,01; si-rVGLUT2 100-200 AMB 9,84 ± 1,1 p<0,01; si-rVGLUT2 166-186 AMB 6,29 ± 1,1 p<0,001).

Dagegen beeinflussen die siRNAs gegen VGLUT1 bzw. gegen VGLUT3 und die mismatch siRNA nicht signifikant (p>0,05) den Anteil der VGLUT2-positiven Zellen (A).

Berechnet man aus diesen Ergebnissen die Effizienz der verwendeten siRNAs als Prozentsatz der Reduktion VGLUT2-positiver Zellen bezogen auf die VGLUT2-Expression ohne Beeinflussung von siRNA-Behandlung, erhält man das in Figur 16 dargestellte Resultat.

Es zeigte sich, dass die gegen VGLUT2 gerichteten siRNAs eine hohe Effizienz im Vergleich zur unwirksamen mismatch siRNA aufweisen. Sie reduzieren den Anteil VGLUT2-exprimierender Zellen um 79 - 82%, wobei die selbst hergestellten siRNAs in den verwendeten Konzentrationen effektiver wirken, als die bei Eurogentec synthetisierte siRNA. Während die siRNA gegen VGLUT3 und die mismatch siRNA keinen signifikanten Effekt auf die VGLUT2-Proteinexpression ausüben, scheint die gegen VGLUT1 gerichtete siRNA mit einer Effizienz von ~47% unspezifisch auf die VGLUT2-Expression zu wirken. Allerdings sind die siRNAs gegen VGLUT2 signifikant effizienter (p<0,001) als die siRNA gegen VGLUT1.

Im Folgenden wurde das Transfektionsexperiment modifiziert: Die wt-PC12 Zellen wurden 6 h vor der Transfektion mit rVGLUT2-Plasmid mit den verschiedenen siRNAs behandelt, sodass sich zum Zeitpunkt der DNA-Transfektion bereits die siRNAs in den Zellen befanden.

Figur 17 zeigt den Anteil VGLUT2-positiver Zellen in diesem Versuchsansatz. Wie bei den Kotransfektionen führen die siRNAs gegen VGLUT2 zu einer signifikanten Reduktion (p<0,01) VGLUT2-exprimierender Zellen im Vergleich zu den Kulturen, die mit mismatch siRNA behandelt wurden. Dagegen ist der Anteil VGLUT2-positiver Zellen bei Behandlung mit siRNAs gegen VGLUT1 bzw. VGLUT3 nicht signifikant verändert (p>0,05).

In einem weiteren Experiment wurde der Einfluss bereits vorhandener VGLUT2-Proteinspiegel auf die Suppressionseffizienz der siRNAs getestet. Dieses Modell entspricht em ehesten den endogen VGLUT2-exprimiereden Zellen und der Situation *in vivo.* Dazu wurden die wt-PC12-Zellen 24 h vor der siRNA-Behandlung mit dem rVGLUT2-Plasmid transfiziert. Nach weiteren 24 h wurde der Anteil der VGLUT2-positiven Zellen ermittelt.

In der Figur 18 ist der Anteil der VGLUT2-positiven Zellen nach einer immunzytochemischen Markierung dargestellt. Wie in den Versuchen zuvor führen die Kontroll-siRNAs (gegen VGLUT1 und VGLUT3, mismatch siRNA) zu keiner signifikanten Reduktion (p>0,05) der VGLUT2-Proteinexpression. Dagegen senken die spezifisch gegen VGLUT2 gerichteten siRNAs (von Ambion) signifikant den Anteil der VGLUT2-positiven Zellen.

Die Effizienz der siRNAs in diesem Versuchsansatz ist in der Figur 19 dargestellt. Aus dieser Abbildung wird nochmals deutlich, dass die gegen VGLUT2-gerichteten siRNAs signifikant den Anteil VGLUT2-positiver Zellen senken und dass dieser Effekt im Vergleich zur mismatch siRNA deutlich signifikant ist (p<0,001).

Die Ergebnisse dieser Versuche belegen die spezifische Wirksamkeit der gegen VGLUT2-gerichteten siRNAs (si-rVGLUT2 100-120 EGT; si-rVGLUT2 100-120 AMB; si-rVGLUT2 166-186 AMB). Dabei gibt es keinen Unterschied zwischen der selbst hergestellten (si-rVGLUT2 100-120 AMB) und der von Eurogentec synthetisierten siRNA (si-rVGLUT2 100-120 EGT). Die siRNA-Kontrollen gegen VGLUT1 (si-rVGLUT1 739-759 ENGT) und gegen VGLUT3 (si-rVGLUT3 220-240 EGT) führen nicht zu einer Reduktion der VGLUT2-Proteinspiegel. Auch die sog. mismatch siRNA (si-rVGLUT2 MM EGT), welche die gleichen Nukleotide wie die spezifische siRNA gegen VGLUT2 besitzt, jedoch in randomisierter und damit nicht komplementärer Anordnung, beeinflusst nicht die VGLUT2-Proteinspiegel.

Die Effizienzen der spezifischen siRNAs liegen bei diesen Versuchen zwischen -15 und ~80%. Hohe siRNA-Effizienzen (78-82%) werden bei gleichzeitigen Kotransfektionen von siRNA und DNA erreicht, während bei den Suppressionsversuchen mit bereits vorhanden VGLUT2-Proteinspiegeln zum Zeitpunkt der siRNA Transfektionen geringere Effizienzen (15-23%) erreicht werden. Daraus lässt sich ableiten, dass erstens die spezifischen siRNAs mit hoher Effizienz die VGLUT2-Proteinneubildung reduzieren und zweitens, dass die VGLUT2-Proteine sehr stabil sind, bzw. nur einen geringen Tumover aufzuweisen.

### Ausführungsbeispiel 3

### Charakterisierung der VGLUT-Expression in Primärkulturen des Spinalganglions

Aus neonatalen Ratten wurden die Spinalganglien präpariert und wie unter den nach den Ausführungsbeispielen näher ausgeführten experimentellen Bedingungen beschrieben in Kultur genommen. Für die Kulturen wurden jeweils 20 - 30 Spinalganglien von 8 - 16 neonatalen Ratten präpariert und die Zellen aufgereinigt. Der Anteil der Neurone an der Gesamtzellzahl wurde durch Aufreinigung über eine BSA-Säule, sowie durch Ausplattieren der Zellsuspension auf Poly-L-Lysin-beschichtete Materialien deutlich gegenüber dem Standardprotokoll erhöht (Grothe and Unsicker, 1987). Die im Vergleich zu den nicht-neuronalen Zellen wesentlich größeren und damit auch schwereren Neurone setzen sich auf dem beschichteten Untergrund schneller ab. Durch Entfernen des Überstandes nach bereits 5 min verringert sich die Anzahl der nicht-neuronalen Zellen, die sich überwiegend als spindelförmige Zellen mit Ausläufern präsentierten. Nach 4 bis 10 Tagen *in vitro* wurden die Zellen fixiert und immunzytochemisch charakterisiert.

Figur 20 zeigt die kultivierten Zellen des Spinalganglions. Bereits lichtmikroskopisch (A) lassen sich verschiedene Zelltypen anhand ihrer Morphologie unterscheiden. Die Neurone sind durch ihre kugelförmige Gestalt und die deutliche Lichtbrechung zu erkennen, während die überwiegende Zahl der nicht-neuronalen Zellen spindelförmige Fibroblasten sind. Die Neurone ließen sich auch immunzytochemisch mit Primärantikörpern gegen den panneuronalen Marker "Protein-Genprodukt 9.5" (PGP9.5) darstellen (B). In den Kulturen befanden sich auch einige wenige Schwannzellen, die mit Antikörpern gegen GFAP identifiziert wurden (C).

Da für die späteren Versuche vor allem die primärsensorischen, nozizeptiven Neurone von Interesse seien würden, wurden mittels immunzytochemischer Marker nachgewiesen, dass diese Neuronenpopulation in der Primärkultur vorhanden ist und sich mindestens 8 Tage *in vitro* kultivieren lässt. Figur 21 zeigt diese Neurone: Peptiderge Spinalganglienzellen (A) mit CGRP-Proteinexpression, sowie hitzesensible Neurone, welche die Ionenkanäle (B) TRPV1 und (C) TRPV2 exprimieren.

Die Figur 22 zeigt die Expression von VGLUT2 (A-C, grünes Signal) in Zellen der DRG-Primärkultur. VGLUT2 wird dort in Neuronen exprimiert (B), wobei alle VGLUT2 positiven Zellen auch positiv für den Neuronenmarker PGP9.5 sind (gelbes Signal), jedoch nicht alle PGP9.5-positiven Zellen VGLUT2-positiv sind (rotes Signal). Eine Koexpression von VGLUT2 in Schwannzellen konnte durch Komarkierungen mit den verwendeten Antikörpern gegen GFAP (C, rotes Signal) ausgeschlossen werden.

Für VGLUT1 verhält sich die Proteinexpression entsprechend (nicht gezeigt): VGLUT1 wird in einer Subpopulation PGP9.5-positiver Neurone exprimiert und kommt nicht in Schwannzellen vor.

Als nächstes wurde die Expression der vesikulären Glutamat-Transporter VGLUT1 und VGLUT2 in peptidergen, CGRP-positiven Neuronen untersucht (Figur 23): VGLUT1 (grün) und CGRP (rot) werden in zwei unterschiedlichen Zellpopulationen exprimiert (C, F), wohingegen VGLUT2 und CGRP koexistieren (I, M). Es zeigt sich, dass alle CGRP immunreaktiven Neurone den vesikulären Glutamat-Transporter VGLUT2 besitzen. Allerdings bilden nicht alle VGLUT2 positiven Neurone das Neuropeptid CGRP.

Um die VGLUT2-Expression in polymodlen Nozizeptoren zu untersuchen, wurde eine Doppelimmunfluoreszenz für VGLUT2 und TRPV1 durchgeführt. Wie Figur 24 zeigt, verwenden alle TRPV1-positiven Neurone den vesikulären Glutamat-Transporter VGLUT2 (C, D). Dabei wurde VGLUT2 vor allem im Zellsoma und dem Axon aufgefunden (D). In Figur 24 (E, F) sind die Häufigkeitsverteilungen für die Zellgrößen der VGLUT2-positiven Neurone (E) bzw. der TRPV1-positiven Neurone (F) dargestellt. Es zeigt sich, dass die TRPV1-positiven Neurone, mit einer durchschnittlichen Zellgröße von ~180 µm², eine Subpopulation der kleineren und mittleren VGLUT2-positiven Neurone ausmachen, die eine durchschnittliche Zellgröße von ~200µm² aufweisen.

### Ausführungsbeispiel 4

***In-vivo*-Versuche zur Wirksamkeit von siRNA gegen VGLUT2 bei der Schmerzbehandlung *in vivo***

Hierzu wurde das Schmerzmodell der Ratte nach Bennett eingesetzt.

Die analgetische Wirkung der erfindungsgemäßen siRNA wurde im Ratten-Modell *in vivo* untersucht. Hierzu wurde als aktive Komponete eine gegen die Targetsequenz von VGLUT2 AAGCAGGATAACCGAGAGACC gerichtete siRNA eingesetzt . Die beiden Stränge dieser doppelsträngigen siRNA haben die folgenden Sequenzen: r(GCAGGAUAACCGAGAGACC)dTT bzw. r(GGUCUCUCGGUUAUCCUGC)d(TT).

Die Kontroll-siRNA ist gegen die folgende Targetsequenz (AAGGACTAGCA-AAGCGAGCCA) (keine VGLUT2-Sequenz) gerichtet. Die Stränge der doppelsträngigen Kontroll-siRNA haben jeweils die folgende Sequenzen: r(GGACUAGCAAAGCGAGCCA)d(TT) bzw. r(UGGCUCGCUUUGCUAGUCC)d(TT).

Alle vorgenannten Sequenzen wurden chemisch synthetisiert (Xeragon, Germantown, USA) und ihre Reinheit mittels MALDI-TOF Analyse überprüft. Gelöst wurden die Sequenzen in 0,9%iger NaCl-Lösung.

Neuropathischer Schmerz tritt u.a. nach Schädigung peripherer oder zentraler Nerven auf und lässt sich dementsprechend tierexperimentell durch gezielte Läsionen einzelner Nerven induzieren und beobachten. Ein Tiermodell ist die Nervenläsion nach Bennett (Bennett und Xie (1988) Pain 33: 87-107). Im Bennett-Modell wird der Ischiasnerv unilateral mit losen Ligaturen versehen. Es ist die Entwicklung von Anzeichen des Neuropathieschmerz zu beobachten und kann mittels thermischer oder mechanischer Allodynie quantifiziert werden.

Hierzu wurden 5 männliche Sprague-Dawley-Ratten (Janvier, Frankreich) mit einem Gewicht von 140 bis 160 Gramm zunächst mit Pentobarbital (50 mg pro kg Körpergewicht der Ratte Nembutal®, i.p., Sanofi, Wirtschaftsgenossenschaft deutscher Tierärzte eG, Hannover, Deutschland) betäubt. Anschließend wurden einseitige mehrfache Ligaturen am rechten Hauptischiasnerv der Ratten vorgenommen. Hierzu wurde der Ischiasnerv auf der Höhe der Oberschenkelmitte freigelegt und vier lockere Ligaturen (softcat®chrom USP 4/0, metric2, Braun Melsungen, Deutschland) wurden so um den Ischiasnerv gebunden, daß die epineurale Durchblutung nicht unterbrochen wurde. Der Operationstag war Tag 1. Die Messung erfolgte 1 Woche nach Nervenligatur.

Die Allodynie wurde auf einer Metallplatte getestet, die mit Hilfe eines Wasserbads auf eine Temperatur von 4°C temperiert wurde. Zur Überprüfung der Allodynie wurden die Ratten auf die kalte Metallplatte gesetzt, die sich in einem Plastikkäfig befand. Dann wurde über einen Zeitraum von zwei Minuten vor der Applikation einer Lösung gezählt, wie häufig die Tiere mit ihrer geschädigten Pfote von der gekühlten Metallplatte heftig zurückzucken (Vorwert). Dann wurden die Lösungen, enthaltend 3,16 µg (5 µl) erfindungsgemäße siRNA in 15 µl NaCl oder 3,16 µg (5 µl) Kontroll-RNA (Sense-Strang der siRNA) in 15 µl NaCl i.t. oder NaCl-Lösungen (5 µl) zur Kontrolle nach einmaliger akuter intrathekaler Applikation unter Ethernarkose appliziert, und die Anzahl der Wegziehreaktionen wurde jeweils nach 60 min wiederum für 2 min gezählt (Testwert). Die Messungen wurden an 4 aufeinanderfolgenden Tagen, jeweils im Abstand von 24h (Tage 2 bis 5) jeweils für drei verschiedene Dosierungen (0.001, 0.01 und 0.1 µg/Tier) durchgeführt. Tiere, denen reine NaCl-Lösung appliziert wurde, dienten sowohl bei den Experimenten mit siRNA als auch mit Kontroll-RNA als Vergleichsgruppe.

Die erfindungsgemäße siRNA gegen VGLUT2 zeigte in diesem Schmerzmodell eine starke analgetische Wirkung, nämlich eine deutliche Hemmung der Kälte-Allodynie ohne Dosisabhängigkeit mit der besten Wirkung bei der niedrigsten Dosisgruppe (1ng/Tier). In der höchsten Dosis zeigten die Tiere eine gesteigerte Spontanaktivität sowohl bei der Kontrolle als auch beim Verum. Dies könnte die Ursache für die schwächere Wirkung in der hohen Dosisgruppe sein. Weitere Nebenwirkungen traten nicht auf. (s. auch Figur 28)

Die in den vorgenannten Ausführungsbeispielen genannten und angewandten Materialien und Methoden bzw. konkreten experimentellen Bedingungen werden im folgenden näher beschrieben:

### 1. Verwendete Materialien

### 1.1 Versuchstiere

Alle adulten Versuchstiere waren männliche oder weibliche Wistar-Ratten (300g), und wurden von Charles River (Sulzfeld) bzw. von der Deutschen Versuchstieranstalt (Hannover) bezogen. Die Tiere wurden in einem 12h/12h Tag-Nacht-Rhythmus, mit freiem Zugang zu Futter und Wasser, gehalten. Zwischen der Anlieferung der Tiere und dem Versuchsbeginn lagen mindestens 4 Tage, in denen der Gesundheitszustand der Tiere überwacht wurde. Die neonatalen Ratten stammten aus einer eigenen Zucht männlichen und weiblichen Wistar-Ratten, die in regelmäßigen Abständen gedeckt wurden. Alle neonatalen Tiere wurden von P0 (postnatal Tag 0) bis P5 für die Herstellung von Primärkulturen verwendet.

### 1.2 Zellinien

### wt-PC12

Die immortale Tumorzelllinie PC12 wurde 1976 aus einem Tumor des Nebennierenmarks der Ratte isoliert (Greene and Tischler, 1976). Die Zellen wachsen nicht adhärent, führen zu transplantierbaren Tumoren in Ratten und reagieren reversibel auf NGF (nerve growth factor) mit der Ausbildung neuronenähnlicher Fortsätze. Die PC12 Zelllinie wurde dem Labor von der Firma Grünenthal (Aachen) zur Verfügung gestellt, welche die Zelllinie ihrerseits von ATCC bezog. Die Zellen wurden in einem modifizierten DMEM Medium in Kultur gehalten. Im Folgenden werden diese PC12-Zellen zur besseren Unterscheidung als wt-PC12 (Wildtyp) bezeichnet.

### PC12 MR-A und PCR12 MR-B

Bei diesen beiden Zelllinien handelte es sich um mutierte oder differenzierte Varianten der wt-PC12 Zellen, die ursprünglich von Dr. Reiner Westermann (Institut für Anatomie und Zellbiologie, Marburg) von ATCC bezogen wurden. Die Zellen wuchsen im Gegensatz zu ihren wt-Mutanten adhärent, waren flach und spindelförmig und bildeten bereits ohne Zugabe von NGF Fortsätze. Hinweise in der Literatur gaben Anlass zu der Vermutung, dass diese Varianten eine glutamatergen Phänotyp ausbilden könnten (Jimenez et al., 2003; Zheng et al., 1996).

### 1.3 Bakterienstämme

E. coli-Stamm DH5 α Clontech (Heidelberg)
E. coli-Stamm XL-1 blue Clontech (Heidelberg)

### 1.4 Geräte

### Zentrifugen

Tischzentrifuge Typ Biofuge pico Heraeus (Hanau)
Labofuge III Heraeus (Hanau)
Kühlzentrifuge, 5403 Eppendorf (Hamburg)
Zentrifuge JS21 Beckmann (München)

### Inkubatoren

Inkubationsofen (16/37°C) WTB Binder (Reiskirchen)
Inkubator (37°C / 5% CO₂) Heraeus (Hanau)
Inkubator (37°C / 5% CO₂) Heraeus (Hanau)

### Elektrophorese

Agarosegelelektrophoresezubehör Kodak/Integra (New Haven)
Geldokumentationsanlage, Gel Doc 1000 BIO-RAD (München)

### Mikroskope und digitale Bildanalyse

Confokales Laser-Scanning-Mikroskop Olympus (Hamburg)
Mikroskop, AX70 Olympus (Hamburg)
Mikroskop, IX70 Olympus (Hamburg)
Olympus SZH10 research stereo Olympus (Hamburg)
MCID M4 Bildanalysesystem Imaging Research (St. Catharines, Kanada)
SPOT Camera Diagnostics Instruments Inc. (Seoul, Korea)
SPOT image analyses (Version 3.4) Diagnostics Instruments Inc. (Seoul)

### Sonstige Geräte und Verbrauchsmaterialien

Absaugvorrichtung Neo-Lab, Vogel (Heidelberg)
Autoklav Integra BioSciences Inc. (Woburn, USA)
Autoradiographiekassette Amersham (Little Chalfont, England)
Bakterienschüttler, Certomat H Braun Biotech (Melsungen)
Deckgläser Menzei (Braunschweig)
Einmalpipetten Greiner bio-one (Frickenhausen)
Gewebekulturflaschen Cellstar^{®}Greiner bio-one (Frickenhausen)
Heizplatte Medax (Kiel)
Kolbenhubpipetten Eppendorf (Hamburg)
Kryoröhrchen Nalge Nunc International (Rochester, USA)
Kryostat CM 3050 Leica (Nussloch)
Neubauer-Zählkammer Marienfeld (Lauda-Königshofen)
Objektträger Menzel (Braunschweig)
Peltier Thermal Cycler PTC-200 MJ Research (Watertown, USA)
pH-Meter 766 Knick (Berlin)
Petrischalen Nalge Nunc International (Rochester, USA)
Pipettenspitzen Eppendorf (Hamburg)
Plastikwannen Neolab (Heidelberg)
Reinstwasseranlage (Milli-Q) Millipore (Billerica, USA)
Speedvac (Kühlfalle) Heraeus (Hanau)
Stereotaktischer Apparat David Kopf Instruments (Tujunga, USA)
Taumler Polymax 1040 MJ Research (New York, USA)
Thermometer IKA Labortechnik (Staufen)
Umluftofen Memmert (Schabach)
Videoprinter Mitsubishi Electric (Tokio, Japan)
Vortex: VF2 und MS2 Minishaker IKA (Staufen)
Vortex Genie 2 Bender & Hohenheim AG (Zürich, Schweiz)
Waage 1012 MP Sartorius (Göttingen)
Wasserbad Julabo 5 Julabo (Seelbach)
Wasserbad Memmert (Schabach)
Zellkulturschalen Nunclon™ (6/24/96 Loch) Nalge Nunc International (Rochester, USA)

### 1.5 Chemikalien

Alle hier nicht im einzelnen aufgeführten Chemikalien wurden, soweit im Methodenteil nicht anders vermerkt, von den Firmen Fluka (Buchs, Schweiz), GibcoBRL (Eggenstein), Merck (Darmstadt), Riedel de Haen (Seelze), Roche (Basel, Schweiz), Roth (Karlsruhe), Serva (Heidelberg) und Sigma (Deisenhofen) bezogen.
Bacto Agar Difco (Detroit, USA)
Bacto Tryptone Difco (Detroit, USA)
Bacto Yeast Extract Difco (Detroit, USA)
Fixierlösung Formamid BDH (Poole, England)
NaOH Baker (Deventer, Holland)
Ketamin Parke-Davis (Freiburg)
Paraffin Vogel Histo-Comp
x-GAL Invitrogen (Karlsruhe)
Xylazin Bayer (Leverkusen)

### 1.6 Puffer und Lösungen

| | |
|---|---|
| H₂O MilliQ | gereinigt und autoklaviert |
| | |
| PBS (phosphate buffered saline): | 6.5 mM Na₂HPO₄ |
| | 1.5 mM KH₂PO₄ |
| | 2.5 mM KCl |
| | 140 mM NaCl pH 7,25 |
| | |
| 6x Probenpuffer (DNA/RNA): | 50% (w/v) Glycerin |
| | 1 mM EDTA |
| | 0,4% (w/v) Bromphenolblau |
| | 0,4% (w/v) Xylencyanol |
| | |
| TE (Tris EDTA): | 10 mM Tris-Base, |
| | 1 mM EDTA pH 8,0 |
| | |
| TAE (Tris- Acetat- EDTA): | 1 x TAE 40mM Tris- Base, 2m M EDTA 50x |
| | 242 g Tris Base |
| | 57,1 ml Eisessig |
| | 37,2 g Na₂EDTA x H₂O |
| | ad 1 l H₂O pH 8,5 |

### 1.7 Medien und Medienzusätze

### Grundmedien

Alle Medien wurden von GibcoBRL (Eggenstein) bezogen:
DMEM (Dulbecco's Modifiziertes Eagle Medium) mit hohem Glucoseanteil Ham's F12
HBSS (10x) Ca²⁺/Mg²⁺-frei
OptiMEM (serumreduziert)
RPMI 1640 (Roswell Park Memorial Institute)

### Medienzusätze

Antibiotika-Mix GibcoBRL (Eggenstein)
Cytosinarabinosid Sigma (Deisenhofen)
Fetales Kälberserum (FCS) Sigma (Deisenhofen)
HyClone FBS GibcoBRL (Eggenstein)
HAT Supplement GibcoBRL (Eggenstein)
Glutamin GibcoBRL (Eggenstein)
Pferdeserum GibcoBRL (Eggenstein)
Nervenwachstumsfaktor NGF 7S Sigma (Deisenhofen)

### Zusammensetzung der Wachstumsmedien

| | |
|---|---|
| **Medium für PC12-Zellen:** | **RPMI mit** |
| | 10% Pferdeserum |
| | 5% FCS |
| | 1% Glutamin (200 mM) |
| | 1% Antibiotika Mix (100x) |
| | |
| **Medium für Spinalganglienzellen:** | **DMEM mit** |
| | 10% FCS |
| | 1% Antibiotika Mix (100x) |
| | 1% Glutamin |
| | 10 µM Cytosinarabi nosid |
| | 25 ng/ml NGF |

### 1.8 Enzyme und andere Proteine

| | |
|---|---|
| Collagenase (267 U/ml) | Seromed, Biochrom (Berlin) |
| Dispase | GibcoBRL (Eggenstein) |
| DNA-Polymerase AmpliTaq Gold™ | Applied Biosystems (Foster City, USA) |
| DNase (LS002139) | Worthington |
| Poly-L-Lysin, Hydrobromid | Sigma (Deisenhofen) |
| Poly-D-Lysin, MG > 300 kDa | Sigma (Deisenhofen) |
| Restriktionsendonukleasen | Boehringer (Mannheim) |
| RNA-Polymersen (SP6, T3, T7) | Boehringer (Mannheim) |
| RNase-free DNase I | Boehringer (Mannheim) |
| RNase (A, T1) | Boehringer (Mannheim) |
| RNAsin | Fermentas (Vilnius, Litauen) |
| Superscript^{™} II H- Reverse Transkriptase | GibcoBRL (Eggenstein) |
| | T4 DNA-Ligase Biolabs (Beverly, USA) |
| Trypsin/EDTA (0,5%/0,2%) | GibcoBRL (Eggenstein) |
| Trypsin | GibcoBRL (Eggenstein) |

### 1.9 Nukleinsäuren und Vektoren

| | |
|---|---|
| 100 bp-DNA-Ladder | GibcoBRL (Eggenstein) |
| 1 kb-DNA-Ladder | GibcoBRL (Eggenstein) |
| ATP, CTP, GTP, UTP | Boehringer (Mannheim) |
| Transkriptionsvektor (pGEM-T) | Promega (Madison, USA) |
| Expressionsvektor (pCR3.1) | Invitrogen (Karlsruhe) |
| Oligo(dT)15-18 primer | Boehringer (Mannheim) |

### 1.10 Oligonukleotide

Alle Oligonukleotide wurden mit dem Programm Oligo 4.0 selbst entworfen, mit BLAST auf ungewollte Sequenzhomologien überprüft und die Synthese bei MWG-Biotech (Ebersberg) in Auftrag gegeben.

### PCR-Primer:

| **rVGLUT1** | | |
|---|---|---|
| rVGluT1 (2_4)F | 5'-TCTGGGTTTCTGCATCAGC-3' | |
| rVGluT1(2_4)R | 5'-CCATGTATGAGGCCGACAGT-3' | PCR-Produktgröße: 153 bp |

| **rVGLUT2** | | |
|---|---|---|
| rVGluT2(8_9)F | 5'-AAGACCCCATGGAGGAAGTT-3' | |
| rVGluT2(8_9)R | 5'-ATTGTCATGACCAGGTGTGG-3' | PCR-Produktgröße:184 bp |

| **rVGLUT3** | | |
|---|---|---|
| rVGluT3(4_5)F | 5'-ATCCAGAGACGGTGGGTCTT-3' | |
| rVGluT3(4_5)R | 5'-ATGACACAGCCGTAATGCAC-3' | PCR-Produktgröße: 185 bp |

| **rGAPDH** | | |
|---|---|---|
| rGAPDH(7_8)F | 5'-ATCCTGGGCTACACTGAGGA-3' | |
| rGAPDH(7_8)R | 5'-ATGTAGGCCATGAGGTCCAC-3' | PCR-Produktgröße: 162 bp |

### Primer für siRNA-Synthese mittels siRNA-Construction Kit (Ambion):

| **für siRNA si-rVGLUT2 100-120 AMB** | |
|---|---|
| rVGluT2 TS10 as | 5'-AAGCAGGATAACCGAGAGACCCCTGTCTC-3' |
| rVGluT2 TS10 s | 5'-AAGGTCTCTCGGTTATCCTGCCCTGTCTC-3' |

| **für siRNA si-rVGLUT2 166-186 AMB** | |
|---|---|
| rVGluT2 TS14 as | 5'-AAGGCTCCGCTATGCGACTGTCCTGTCTC-3' |
| rVGluT2 TS14 s | 5'-AAACAGTCGCATAGCGGAGCCCCTGTCTC-3' |

### 1.11 Kits

| | |
|---|---|
| qPCR Core kit for SYBR^{®} Green | Eurogentec (Liege, Belgien) |
| RNeasy Mini Kit | QIAGEN (Hilden) |
| Silencer^{™} siRNA Construction Kit | Ambion (Austin, USA) |
| Silencer^{™} siRNA Labeling Kit | Ambion (Austin, USA) |
| QIAamp DNA Mini Kit | QIAGEN (Hilden) |
| QIAprep Spin MAxiprep Kit | QIAGEN (Hilden) |
| QIAprep Spin Miniprep Kit | QIAGEN (Hilden) |
| QIAquick Gel Extraction Kit | QIAGEN (Hilden) |
| QIAquick Nucleotide Removal Kit | QIAGEN (Hilden) |
| QIAquick PCR Purification Kit | QIAGEN (Hilden) |
| Vectastain Elite Avdin-Biotin-Blocking Kit Vector Laboratories | |
| Vectastain Elite ABC Kit | Vector Laboratories |

### 1.12 Antikörper und Detektionssysteme

Die unterschiedlichen Zelltypen wurden mit Primärantikörpern gegen allgemein anerkannte, gut charakterisierte Epitope (Marker) im jeweiligen Zelltyp nachgewiesen. Diese sind PGP9.5 für Neurone, GFAP für Astrozyten und S100 für Oligodendrozyten. Neben diesen wurden weitere Antikörper gegen spezielle Proteine verwendet. Die optimalen Konzentrationen der primären Antikörper wurden jeweils austitriert. Die Tabelle 1 gibt eine Übersicht der verwendeten Primärantikörper und auch deren Arbeitsverdünnungen wieder.

Der Nachweis der Primärantikörper erfolgte dann durch Fluorochom-gekoppelte Sekundärantikörper oder Fluorochrom-gekoppeltes Streptavidin (Tabelle 2), das mit Spezies-spezifischen biotinylierten Antikörpern interagierte. Als Fluorochrome wurden Cy3 (rote Fluoreszenz) oder Alexa 488 (grüne Fluoreszenz) verwendet.

**Tab. 1: Liste der verwendeten Primärantikörper**

| **Antigen** | **interne Bezeichnung** | **Quelle** | **Verdünnung** | **Spezies** |
|---|---|---|---|---|
| **β-III Tubulin** | Tubulin | DPC/Biermann (Bad Nauheim) | 1:4 | Maus, monoklonal |
| **CGRP** | CGRP/Ste | Fred Nyberg, Stefan Persson | 1 :8000 | Kaninchen |
| **GFAP** | GFAP-Dako | DakoCytomation (Dänemark) | 1:5000 | Kaninchen |
| **GFAP** | GFAP | Boehringer (Mannheim) | 1:4 | Maus, monoklonal |
| **PGP9.5** | PGP | UltraClone (Wellow, England) | 1:1500 | Kaninchen, polyklonal |
| **S100** | S100 | Biogenesis (Poole, England) | vorverdünnt | Kaninchen, polyklonal |
| **Substanz P** | SP | Lee Eiden (NIH, USA) | 1:1000 | Kaninchen |
| **TRPV1** | Caps.-Rezeptor (SA 6583) | Eurogentec (Belgien) | 1:250 | Kaninchen |
| **TRPV2** | VRL-1 | Chemicon (Temecula, USA) | 1 :400 | Kaninchen, polyklonal |
| **VGLUT1** | BNPI/BC66 | J. Erickson (New Orleans) (Varoqui et al., 2002b) | 1:600 | Kaninchen |
| **VGLUT1** | BNPI/p437/7/01 | J. Erickson (New Orieans) (Varoqui et al., 2002b) | 1:800 | Meerschweinchen |
| **VGLUT2** | DNPI/p438/7101 | J. Erickson (New Orleans) (Varoqui et al., 2002b) | 1:800 | Meerschweinchen |
| **VGLUT2** | DNPI/DC68 | J. Erickson (New Orleans) (Varoqui et al., 2002b) | 1:100 | Kaninchen |
| **VGLUT3** | VGLUT3/447/bl9 | J. Erickson (New Orleans) (Schafer et al., 2002) | 1:600 | Meerschweinchen |
| **VGLUT3** | VGLUT3/94/bl5 affi. | J. Erickson (New Orleans) (Schafer et al., 2002) | 1:50 | Kaninchen |
| **VGLUT3** | VGLUT3 Chemicon | Chemicon (Temecula, USA) | 1:5000 | Meerschweinchen, polyklonal |

Zudem wurde das biotinylierte Isolektin B4 (Sigma, Deisenhofen) in einer Konzentration von 1:20 verwendet.

**Tab.2: Liste der verwendeten Sekundärantikörper**

| **Antikörper** | **Quelle** | **Verdünnung** | **Donorspezies** |
|---|---|---|---|
| Anti-Meerschweinchen-IgG-Cy3 | Dianova (Hamburg) | 1:100 | Esel |
| Anti-Maus-IgG-Cy3 | Dianova (Hamburg) | 1:100 | Esel |
| Anti-Kaninchen-IgG-Cy3 | Dianova (Hamburg) | 1:100 | Esel |
| Anti-Meerschweinchen-IgG-A488 | Dianova (Hamburg) | 1:100 | Esel |
| Anti-Maus-IgG-A488 | Dianova (Hamburg) | 1:100 | Esel |
| Anti-Kaninchen-IgG-A488 | Dianova (Hamburg) | 1:100 | Esel |
| Avdin-Biotin-Peroxidase | Boehrinher (Mannheim) | 1:500 | Esel |
| Streptavidin-Alexa 488 | MoBiTec (Göttingen) | 1:200 | Esel |

### 2. Methoden

### 2.1 Gewebeentnahme und -aufbereitung

Die Tiere wurden durch CO₂-Inhalation und anschließende Dekapitation getötet. Anschließend wurde das benötigte Gewebe für die verschiedenen Verwendungszwecke auf unterschiedliche Arten entnommen:

### Nukleinsäureextraktion:

Das Gewebe wurde nach der Entnahme auf Eis zerteilt, in ein Kryoröhrchen gegeben und nach dem Schockfrieren in flüssigem Stickstoff wurde das Gewebe zunächst auf Trockeneis und dann bei -70°C gelagert.

### Primärkultur:

Für das Anlegen von Primärkulturen wurde das Gewebe (Spinalganglien) zügig auf selbst hergestellten, mit Eis gekühlten Präparationsschalen entnommen und zunächst in 1x CMF Medium bis zur weiteren Aufbereitung gesammelt. Das calcium- und magnesiumfreie 1 x CMF Medium bestand aus 10% HBSS (10x), 1 % Antibiotika Mix und 0,2% Phenolrot (0,5%). Der pH-Wert wurde mit Bikarbonat (7,5%) titriert und war an der kirschroten Farbe des Indikators zu erkennen.

### 2.2 Anlegen von Primärkulturen

Für das Anlegen der Primärkulturen wurden neonatale Ratten der Stadien P0 bis P5 für die Spinalganglienzellkultur bzw. P0 bis P3 für die Kleinhirnkulturen verwendet. Die Tiere wurden mit Alkohol desinfiziert und mit einer sterilen Schere durch Dekapitation getötet.

### 2.2.1 Neuronale Primärkulturen aus Spinalganglien

Die Präparation der Spinalganglien erfolgte nach einem modifizierten Protokoll von C. Grothe (Grothe and Unsicker, 1987).

### Dissektion der Spinalganglien

Der Körper der getöteten Ratte wurde ventral aufliegend auf einer Korkplatte fixiert und die Wirbelsäule durch Entfernung der Haut, sowie der Nacken- und Schultermuskulatur freigelegt. Anschließend wurde die Wirbelsäule von caudal nach cranial eröffnet und das Rückenmark dargestellt. Bei den juvenilen Tieren wurde das Rückenmark im Spinalkanal belassen und die geöffnete Wirbelsäule in toto herauspräpariert, um es in einer gekühlten Präparationsschale zu fixieren. Erst dann wurde das Rückenmark vorsichtig schrittweise entfernt und aus den freigelegten Intervertebrallöchern die Spinalganglien entnommen.

Die dissektierten Spinalganglien wurden bis zur Feinpräparation in einer gekühlten Petrischale mit 1x CMF Medium gesammelt. Unter dem Binokular wurde das Spinalganglion von Nerven, Bindegewebe und Blutresten gesäubert und anschließend in ein mit 1x CMF Medium gefülltes und auf Eis gekühltes Röhrchen überführt.

### Dissoziation und Aufreinigung der Spinalganglienzellen

Nach Entfernen des Mediums wurden die Spinalganglien zur chemischen Dissoziation mit einem Enzymgemisch aus 0,075% Collagenase und 0,15% Dispase in CMF Medium 30 bis 45 min bei 37°C und 5% CO₂ inkubiert. Durch mehrmaliges Aufschütteln während der Inkubation sollte ein Aneinanderhaften der Ganglien verhindert werden. Die Hälfte des Enzymgemisches wurde nach Ende der Inkubation entfernt und zum verbliebenen Rest das gleiche Volumen einer 0,25%igen Trypsinlösung zugeführt. Nach 15 bis 25 min Inkubation bei 37°C und 5% CO₂ wurde die Enzymlösung bis auf 300 µl entfernt und die Ganglien in diesem Volumen mechanisch dissoziiert. Dazu wurden drei silikonisierte sterile Pasteurpipetten mit unterschiedlich großen Pipettenöffnungen, die unter der Bunsenbrennerflamme hergestellt wurden, verwendet. Durch zunehmende Verringerung des Öffnungsdurchmessers konnte so eine einheitliche Zellsuspension der Spinalganglien hergestellt werden. Die Zellsuspension wurde schließlich bei 1.000 U/min für 5 min zentrifugiert, der Überstand verworfen und das Zellpellet in ~1 ml CMF resuspendiert. Zur Anreicherung der Kultur mit neuronalen Zellen wurde die Zellsuspension über eine 20%ige BSA-Säule (20% BSA w/v in 0,5 ml CMF Medium) geschichtet und anschließend bei 1.000 U/min für 5 min zentrifugiert. Das Medium wurde bis auf ~ 100 µl abgesaugt und in 1 ml frischem Medium resuspendiert. Hierfür wurde bereits das spätere Kulturmedium (DMEM/FCS) verwendet.

### Ausplattieren der Zellen

Nach Bestimmung der Zellzahl wurden die Spinalganglienzellen entsprechend der weiteren Verwendung auf die Kulturgefäße verteilt und bei 37°C und 5% CO₂ in kultiviert. Zur Kultivierung wurde ein glukosereiches DMEM Medium mit verschiedenen Zusätzen verwendet (genaue Zusammensetzung unter dem Punkt Zellkultur beschrieben). Neben Glutamin und einem Antibiotika Mix wurde der Nervenwachstumsfaktor NGF 7S in einer Konzentration von 25 ng/ml zugeführt, da das Überleben und die Differenzierung der neonatalen Neurone des Spinalganglions NGF-abhängig sind. Zudem wurde der Mitosehemmer Cytosinarabinosid in einer Konzentration von 10 µM dem Medium zugesetzt, um eine Proliferation der nicht-neuronalen Zellen zu verhindern.

Die ausplattierten Spinalganglienzellen konnten bei einem Mediumwechsel alle zwei bis drei Tage bis zu zwei Wochen problemlos in Kultur gehalten werden.

### 2.3 Eukaryonten-Zellkultur

### Kultivierung und Passagieren von Eukaryontenzellen

Alle oben aufgeführten Zelllinien wurden in ihrem jeweiligem Wachstumsmedium bei 37°C im Begasungsbrutschrank bei 95% rel. Luftfeuchte und 5% CO₂. Medien und Lösungen wurden vor Benutzung auf 37°C erwärmt.

Die adhärent wachsenden Zelllinien (F-11, wt-PC12 MR-A, PC12 MR-B) wurden routinemäßig alle 4-6 Tage gesplittet. Hierzu wurden sie bei 37°C einige Minuten lang mit Trypsin/EDTA-Lösung (0,05% Trypsin, 0,02% EDTA in PBS) behandelt (mikroskopische Kontrolle). Trypsin ist ein proteolytisches Enzym, das Peptidbindungen von Zell-Zell-Verbindungen hydrolysiert bei denen die Carbonylgruppe vom Lysin oder Arginin gestellt wird. Geringe Spuren von Medium können die Wirkung von Trypsin beeinträchtigen, weswegen die Zellen vor der Trypsinbehandlung mit phosphatgepufferter calcium- und magnesiumfreier Salzlösung (PBS) gewaschen werden mussten. Später konnte durch Zugabe von Medium mit FCS-Zusatz das eingesetzte Trypsin wieder inaktiviert werden. Der Chelator ED-TA bindet Calcium, das von einigen Zell-Zell-Verbindungen benötigt wird. Nachdem sich die Zellen gelöst hatten, wurden sie mit serumhaltigem Medium aus dem Kulturgefäß in ein Falcon Röhrchen überführt. Nach Abzentrifugation und Absaugen des alten Mediums, wurden die Zellpellets in 5 ml frischem Medium resuspendiert und entsprechend der weiteren Verwendung auf die jeweiligen Kulturgefäße in ihrem Wachstumsmedium ausplattiert.

Bei den wt-PC12 Zellen handelt es sich um Suspensionszellen. Zum Passagieren wurden die Kulturgefäße schräg aufgestellt, sodass die in traubenförm igen Haufen wachsenden wt-PC12 Zellen allmählich in einer Ecke des Gefäßes sedimentierten. Der Überstand, in dem sich auch der leichtere Zelldebris befand, wurde entfernt und die Zellen mit frischem Medium in ein Falcon-Röhrchen überführt. Die Suspension wurde anschließend bei 1.000 U/min 5 min zentrifugiert, der Überstand entfernt und die Zellen in frischem Wachstumsmedium ausplattiert.

### Einfrieren von Eukaryontenzellen

Zur langfristigen Lagerung von Zellen wurden diese in DMSO-haltigem Medium in flüssigem Stickstoff eingefroren, um sie vor genetischen Veränderungen zu schützen und das Risiko einer Kontamination zu minimieren. Ohne Zusatz von Reagenzien, die als Kryoschutz für die Zellen wirken, sterben die meisten Säugerzellen beim Einfrieren. Die Sterblichkeit von Zellen wird durch DMSO im Medium minimiert, da der Gefrierpunkt erniedrigt und so der Abkühlvorgang verlangsamt wird.

Die Zellen wurden nach Ablösung mit Trypsin / EDTA - Lösung zunächst mit PBS gewaschen und die Zellzahl mit Hilfe der Neubauer-Zählkammer ermittelt. Die einzufrierende Zellmenge wurde in einem Falcon Röhrchen vorgelegt und der Überstand nach Abzentrifugation bei 1.000 U/min verworfen. Das Zellpellet wurde schließlich in Einfriermedium (90% Wachstumsmedium, 10% DMSO) resuspendiert und jeweils 2 ml (mit z.B. 2 Mio. Zellen) in Nunc Röhrchen aliquotiert. Die Röhrchen wurden in einem Kryo-Einfriergerät mit einer Abkühlrate von 1 °C pro Minute für mehrere Stunden bei -80°C eingefroren und anschließend in flüssigen Stickstoff eingelagert.

### Revitalisierung von Eukaryontenzellen

In einigen Fällen wurden Zellen neu in Kultur genommen. Dazu wurden sie nach Entnahme aus dem Stickstofftank (-196°C) im Wasserbad schnell auf 37°C erwärmt. Die Zellsuspension wurde entnommen, in 5 ml vorgewärmtes Medium überführt und in der Zentrifuge sedim entiert (3 min, 200 x g). Das Medium wurde abgesaugt, das Zellpellet in frischem Medium resuspendiert und in eine Zellkulturschale überführt. Am nächsten Tag wurden die Zellen mit PBS gewaschen und mit frischem Medium versorgt.

### Zellzahlbestimmung

Zur Bestimmung der Zellzahl wurde eine Neubauer-Zählkammer verwendet. Dazu wurde ein Tropfen der Zellsuspension auf die Neubauer-Zählkammer aufgetragen und die Zellzahl (Z = Zellen / 0,01 mm²) von vier Eckquadraten unter dem Mikroskop ausgezählt und die Zellzahl pro ml ermittelt (Zellzahl = Z x 2.500) (Amiri et al.).

### Mycoplasmentest

Alle Zelllinien wurde in regelmäßigen Abständen auf Mycoplsmenkontamination getestet. Mycoplasmen sind obligat parasitäre Bakterien. Sie sind wandlose, sehr kleine, intrazelluläre Parasiten und können sich nicht unabhängig von der Wirtszelle vermehren. Da sie nur eine Zellmembran, aber keine Bakterienwand aus Murein haben, sind sie ohne feste Gestalt und gegen Penicillin unempfindlich. Ihre Größe schwankt zwischen 0,22 und 2 µm. Eine Filtration durch eine Membranen mit einer Porengröße von 0,1 µm machen eine Mycoplasmenabtrennung möglich. Kontaminationen mit Mycoplasmen können am schnellsten durch die Anfärbung der Mycoplasmen-DNA mit dem speziell an DNA bindenden Fluorochrom DAPI (4-6-Diamidino-2-phenylindol-di-hydrochlorid) festgestellt werden. Bei einer Mycoplasmen-Kontamination von Zellkulturen findet man einzelne fluoreszierende Punkte im Zytoplasma und manchmal auch im interzellulären Raum.

Für den Mycoplasmentest wurde eine entsprechende Menge DAPI-Stammlösung (1 mg/ml, 10 mg DAPI in 10 ml Wasser gelöst, aliquotiert und bei -20°C gelagert) mit Methanol auf eine Arbeitskonzentration von 1 µg/ml verdünnt (bei 4°C ca. 6 Monate stabil). Zum Anfärben der Zellen wurden die auf Deckgläschen oder Petrischalen kultivierten Zellen einmal mit der Arbeitslösung gewaschen und für 15 min bei RT mit der Arbeitslösung inkubiert. Anschließend wurde einmal mit Methanol gewaschen und die Deckgläschen nach Einbettung in einem Tropfen Glycerin oder PBS unter dem Fluoreszenzmikroskop ausgewertet.

### Beschichtung von Kulturgefäßen

Für bestimmte Versuche wurden die Kulturgefäße oder Deckgläschen zum besseren Anhaften der Zellen beschichtet. Als Beschichtungsmaterialien dienten entweder Poly-L-Lysin (0,1 mg/ml) für wt-PC12 Zellen oder Poly-D-Lysin (0,5 mg/ml) - für die Cerebellum Kultur. Die Kulturgefäße wurden mind. 2 h bei RT mit Poly-L-Lysin bzw. Poly-D-Lysin inkubiert, anschließend zwei mal mit H₂O gewaschen und mit H₂O überschichtet bis zur Verwendung bei 4°C aufbewahrt. Während die Poly-L-Lysin beschichteten Materialien in feuchtem Zustand verwendet wurden, kamen die Poly-D-Lysin beschichteten Materialien erst nach Trocknung unter der Sterilbank zum Einsatz.

### Transfektion in eukaryontische Zellen

Unter einer Transfektion versteht man das Einbringen von Fremd-DNA bzw. -RNA in eukaryontische Zellen mittels physikalischer oder chemischer Methoden. Die wichtigste chemische Methode zum Transfer von Nukleinsäuren ist die Lipofektion: Reagenzien aus kationischen Lipiden formen in wässriger Lösung unter optimalen Bedingungen kleine (100 - 400 nm), unilamellare Liposomen. Die Oberfläche dieser Liposomen ist positiv geladen und wird elektrostatisch sowohl vom Phosphat-Rückgrat der Nukleinsäuren, als auch von der negativ geladenen Zellmembran angezogen (Gareis et al., 1991; Gershon et al., 1993; Smith et al., 1993). Die Nukleinsäuren sind dabei nicht innehalb der Liposomen eingeschiossen, sondern binden spontan an die positiv geladenen Liposomen und bilden DNA/RNA-Lipid-Komplexe (Felgner et al., 1987). Es gibt Hinweise darauf, dass die Aufnahme der Komplexe über den endosomalen oder lysosomalen Weg abläuft (Coonrod et al., 1997).

### Transfektion von DNA mittels Lipofektamine^{™} 2000

Für die Transfektion von DNA in eukaryontische Zellen wurde überwiegend das kationische Lipidreagenz Lipofectamine^{™} 2000 (LF2000™) verwendet und folgende allgemeine Empfehlungen des Herstellers Invitrogen beachtet:
Um DNA-LF2000™-Komplexe herzustellen wurde ein Verhältnis von DNA zu LF2000™ von 1:2 bis 1:3 empfohlen. Zudem sollte eine Zelldichte von 90-95% zum Zeitpunkt der Transfektion vorliegen, um eine hohe Effizienz und hohe Expressionslevel zu erreichen. Während der Transfektion wurde auf den Zusatz von Antibiotika verzichtet, da diese Zelltod auslösen würden. Zur Optimierung der Transfektionseffizienz wurden Transfektionen unter verschiedenen Bedingungen mit einem EGFP-Vektor durchgeführt. Zur Messung der Transfektionseffizienz wurde die Proteinexpression des grün fluoreszierenden Proteins GFP semiquantitativ mittels Fluoreszenzmikroskopie ermittelt.

### Transfektion von DNA in wt-PC12 Zellen

Am Tag vor der Transfektion wurden 2,5 x 10⁵ wt-PC12 Zellen in einer 24-Loch Platte auf Poly-L-Lysin beschichtete Deckgläschen in 0,5 ml Medium ausplattiert. Bei dem Medium handelte es sich um das normale Wachsturnsmedium für wt-PC12 Zellen, jedoch ohne Zusatz von Antibiotika. Für jedes zu transfizierende Well wurden 0,8-1 µg DNA in 50 µl OPTI-MEM^{®} gelöst. Zudem wurden für jedes Weil 1-2 µl LF2000™ in 50 µl OPTI-MEM^{®} verdünnt und für 5 min bei RT inkubiert. Die gelöste DNA wurde nun mit der verdünnten LF2000™-Lösung vermischt und zur Ausbildung der DNA-LF2000™ Komplexe für 20 min bei RT inkubiert und anschließend 100 µl der Komplexe direkt in die entsprechenden Wells gegeben, die durch vorsichtiges schwenken gemischt wurden. Die Zellen wurden unter normalen Bedingungen im selben Medium für weitere 24-72 h bis zur Expressionsanalyse in Kultur gehalten.

### Transfektion von DNA in Spinalganglienzellen

Für die Transfektion wurden 5 x 10⁵ die Zellen der frisch aufgereinigten DRG-Suspension verwendet, die in einer 24-Loch Platte auf Poly-L-Lysin beschichtete Deckgläschen in 0,5 ml Wachstumsmedium ohne Antibiotikazusatz ausplattiert wurden. Für jedes zu transfizierende Weil wurden 0,8-1 µg DNA in 50 µl OPTIMEM^{®} gelöst. Zudem wurden für jedes Weil 1-2 µl LF2000™ in 50 µl OPTI-MEM^{®} verdünnt und für 5 min bei RT inkubiert. Die gelöste DNA wurde nun mit der verdünnten LF2000™-Lösung vermischt und zur Ausbildung der DNA-LF2000™ Komplexe für 20 min bei RT inkubiert und anschließend 100 µl der Komplexe direkt in die entsprechenden Wells gegeben, die durch vorsichtiges schwenken gemischt wurden. Die Zellen wurden unter normalen Bedingung en im selben Medium für weitere 24-72 h bis zur Expressionsanalyse in Kultur ge halten.

### Transfektion von siRNA

Die Transfektionen von siRNA in eukaryontische Zellen wurde mittels Lipofectamine™ 2000 durchgeführt. LF2000™ wurde bereits von anderen Gruppen erfolgreich für RNAi-Experimente in Säugetierzellen verwendet (Gitlin et al., 2002; Yu et al., 2002). Wie bei der Transfektion von DNA mussten die optimalen Transfektionsbedingungen experimentell ermittelt werden. Dazu wurde Cy3-markierte siRNA verwendet.

### Transfektion von siRNA in wt-PC12-Zellen

Am Tag vor der Transfektion wurden 2,5 x 10⁵ wt-PC12 Zellen in einer 24-Loch Platte auf Poly-L-Lysin beschichtete Deckgläschen in 0,5 ml Medium ausplattiert. Bei dem Medium handelte es sich um das normale Wachsturnsmedium für wt-PC12 Zellen, jedoch ohne Zusatz von Antibiotika. Für jedes zu transfizierende Weil wurden 20 - 100 pmol siRNA in 50 µl OPTI-MEM^{®} gelöst. Zudem wurden für jedes Weil 1-2 µl LF2000™ in 50 µl OPTI-MEM^{®} verdünnt und für 5 min bei RT inkubiert. Die gelöste siRNA wurde nun mit der verdünnten LF2000^{™}-Lösung vermischt und zur Ausbildung der DNA-LF2000^{™} Komplexe für 20 min bei RT inkubiert und anschließend 100 µl der Komplexe direkt in die entsprechenden Wells gegeben und durch vorsichtiges schwenken gemischt. Die Zellen wurden unter normalen Bedingungen im selben Medium für weitere 24-72 h bis zur Expressionsanalyse in Kultur gehalten.

### Transfektion von siRNA in Zellen der Linien PC12 MR-A und PC12 MR-B

Am Tag vor der Transfektion wurden 2,5 x 10⁵ PC12 Zellen in einer 24-Loch Platte auf Deckgläschen in 0,5 ml Medium ausplattiert. Bei dem Medium handelte es sich um das normale Wachstumsmedium für PC12 MR-A/B Zellen, jedoch ohne Zusatz von Antibiotika. Für jedes zu transfizierende Weil wurden 20 - 200 pmol siRNA in 50 µl OPTI-MEM^{®} gelöst. Zudem wurden für jedes Weil 1-2 µl LF2000™ in 50 µl OPTI-MEM^{®} verdünnt und für 5 min bei RT inkubiert. Die gelöste siRNA wurde nun mit der verdünnten LF2000™-Lösung vermischt und zur Ausbildung der DNA-LF2000™ Komplexe für 20 min bei RT inkubiert und anschließend 100 µl der Komplexe direkt in die entsprechenden Wells gegeben und du rch vorsichtiges schwenken gemischt. Die Zellen wurden unter normalen Bedingungen im selben Medium für weitere 24-72 h bis zur Expressionsanalyse in Kultur gehalten.

### Transfektion von siRNA in Zellen der DRG-Primärkulturen

Für die Transfektion wurden 5 x 10⁵ die Zellen der frisch aufgereinigten DRG-Suspension verwendet, die in einer 24-Loch Platte auf Poly-L-Lysin beschichtete Deckgläschen in 0,5 ml Wachstumsmedium ohne Antibiotikazusatz ausplattiert wurden. Für jedes zu transfizierende Weil wurden 20 - 200 pmol siRNA in 50 µl OPTI-MEM^{®} gelöst. Zudem wurden für jedes Weil 1-2 µl LF2000™ in 50 µl OPTIMEM^{®} verdünnt und für 5 min bei RT inkubiert. Die gelöste siRNA wurde nun mit der verdünnten LF2000™-Lösung vermischt und zur Ausbildung der DNA-LF2000™ Komplexe für 20 min bei RT inkubiert und anschließend 100 µl der Komplexe direkt in die entsprechenden Wells gegeben und durch vorsichtiges schwenken gemischt. Die Zellen wurden unter normalen Bedingungen im selben Medium für weitere 24-72 h bis zur Expressionsanalyse in Kultur gehalten.

### Toxizitätsstudie

Um den zytotoxischen Einfluss der Transfektionsreagenzien LF2000™ auf die Zellen zu untersuchen, wurde ein Assay mit Trypanblau durchgeführt. Dazu wurden 24 h vor der Transfektion 3,0 x 10⁴ Zellen in eine 24-Loch Platte ausplattiert. Die Transfektion erfolgte mit siRNA-Negativkontrollen und unterschiedlichen Mengen Transfektionsreagenzien. Alle Zellen wurden 48 h nach der Transfektion mit PBS gewaschen und mit 10% Trypanblau gefärbt. Die Überlebensrate wurde folgendermaßen berechnet: $Vitalitätsrate = \left(Gesamtzellzahl - Anzahl gefärbter Zellen\right) / Gesamtzellzahl \times 100.$

### Fixierung von Zellen

Die Zellen wurden entweder mit 3-4% (v/v) Paraformaldehyd in PBS für 20 min bei RT oder mit Methanol (-20°C) für 15 min bei RT fixiert.

### 2.4 Mikrobiologie

Zur Amplifikation von doppelsträngigen DNA-Fragmenten wurden kompetente Bakterien verwendet. Dazu wurde die zu amplifizierende DNA in Plasmide mit Selektionsmarkern eingebaut und in Bakterien transformiert. Die Bakterien wurden auf selektiven Agarplatten ausplattiert, entsprechende Klone entnommen und in antibiotikumhaltigem LB-Medium gezüchtet. Von den vermehrten Bakterien wurden später die Plasmide mit Insert isoliert und weiter bearbeitet.

### Herstellung transformationskompetenter Zellen

Eine Behandlung mit eiskalter Magnesium- und Calciumchloridlösung befähigt die Bakterien zur spontanen Aufnahme von Fremd-DNA (Transformation). Zur Herstellung der kompetenten Bakterienzellen wurden die E. coli-Stämme DH5 α, bzw. XL-1 blue verwendet. Dazu wurde eine Kultur der Zellen in TYM-Medium (LB-Medium + 10 mM MgSO₄) angesetzt und diese inkubiert, bis eine ausreichende Dichte (OD600nm = 0,4 - 0,6) erreicht wurde. Die Zellen sollten sich in der log-Phase befinden. Nach kurzer Zentrifugation und Abgießen des Überstandes wurden das Pellet in 40 ml Tfb1-Puffer (30 mM KAc, 50 mM MgCl₂ x 2H₂O, 100 mM KCl, 10 mM CaCl₂ x 2H₂O, 15% Glyzerol) pro 100 ml Kultur resuspendiert. Nach 45 min Inkubation auf Eis wurden die Zellen wieder pelletiert und in 1/10 Volumen Tfb2-Puffer (10 mM Na-MOPS, 10 mM KCl, 75 mM CaCl₂ x 2H₂O, 15% Glyzerol) aufgenommen. Die kompetenten Zellen wurden bis zum Gebrauch als 100 µl Aliquots bei -70°C gelagert.

### Transformation von E. coli

Kompetente Bakterien (etwa 10⁸ Klone pro µg Plasmid-DNA) wurden mit Plasmid DNA versetzt, 45 min auf Eis und knapp 1 min bei 42°C inkubiert. Danach wurde LB-Medium (Sambrook *et al.,* 1989) zugegeben und unter Schütteln weitere 30 min bei 37°C inkubiert. Die Bakterien wurden auf selektiven Agarplatten (Sambrook *et al*., 1989) vereinzelt und über Nacht bei 37°C bebrütet. Vorkulturen (5 ml LB-Medium mit einem geeignetem Antibiotikum) wurden mit vereinzelten Kolonien angeimpft und für mehrere Stunden bei 37°C geschüttelt. Präparative Kulturen (100 ml LB mit einem geeigneten Antibiotikum) wurden 1:1.000 mit den Vorkulturen angeimpft und unter Schütteln über Nacht bei 37° inkubiert.

### Analytische Plasmidisolierung

Um die Plasmide aus den Bakterien zu isolieren, müssen diese lysiert werden. Anschließend werden die Plasmide, durch Zentrifugation von Proteinen und genomischer Bakterien-DNA getrennt. Die Aufreinigung der Plasmide geschah säulenchromatographisch mit käuflichen Kits von QIAGEN.

Die Bakterien wurden durch Zentrifugation für 5 min bei 5000 x g pelletiert, der Überstand verworfen und das Pellet in 250 µl Puffer P1 resuspendiert. Durch Zugabe von 250 µl Puffer P2 wurden die Bakterien lysiert, weitere 350 µl Puffer N3 sorgten für Neutralisierung der Lösung. Das Gemisch wurde anschließend für 10 min zentrifugiert (soweit nicht anders angegeben erfolgten die Zentrifugationsschritte bei Höchstgeschwindigkeit), der Überstand vorsichtig entnommen und in eine QIAprep Säule überführt. Nach 1 min Zentrifugation wurde die Säule mit 750 µl Puffer PE gewaschen wiederum 1 min zentrifugiert. Die Eluierung der Plasmid-DNA in ein sauberes Auffanggefäß erfolgte durch Zugabe von 30 - 50 µl H₂0 auf die Säule und abschließende Zentrifugation für 1 min Zur Qualitätskontroiie wurden 5 bis 10 µl dieses Ansatzes durch Restriktionsverdau und anschließende Agarosegelelektrophorese analysiert.

### Quantitative Plasmidisolierung

Größere Mengen an Plasmid DNA mit hohem Reinheitsgrad zur Durchführung von Transfektionen wurden mit Hilfe des Qiagen Plasmid Maxi Kit isoliert. Dazu wurde tagsüber eine Vorkultur angelegt, d.h. eine Bakterienkolonie wurde in 3 ml Ampizillin-haltigem LB-Medium angeimpft und für ca. sieben Stunden im Schüttelinkubator bei 37°C kultiviert. Diese Vorkultur wurde dann in 500 ml LB-Medium überführt und über Nacht bei 37°C geschüttelt. Die Bakteriensuspension wurde am nächsten Morgen mit 3000 U/min bei 4°C abzentrifugiert. Das entstehende Bakterienpellet wurde zur Plasmidisolierung gemäß den Angaben des Herstellers verwendet.

Nach der Aufnahme der DNA Pellets in sterilem H₂O wurde die Konzentration photometrisch bestimmt und die DNA Qualität durch Restriktionsverdau und anschließende Agarosegelelektrophorese überprüft.

### 2.5 Molekularbiologische Methoden

### 2.5.1 Nukleinsäuren

### Aufreinigung von DNA

Um Nukleinsäuren aus verdünnten wässrigen Lösungen anzureichern oder sie von nicht fällbaren Substanzen zu reinigen, bestehen die Möglichkeiten der Salzbildung und darauffolgende Alkoholpräzipitation oder die Reinigung mittels Silikagelsäulen (QIAGEN). Ein Vorteil der ersten Methode ist z. B. die Möglichkeit der Konzentrierung der Nukleinsäure bei der Aufnahme im Eluat nach dem Trocknen. Vorteile der Reinigung mittels Säulen ist die einfache Handhabung.

### Fällung von DNA

Zur DNA-Lösung wurden 1/10 Volumen an 3 M Natriumacetat (pH 5,2) und 3 Volumen an 100% Ethanol, Mischung zugegeben und bei -20°C mind. 4 h aufbewahrt. Anschließend wurde bei +4°C mit 13.000 x g für 30 min zentrifugiert, der Überstand verworfen und das Pellet durch Zugabe von 500 µl 70% Ethanol (-20°C) resuspendiert. Nach einem weiteren Zentrifugationsschritt für 10 min (10 min, 13.000 x g, 4°C) wurde der Überstand verworfen und das Pellet in der vorgekühlten Kühlfalle 10 min getrocknet und schließlich in einer entsprechenden Menge Wasser gelöst.

### Reinigung der PCR-Amplifikat DNA mit dem QIAGEN PCR Purification Kit

Das PCR-Gemisch wurde in 5 Volumen Puffer PB verdünnt und nach gründlicher Durchmischung auf die Säule überführt und 1 min zentrifugiert. Auf die Säule wurden 500 µl Waschpuffer PE aufgetragen und wiederum abzentrifugiert. Die gebundenen DNA-Amplifikate wurden in ein sauberes Auffanggefäß durch Zugabe von mit 30 - 50 µl Wasser auf die Säule und durch Zentrifugation eluiert.

### Aufreinigung von Nukleinsäuren aus Agarosegelen mit dem QIAGEN Gel Extraction Kit

Die Agarosegelbande mit der zu isolierenden Nukleinsäure wurde ausgeschnitten, gewogen und eine entsprechende Menge Puffer QX1 (3 Volumen des Gel-Gewichtes) zugegeben. Durch 10 min Inkubation bei 50°C wurde das Gel aufgelöst, die Lösung auf eine Säule überführt und abzentrifugiert. Nach Zugabe von 750 µl Waschpuffer PE und anschließender Zentrifugation wurde die gebundene Nukleinsäure in ein sauberes Auffanggefäß durch Zugabe von 30 - 50 µl Wasser auf die Säule und anschließende Zentrifugation eluiert.

### Isolierung und Aufreinigung von RNA

Für Expressionsanalysen mittels RT-PCR wurde RNA aus Gewebe bzw. Zellen extrahiert. Die Extraktion erfolgte entweder durch die TRlzol-Chlorophorm-Methode mit entsprechenden RNA-Isolierungskits von QIAGEN bzw. Roche.

### Extraktion der RNA mittels TRIzol Reagenz

Bei RNA-Aufreinigung aus Gewebe wurden die Gewebestücke (< 100 mg) abgewogen und nach Zugabe von 1 ml TRIzol Reagenz mit einem Glaspotter homogenisiert. Bei RNA-Aufreinigung aus kultivierten Zellen wurde 1 ml TRIzol Reagenz für 3,5 cm² Kulturschalen-Monolayer oder 1 mi TRIzol Reagenz für abzentrifugierte Suspensionskultur (5 - 10 x 10⁶ Zellen) direkt auf die Zellen gegeben und durch Auf- und Abpipettieren homogenisiert. Das TRIzol-Gemisch wurde ca. 5 min bei RT inkubiert. Nach Zugabe von 20 µl Chloroform wurde kräftig geschüttelt, weitere 5 min inkubiert und schließlich zentrifugiert (20 min, 12.000 x g, 4°C), sodass sich drei Phasen ausbilden konnten: (a) RNA in der oberen wässrigen Phase, (b) DNA in der mittleren Interphase und (c) Protein in der unteren organischen Phase.

Die wässrige Phase wurde vorsichtig, ohne die Interphase dabei zu berühren, entnommen und mit 500 µl 100% Isopropanol versetzt. Nach kurzem Vortexen erfolgte eine Inkubation für 10 min und anschließende Zentrifugation (10 min, 16000 x g, 4°C). Der Überstand wurde verworfen und das Pellet mit 1 ml 70% Ethanol (-20°C) gewaschen und erneut pelletiert (10 min, 12000 x g, 4°C). Der Überstand wurde verworfen und das Pellet in der vorgekühlten Kühlfalle getrocknet. Die RNA wurde schließlich in einem beliebigem Volumen Wasser gelöst und beim -80°C gelagert

### RNA-Extraktion mittels QIAGEN RNeasy Mini Kit

Zum Zellpellet (etwa 5 x 10⁶ Zellen) wurden 350 µl Puffer RLT gegeben und das Zellpellet mittels QIAGEN Shredder Säulen homogenisiert. Nach Zugabe von 350 µl 70% Ethanol und starkem Schütteln wurde die Suspension auf die Säule aufgetragen und zentrifugiert (30 sec bei 8.000 x g). Das Waschen der Säule erfolgte durch Zugabe von 700 µl Puffer RW1 und Zentrifugation (30 sec bei 8.000 x g). Es folgte ein zweimaliges Waschen durch Zugabe von je 500 µl Puffer RPE und Zentrifugation (30 sec, 8.000 x g). Anschließend wurde die Säule durch Zentrifugation getrocknet (2 min, 12.000 U/min). Die gebundene RNA wurde durch Zugabe von 30 - 50 µl Wasser und Zentrifugation (1 min bei 8.000 x g) eluiert.

### DNase-I-Behandlung

Um mögliche DNA-Kontaminationen der aufgereinigten RNA auszuschließen, wurde die RNA-Lösung mit DNase-I enzymatisch behandelt. Dazu wurden 50 µl RNA mit 4 µl DNase-I und 6 µl 10x Transkriptionspuffer versetzt und für 30 - 45 min bei 37°C inkubiert. Anschließend erfolgte eine RNA-Aufreinigung mittels QIAGEN RNeasy Mini Säulen.

### Restriktionsverdau

Der Restriktionsverdau dient der Überprüfung ob in einem Plasmid auch das gewünschte Insert enthalten ist. Dies kann durch den Verdau herausgeschnitten werden und anhand seiner Größe identifiziert werden. Restriktionsverdaus wurden mit Enzymen und Reaktionspuffer der Firmen GibcoBRL/Eggenstein und Boehringer/Mannheim durchgeführt. Dabei wurde 1 µg Plasmid DNA in einem Gesamtvolumen von 20 µl verdaut. Die Inkubationszeit betrug 1 bis 2 Stunden bei einer Temperatur, die dem Temperaturoptimum des Enzyms entspricht.

### Quantifizierung von Nukleinsäuren

Die Konzentrationserfassung fand mittels UV-Spektrophotometer statt. Dabei wurde die Konzentration aus der Absorption bei der spezifischen Wellenlänge l (für RNA, Oligonukleotide und DNA 260 nm) berechnet.

A= d x e x c ( A Absorption; d Schichtdicke; e Stoffkonstante; c Konzentration) Eine OD260 von 1 entspricht dabei einer Konzentration von: 50 µg/ml DNA, 40 µg/ml RNA oder 30 µg/ml Oligonukleotid. Gemessen wurde in Quarzküvetten, die zwischen den Messungen gründlich mit autoklaviertem Wasser gewaschen wurden.

### DNA-Oligonukleotidvorlagen für siRNA Herstellung

Die Oligonukleotid-Vorlagen (ONV) wurden in nukleasefreiem Wasser auf eine Endkonzentration von 200 µM verdünnt und die Absorption A bei 260 nm einer 1:250 Verdünnung gemessen. Mit Hilfe der gemessenen Absorption wird die ONV Konzentration B in µg/ml und B' in µM berechnet: $A \times 5000 = B \left[μg/ml\right]$
(5000 = 250fache Verdünnung x 20 µg Oligo / ml / Absorptionseinheit)
B [µg/ml] / 9,7 = B' [µM] $\left(29 nt \times 0,333 μg/nmol für jedes nt = 9,7 μg/nmol\right)$

### siRNA

Zur Quantifizierung der siRNA-Lösungen wurde die Absorption A bei 260 nm einer 1:25 Verdünnung gemessen. Mit Hilfe der gemessenen Absorption wird die siRNA Konzentration B in µg/ml und B' in µM berechnet: $A \times 1000 = B \left[μg/ml\right]$
(1000 = 25fache Verdünnung x 40 µg siRNA / ml / Absorptionseinheit)
B [µg/ml] / 14 = B' [µM] $\left(21 nt \times 2 Stränge = 42 nt \times 0,333 μg/nmol für jedes nt = 14 μg/nmol\right)$

### Qualitätsüberprüfung mittels Agarosegelelektrophorese

Zur Auftrennung von Plasmid DNA, restriktionsverdauter Plasmid DNA, PCR Produkten und RNA wurden 1 bis 2 prozentige TAE-Agarosegele mit 0,5 µg/ml Ethidiumbromid verwendet. Die Elektrophorese wurde in 1 x TAE Puffer (s. 2.1.3) durchgeführt. Die Proben und 1 µg Größenmarker ("100 bp DNA Leiter" "1 kb DNA Leiter") wurden mit 1/6 Volumen Probenpuffer versetzt und bei 10 V/cm aufgetrennt. Über den interkalierenden Farbstoff Ethidiumbromid konnte die DNA bzw. RNA unter UV Licht sichtbar gemacht und fotographisch dokumentiert werden. Bei der Elektrophorese von RNA wurde zur Vermeidung von Degeneration zunächst sowohl die Kammer, als auch der Kamm mit Ethanol gereinigt und der Laufpuffer mit autoklaviertem DE PC-H₂O frisch angesetzt.

### 2.5.2 Reverse Transkription Polymerasekettenreaktion (RT-PCR)

Bei der reversen Transkription wird RNA in cDNA umgeschrieben. Als Ansatzstelle der Reversen Transkriptase dienen beispielsweise Oligo(dT)15 - 18 Primer, die spezifisch an den Poly-A-Schwanz der mRNA binden. Je nach Ausgangsmaterial wurden 0,5 - 2,5 µg RNA (in 11 µl Wasser gelöst) mit 1 µl 100 nM Oligo(dT)15 - 18 Primer gemischt und 10 min bei 70°C denaturiert. Nach Abkühlung auf 4°C erfolgte die Zugabe von 4 µl 5x RT-Puffer, 2 µl 100 nM DTT, 1 µl 10 mM dNTP-Mix, 1 µl RNAsin und 1 pi Superscript™ II (200 U/µl) und eine Inkubation bei 42°C für 1 h. Die cDNA wurde bei -20°C bis zum Gebrauch gelagert.

### 2.5.3 Polymerasekettenreaktion

Mit der 1987 entwickelten Polymerasekettenreaktion (PCR) ist es möglich, *in vitro* bestimmte Nukleotidsequenzen millionenfach enzymatisch zu kopieren (Saiki et al., 1988). Dieser als Amplifikation bezeichnete Vorgang ermöglicht es, auch sehr geringe Mengen an DNA zu analysieren. Für eine PCR Reaktion (25 µl) wurde folgender Reaktionsansatz zusammengestellt:

| | | |
|---|---|---|
| 10x Puffer | 2,5 µl | (1x) |
| MgCl₂ (25 mM) | 3 µl | (3 mM) |
| dNTPs (10 mM) | 0,5 µl | (0,2 mM) |
| Primermix (2 µM) | 5 µl | (0,4 µM) |
| cDNA/RNA | 1 µl | |
| Ampli-TaqPolymerase (5 U/µl) | 0,1 µl | (0,5 U) |
| H₂O | 12,9 µl | |

Die optimale MgCl₂-Konzentration und die Anzahl der Amplifikationszyklen wurden für jedes Primerpaar einzeln ermittelt (Sequenz, Lage und Größe des Amplikons sind unter (0) dargestellt). Vor dem Start wurde für 1 min bei 94°C denaturiert und die Dauer der einzelnen Schritte eines Zyklus betrug jeweils 20 sec. Es wurde der Thermozykler PTC-200 (MJ Research) verwendet.

### Standard-PCR:

| | | |
|---|---|---|
| 95°C | 2 min | |
| 95°C | 30 sec | |
| 57°C | 30 sec | 40 x |
| 70°C | 30 sec | |
| 70°C | 5 min | |
| +4°C | ∞ | |

### 2.5.4 Ligation

Die Ligationen wurden mit der T4-DNA-Ligase (Promega) durchgeführt. Die T4 DNA Ligase ist ein Enzym, das 3'- und 5'-Enden von linearer DNA energieabhängig (ATP) miteinander kovalent verbindet. Die verbrauchte Energie ist bei ,cohesive-ended' Überhängen viel geringer als bei ,blunt-ended' Überhängen. Daher wurde bei kohäsiven Enden für 1 h bei Raumtemperatur, bei PCR-Produkten oder DNA ohne Überhänge hingegen über Nacht bei 4°C ligiert. Als Plasmid wurde der pGEM-T Vektor verwendet.

### Literaturverzeichnis

Ahlquist, P. (2002). RNA-dependent RNA polymerases, viruses, and RNA silencing. Science 296, 1270-1273.
Aihara, Y., Mashima, H., Onda, H., Hisano, S., Kasuya, H., Hori, T., Yamada, S., Tomura, H., Yamada, Y., Inoue, I., et al. (2000). Molecular cloning of a novel brain-type Na(+)-dependent inorganic phosphate cotransporter. J Neurochem 74, 2622-2625.
Amiri, A., Keiper, B. D., Kawasaki, I., Fan, Y., Kohara, Y., Rhoads, R. E., and Strome, S. (2001). An isoform of elF4E is a component of germ granules and is required for spermatogenesis in C. elegans. Development 128, 3899-3912.
Andres, K. H. (1961). Untersuchungen über den Feinbau von Spinalganglien. Z Zellforsch A55, 1-48.
Arriza, J. L., Eliasof, S., Kavanaugh, M. P., and Amara, S. G. (1997). Excitatory amino acid transporter 5, a retinal glutamate transporter coupled to a chloride conductance. Proc Natl Acad Sci U S A 94, 4155-4160.
Arriza, J. L., Fairman, W. A., Wadiche, J. I., Murdoch, G. H., Kavanaugh, M. P., and Amara, S. G. (1994). Functional comparisons of three glutamate transporter subtypes cloned from human motor cortex. J Neurosci 14, 5559-5569.
Baccei, M. L., Bardoni, R., and Fitzgerald, M. (2003). Development of nociceptive synaptic inputs to the neonatal rat dorsal horn: glutamate release by capsaicin and menthol. J Physiol 549, 231-242.
Bai, L., Xu, H., Collins, J. F., and Ghishan, F. K. (2001). Molecular and functional analysis of a novel neuronal vesicular glutamate transporter. J Biol Chem 276, 36764-36769.
Bai, L., Zhang. X., and Ghishan, F. K. (2003). Characterization of vesicular glutamate transporter in pancreatic alpha - and beta -cells and its regulation by glucose. Am J Physiol Gastrointest Liver Physiol 284, G808-814.
Banerjea, A., Li, M. J., Bauer, G., Remling, L., Lee, N. S., Rossi, J., and Akkina, R. (2003). Inhibition of HIV-1 by lentlviral vector-transduced siRNAs In T lymphocytes differentiated in SCIDhu mice and CD34+ progenitor cell-derived macrophages. Mol Ther 8, 62-71.
Baranauskas, G., and Nistri, A. (1998). Sensitization of pain pathways in the spinal cord: cellular mechanisms. Prog Neurobiol 54, 349-365.
Barton, G. M., and Medzhitov, R. (2002). Retroviral delivery of small interfering RNA into primary cells. Proc Natl Acad Sci U S A 99, 14943-14945.
Bass, B. L. (2000). Double-stranded RNA as a template for gene silencing. Cell 101, 235-238.
Bellocchio, E. E., Hu, H., Pohorille, A., Chan, J., Pickel, V. M., and Edwards, R. H. (1998). The localization of the brain-specific inorganic phosphate transporter suggests a specific presynaptic role in glutamatergic transmission. J Neurosci 18, 8643-8659.
Bellocchio, E. E., Reimer, R. J., Fremeau, R. T., Jr., and Edwards, R. H. (2000). Uptake of glutamate into synaptic vesicles by an inorganic phosphate transporter. Science 289, 957-960.
Bender, A. S., Reichelt, W., and Norenberg, M. D. (2002a). Characterization of cystine uptake in cultured astrocytes. Neurochem Int 37, 269-276.
Bender, F. L. P., Mederos y Schnitzler, M., Li, Y., Ji, A., Weihe, E., Gudermann, T., and Schafer, M. K.-H. (2002b). Expression and functional characterization of the vanilloid receptor-like TRP channel VRL-1 in the primary sensory cell line F-11. 2002 Abstract Viewer Itinerary Planner Program No. 48.23*.*
Bernstein, E., Caudy, A. A., Hammond, S. M., and Hannon, G. J. (2001). Role for a bidentate ribonuclease in the initiation step of RNA interference. Nature 409, 363-366.
Boland, L. M., and Dingledine, R. (1990). Expression of sensory neuron antigens by a dorsal root ganglion cell line, F-11. Brain Res Dev Brain Res 51, 259-266.
Bothwell, M. A., Schechter, A. L., and Vaughn, K. M. (1980). Clonal variants of PC12 pheochromocytoma cells with altered response to nerve growth factor. Cell 21, 857-866.
Brummelkamp, T. R., Bernards, R., and Agami, R. (2002). A system for stable expression of short interfering RNAs in mammalian cells. Science 296, 550-553.
Burger, P. M., Mehl, E., Cameron, P. L., Maycox, P. R., Baumert, M., Lottspeich, F., De Camilli, P., and Jahn, R. (1989). Synaptic vesicles immunoisolated from rat cerebral cortex contain high level of glutamate. Neuron 7, 287-293.
Bushman, F. (2003). RNA interference: applications in vertebrates. Mol Ther 7, 9-10.
Calderon-Martinez, D., Garavito, Z., Spinel, C., and Hurtado, H. (2002). Schwann cell-enriched cultures from adult human peripheral nerve: a technique combining short enzymatic dissociation and treatment with cytosine arabinoside (Ara-C). J N eurosci Methods 114, 1-8.
Carlton, S. M., Zhou, S., and Coggeshall, R. E. (1998). Evidence for the interaction of glutamate and NK1 receptors in the periphery. Brain Res 790, 160-169.
Carrigan, C. N., Bartlett, R. D., Esslinger, C. S., Cybulski, K. A., Tongcharoensirikul, P., Bridges, R. J., and Thompson, C. M. (2002). Synthesis and in vitro pharmacology of substituted quinoline-2,4-dicarboxylic acids as inhibitors of vesicular Glutamate transport. J Med Chem 45, 2260-2276.
Casey, B. P., and Glazer, P. M. (2001). Gene targeting via triple-helix formation. Prog Nucleic Acid Res Mol Biol 67, 163-192.
Caterina, M. J., Rosen, T. A., Tominaga, M., Brake, A. J., and Julius, D. (1999). A capsaicinreceptor homologue with a high threshold for noxious heat. Nature 398, 436-441.
Caterina, M. J., Schumacher, M. A., Tominaga, M., Rosen, T. A., Levine, J. D., and Julius, D. (1997). The capsaicin receptor: a heat-activated ion channel in the pain pathway. Nature 389, 816-824.
Chen, L., and Huang, M. L.-Y. (1992). Protein kinase C reduces Mg2+ block of NMDA-receptor channels as a mechanism of modulation. Nature 356, 521-523.
Chong, S. S., Kristjansson, K., Zoghbi, H. Y., and Hughes, M. R. (1993). Molecular cloning of the cDNA encoding a human renal sodium phosphate transport protein and its assignment to chromosome 6p21.3-p23. Genomics 18, 355-359.
Coburn, G. A., and Cullen, B. R. (2002). Potent and specific inhibition of human immunodeficiency virus type 1 replication by RNA interference. J Virol 76, 9225-9231.
Coonrod, A., Li, F. Q., and Horwitz, M. (1997). On the mechanism of DNA transfection: efficient gene transfer without viruses. Gene Ther 4, 1313-1321.
Davis, J. B., Gray, J., Gunthorpe, M. J., Hatcher, J. P., Davey, P. T., Overend, P., Harries, M. H., Latcham, J., Clapham, C., Atkinson, K., et al. (2000). Vanilloid receptor-1 is essential for inflammatory thermal hyperalgesia. Nature 405, 183-187.
Devroe, E., and Silver, P. A. (2002). Retrovirus-delivered siRNA. BMC Biotechnol 2, 15.
Disbrow, J., Gershten, M., and Ruth, J. (1982). Uptake of L-[3H]glutamic acid by crude and purified synaptic vesicles from rat brain. Biochem Biophys Res Commun 108, 1221-1227.
Doench, J. G., Petersen, C. P., and Sharp, P. A. (2003), siRNAs can function as miRNA. Genes Dev 17, 438-442.
Dykxhoorn, D. M., Novina, C. D., and Sharp, P. A. (2003). Killing the messenger: Short RNAs that silence gene expression. Nat Rev Mol Cell Biol 4, 457-467.
Eggert, C., and Fischer, U. (2003). RNA-Interferenz: Ein Werkzeug zur Analyse der Genfunktion. BIOspektrum 9, 372-274.
Elbashir, S. M., Harborth, J., Lendeckel, W., Yalcin, A., Weber, K., and Tuschl, T. (2O01a). Duplexes of 21-nucleotide RNAs mediate RNA interference in cultured mammalian cells. Nature 411, 494-498.
Elbashir, S. M., Harborth, J., Weber, K., and Tuschl, T. (2002). Analysis of gene function in somatic mammalian cells using small interfering RNAs. Methods 26, 199-213.
Elbashir, S. M., Martinez, J., Patkaniowska, A., Lendeckel, W., and Tuschl, T. (2001 b). Functional anatomy of siRNAs for mediating efficient RNAi in Drosophila melanogaster embryo lysate. Embo J 20, 6877-6888.
Erdreich-Epstein, A., and Shackleford, G. M. (1998). Differential expression of Wnt genes in normal and flat variants of PC12 cells, a cell line responsive to ectopic Wnt1 expression. Growth Factors 15, 149-158.
Fagg, G. E., and Foster, A. C. (1983). Amino acid neurotransmitters and their pathways in the mammalian brain. Neuroscience 9, 701-719.
Fairman, W. A., Vandenberg, R. J., Arriza, J. L., Kavanaugh, M. P., and Amara, S. G. (1995). An excitatory amino-acid transporter with properties of a ligend-gated chloride channel. Nature 375, 599-603.
Felgner, P. L., Gadek, T. R., Holm, M., Roman, R., Chan, H. W., Wenz, M., Northrop, J. P., Ringold, G. M., and Danielsen, M. (1987). Lipofection: a highly efficient, lipid-mediated DNAtransfection procedure. Proc Natl Acad Sci U S A 84, 7413-7417.
Fire, A., Xu, S., Montgomery, M. K., Kostas, S. A., Driver, S. E., and Mello, C. C. (1998). Potent and specific genetic interference by double-stranded RNA in Caenorhabditis elegans. Nature 391, 806-811.
Fonnum, F. (1984). Glutamate: A neurotransmitter in the mammalian brain. J Neurochem 42.
Forth, W., Henschler, D., and Rummel, W. (2001). Allgemeine und spezielle Pharmakologie und Toxikologie, 8 ed n (München - Jena, Urban & Fischer Verlag).
Francel, P. C., Harris, K., Smith, M., Fishman, M. C., Dawson, G., and Miller, R. J. (1 987). Neurochemical characteristics of a novel dorsal root ganglion X neuroblastoma hybrid cell line, F-11. J Neurochem 48, 1624-1631.
Fremeau, R. T., Jr., Burman, J., Qureshi, T., Tran, C. H., Proctor, J., Johnson, J., Zhang, H., Sulzer, D., Copenhagen, D. R., Storm-Mathisen, J., et al. (2002). The identification of vesicular glutamate transporter 3 suggests novel modes of signaling by glutamate. Proc Natl Acad Sci U S A 99, 14488-14493.
Fremeau, R. T., Jr., Troyer, M. D., Pahner, I., Nygaard, G. O., Tran, C. H., Reimer, R. J., Bellocchio, E. E., Fortin, D., Storm-Mathisen, J., and Edwards, R. H. (2001). The expression of vesicular glutamate transporters defines two classes of excitatory synapse. Neuron 31, 247-260.
Gareis, M., Harrer, P., and Bertling, W. M. (1991). Homologous recombination of exogenous DNA fragments with genomic DNA in somatic cells of mice. Cell Mol Biol 37, 191-203.
Gasnier, B. (2000). The loading of neurotransmitters into synaptic vesicles. Biochimie 82, 327-337.
Gershon, H., Ghirlando, R., Guttman, S. B., and Minsky, A. (1993). Mode of formation and structural features of DNA-cationic liposome complexes used for transfection. Biochemistry 32, 7143-7151.
Gil, J., and Esteban, M. (2000). Induction of apoptosis by the dsRNA-dependent protein kinase (PKR): mechanisms of action. Apoptosis 5.
Gitlin, L., Karelsky, S., and Andino, R. (2002). Short interfering RNA confers intracellular antiviral immunity in human cells. Nature 418, 430-434.
Gonzalez, M. P., Herrero, M. T., Vicente, S., and Oset-Gasque, M. J. (1998). Effect of glutamate receptor agonists on catecholamine secretion in bovine chromaffin cells. Neuroendocrinology 67, 181-189.
Gras, C., Herzog, E., Bellenchi, G. C., Bernard, V., Ravassard, P., Pohl, M., Gasnier, B., Giros, B., and EI Mestikawy, S. (2002). A third vesicular glutamate transporter expressed by cholinergic and serotoninergic neurons. J Neurosci 22, 5442-5451.
Greene, G. C. (1949). Gross anatomy in the rat in laboratory investigation. (Philadelphia, J. B. Lippincott Company).
Greene, L. A., and Rein, G. (1977). Release, storage and uptake of catecholamines by a clonal cell line of nerve growth factor responsive pheo-chromocytoma cells. Brain Res 129, 247-263.
Greene, L. A., and Tischler, A. S. (1976). Establishment of a noradrenergic clonal line of rat adrenal pheochromocytoma cells which respond to nerve growth factor. Proc Natl Acad Sci U S A 73, 2424-2428.
Grothe, C., and Unsicker, K. (1987). Neuron-enriched cultures of adult rat dorsal root ganglia: establishment, characterization, survival, and neuropeptide expression in response to trophic factors. J Neurosci Res 18, 539-550.
Grunweller, A., Wyszko, E., Bieber, B., Jahnel, R., Erdmann, V. A., and Kurreck, J. (2003). Comparison of different antisense strategies in mammalian cells using locked nucleic acids, 2'-O-methyl RNA, phosphorothioates and small interfering RNA. Nucleic Acids Res 31, 3185-3193.
Grynfeld, A., Pahlman, S., and Axelson, H. (2000). Induced neuroblastoma cell differentiation, associated with transient HES-1 activity and reduced HASH-1 expression, is inhibited by Notch1. Int J Cancer 88, 401-410.
Guerrero, I., Pellicer, A., and Burstein, D. E. (1988). Dissociation of c-fos from ODC expression and neuronal differentiation in a PC12 subline stably transfected with an inducible N-ras oncogene. Biochem Biophys Res Commun 150,1185-1192.
Guerrero, I., Wong, H., Pellicer, A., and Burstein, D. E. (1986). Activated N-ras gene induces neuronal differentiation of PC12 rat pheochromocytoma cells. J Cell Physiol 129, 71-76.
Guo, S., and Kemphues, K. J. (1995). par-1, a gene required for establishing polarity in C. elegans embryos, encodes a putative Ser/Thr kinase that is asymmetrically distributed. Cell 81, 61 1-620.
Hammond, S. M., Bernstein, E., Beach, D., and Hannon, G. J. (2000). An RNA-directed nuclease mediates posttranscriptional gene silencing in Drosophila cells. Nature 404, 293-296.
Hayashi, M., Otsuka, M., Morimoto, R., Hirota, S., Yatsushiro, S., Takeda, J., Yamamoto, A., and Moriyama, Y. (2001). Differentiation-associated Na+-dependent inorganic phosphate cotransporter (DNPI) is a vesicular glutamate transporter in endocrine glutamatergic system s. J Biol Chem 276, 43400-43406.
Hayashi, M., Yamada, H., Uehara, S., Morimoto, R., Muroyama, A., Yatsushiro, S., Takeda, J., Yamamoto, A., and Moriyama, Y. (2003). Secretory granule-mediated co-secretion of L-glutamate and glucagon triggers glutamatergic signal transmission in islets of Langerhans. J Biol Chem 278, 1966-1974.
Hemann, M. T., Fridman, J. S., Zilfou, J. T., Hernando, E., Paddison, P. J., Cordon-Cardo, C., Hannon, G. J., and Lowe, S. W. (2003). An epi-allelic series of p53 hypomorphs created by stable RNAi produces distinct tumor phenotypes in vivo. Nat Genet 33, 396-400.
Herzog, E., Bellenchi, G. C., Gras, C., Bernard, V., Ravassard, P., Bedet, C., Gasnier, B., Giros, B., and EI Mestikawy, S. (2001). The existence of a second vesicular glutamate transporter specifies subpopulations of glutamatergic neurons. J Neurosci 21, RC181.
Hioki, H., Fujiyama, F., Taki, K., Tomioka, R., Furuta, T., Tamamaki, N., and Kaneko, T. (2003). Differential distribution of vesicular glutamate transporters in the rat cerebellar cortex. Neuroscience 117, 1-6.
Hisano, S., Sawada, K., Kawano, M., Kanemoto, M., Xiong, G., Mogi, K., Sakata-Haga, H., Takeda, J., Fukui, Y., and Nogami, H. (2002). Expression of inorganic phosphatelvesicular glutamate transporters (BNPI/VGLUT1 and DNPI/VGLUT2) in the cerebellum and precerebellar nuclei of the rat. Brain Res Mol Brain Res 107, 23-31.
Holen, T., Amarzguioui, M., Wiiger, M. T., Babaie, E., and Prydz, H. (2002). Positional effects of short interfering RNAs targeting the human coagulation trigger Tissue Factor. Nucleic Acids Res 30, 1757-1766.
Hu, W. Y., Myers, C. P., Kilzer, J. M., Pfaff, S. L., and Bushman, F. D. (2002). Inhibition of retroviral pathogenesis by RNA interference. Curr Biol 12, 1301-1311.
Huber, K., Brühl, B., Guillemont, F., Olson, E. N., Ernsberger, U., and Unsicker, K. (2002a). Development of chromaffin cells depends on MASH1 function. Development 129, 4729-4738.
Huber, K., Combs, S., Ernsberger, U., Kalcheim, C., and Unsicker, K. (2002b). Generation of neuroendocrine chromaffin cells from sympathoadrenal preogenitors: beyond the glucocorticoid hypothesis. Ann N Y Acad Sci 971, 554-549.
Hunt, S. P., and Mantyh, P. W. (2001). The molecular dynamics of pain control. Nat Rev Neuroscience 2, 83-91.
Israel, M., Tomasi, M., Bostel, S., and Meunier, F. M. (2001). Cellular resistance to Evans blue toxicity involves an up-regulation of a phosphate transporter implicated in vesicular glutamate storage. J Neurochem 78, 658-663.
Issack, P. S., and Ziff, E. B. (1998a). Altered expression of helix-loop-helix transcriptional regulators and cyclin D1 in Wnt-1-transformed PC12 cells. Cell Growth Differ 9, 837-845.
Issack, P. S., and Ziff, E. B. (1998b). Genetic elements regulating HES-1 induction in Wnt-1-transformed PC12 cells. Cell Growth Differ 9, 827-836.
Jacque, J. M., Triques, K., and Stevenson, M. (2002). Modulation of HIV-1 replication by RNA interference. Nature 418, 435-438.
Jahn, R., and Südhof, T. C. (1993). Synaptic vesicle traffic: rush hour in the nerve terminal. J Neurochem 61, 12-21.
Jimenez, A. L., Chou, A. H., Khadadadi, O., Palos, T. P., and Howard, B. D. (2003). Wnt-1 has multiple effects on the expression of glutamate transporters. Neurochem Int 42, 345-351.
Jogi, A., Persson, P., Grynfeld, A., Pahlman, S., and Axelson, H. (2002). Modulation of basic helix-loop-helix transcription complex formation by Id proteins during neuronal differentiation. J Biol Chem 277, 9118-9126.
Jue, S. F., Bradlev. R. S., Rudnicki, J. A., Varmus, H. E., and Brown, A. M. (1992). The mouse Wnt-1 gene can act via paracrine mechanism in transformation of mammary epithelial cells. Mol Cell Biol 12, 321-328.
Kamath, R. S., Fraser, A. G., Dong, Y., Poulin, G., Durbin, R., Gotta, M., Kanapin, A., Le Bot, N., Moreno, S., Sohrmann, M., et al. (2003). Systematic functional analysis of the Caenorhabditis elegans genome using RNAi. Nature 421, 231-237.
Kanai, Y., and Hediger, M. A. (1992). Primary structure and functional characterization of a highaffinity glutamate transporter. Nature 360, 467-471-
Kaneko, T., and Fujiyama, F. (2002). Complementary distribution of vesicular glutamate transporters in the central nervous system. Neurosci Res 42, 243-250.
Kapadia, S. B., Brideau-Andersen, A., and Chisari, F. V. (2003). Interference of hepatitis C virus RNA replication by short interfering RNAs. Proc Natl Acad Sci U S A 100, 2014-2018.
Katoh-Semba, R., Oohira, A., Sano, M., Watanabe, K., Kitajima, S., and Kashiwamata, S. (1989). Glycosaminoglycan composition of PC12 pheochromocytoma cells: a comparison with PC12D cells, a new subline of PC12 cells. J Neurochem 52, 889-895.
Keck, J. L., Roche, D. D., Lynch, A. S., and Berger, J. M. (2000). Structure of the RNA polymerase domain of E. coli primase. Science 287, 2482-2486.
Kennerdell, J. R., and Carthew, R. W. (2000). Heritable gene silencing in Drosophila using double-stranded RNA. Nat Biotechnol 18, 896-898.
Kish, P. E., Fischer-Bovenkerk, C., and Ueda, T. (1989). Active transport of GABA and glycin into synaptic vesicles. Proc Natl Acad Sci U S A 86, 3877-3881.
Kobayashi, S., and Millhorn, D. E. (2001). Hypoxia regulates glutamate metabolism and membrane transport in rat PC12 cells. J Neurochem 76, 1935-1948.
Lee, N. S., Dohjima, T., Bauer, G., Li, H., Li, M. J., Ehsan i, A., Salvaterra, P., and Rossi, J. (2002). Expression of small interfering RNAs targeted against HIV-1 rev transcripts in human cells. Nat Biotechnol 20, 500-505.
Lee, R. Y., Sawin, E. R., Chalfie, M., Horvitz, H. R., and Avery, L. (1999). EAT-4, a homolog of a mammalian sodium-dependent inorganic phosphate cotransporter, is necessary for glutamatergic neurotransmission in caenorhabditis elegans J Neurosci 19, 159-167.
Levy, L. M., Warr, O., and Attwell, D. (1998). Stoichiometry of the glial glutamate transporter GLT-1 expressed inducibly in a Chinese hamster ovary cell line selected for low endogenous Na+-dependent glutamate uptake. J Neurosci 18, 9620-9628.
Li, H., Li, W. X., and Ding, S. W. (2002). Induction and suppression of RNA silencing by an animal virus. Science 296, 1319-1321.
Li, J. L., Fujiyama, F., Kaneko, T., and Mizuno, N. (2003a). Expression of vesicular glutamate transporters, VGluT1 and VGluT2, in axon terminals of nociceptive primary afferent fibers in the superficial layers of the medullary and spinal dorsal horns of the rat. J Comp Neurol 457, 236-249.
Li, J. L., Xiong, K. H., Dong, Y. L., Fujiyama, F., Kaneko, T., and Mizuno, N. (2003b). Vesicular glutamate transporters, VGluT1 and VGluT2, in the trigeminal ganglion neurons of the rat, with special reference to coexpression. J Comp Neurol 463, 212-220.
Lindbo, J. A., and Dougherty, W. G. (1992). Untranslatable transcripts of the tobacco etch virus coat protein gene sequence can interfere with tobacco etch virus replication in transgenic plants and protoplasts. Virology 189, 725-733.
Luqmani, Y. A. (1981). Nucleotide uptake by isolated cholinergic synaptic vesicles: Evidence for a carrier of adenosine 5'-triphosphate. Neuroscience 6, 1011-1021.
Mackenzie, B., and Erickson, J. (2003). Sodium-coupled neutral amino acid (System N/A) transporters of the SLC38 family. Pflugers Arch.
Macnaughton, T. B., Shi, S. T., Modahl, L. E., and Lai, M. M. (2002). Rolling circle replication of hepatitis delta virus RNA is carried out by two different cellular RNA polymerases. J Virol 76, 3920-3927.
Manfras, B. J., and Boehm, B. O. (1995). Expression of a glutamate transporter cDNA in human pancreatic islets. Exp Clin Endocrinol Diabetes 103 Suppl 2, 95-98.
Masson, J., Sagne, C., Hamon, M., and EI Mestikawy, S. (1999). Neurotransmitter transporters in the central nervous system. Pharmacol Rev 51, 439-464.
McIntire, S. L., Reimer, R. J., Schuske, K., Edwards, R. H., and Jorgensen, E. M. (1997). Identification and characterization of the vesicular GABA transporter. Nature 389, 870-876.
McMahon, A. P., and Bradley, A. (1990). The Wnt-1 (Int-1) proto-oncogen is required for development of brain. Cell 62, 1071-1085.
McManus, M. T., and Sharp, P. A. (2002). Gene silencing in mammals by small interfering RNAs. Nat Rev Genet 3, 737-747.
Mertz, K. D., Weisheit, G., Schilling, K., and Luers, G. H. (2002). Electroporation of primary neural cultures: a simple method for directed gene transfer in vitro. Histochem Cell Biol 118, 501-506.
Miyagishi, M., and Taira, K. (2002). U6 promoter-driven siRNAs with four uridine 3' overhangs efficiently suppress targeted gene expression in mammalian cells. Nat Biotechnol 20, 497-500.
Moraleda, G., and Taylor, J. (2001). Host RNA polymerase requirements for transcription of the human hepatitis delta virus genome. J Virol 75, 10161-101 69.
Myslinski, E. (2001). An unusually compact external promotor for RNA polymerase III transcription of the human H1 RNA gene. Nucleic Acids Res 29, 2502-2509.
Naito, S., and Ueda, T. (1985). Characterization of glutamate uptake into synaptic vesicles. J Neurochem 44, 99-109.
Napoli, C., Lemieux, C., and Jorgensen, R. (1990). Introduction of a Chimeric Chalcone Synthase Gene into Petunia Results in Reversible Co-Suppression of Homologous Genes in trans. Plant Cell 2, 279-289.
Nestler, E. J., Hyman, S. E., and Malenka, R. C. (2001). Molecular Neuropharmacology, 1 edn, McGraw-Hill Companies).
Ni, B., Du, Y., Wu, X., DeHoff, B. S., Rosteck, P. R., Jr., and Paul, S. M. (1996). Molecular cloning, expression, and chromosomal localization of a human brain-specific Na(+)-dependent inorganic phosphate cotransporter. J Neurochem 66, 2227-2238.
Ni, B., Rosteck, P. R., Jr., Nadi, N. S., and Paul, S. M. (1994). Cloning and expression of a cDNA encoding a brain-specific Na(+)-dependent inorganic phosphate cotransporter. Proc Natl Acad Sci U S A 91, 5607-5611.
Ni, B., Wu, X., Yan, G. M., Wang, J., and Paul, S. M. (1995). Regional expression and cellular localization of the Na(+)-dependent inorganic phosphate cotransporter of rat brain. J Neurosci 15, 5789-5799.
Njus, D., Kelley, P. M., and Hardabek, G. J. (1986). Bioenegetics of secretory vesicles. Biochim Biophys Acta 853, 237-265.
Noda, M., Ko, M., Ogura, A., Liu, D. G., Amano, T., Takano, T., a nd Ikawa, Y. (1985). Sarcoma viruses carrying ras oncogenes induce differentiation-associated properties in a neuronal cell line. Nature 318, 73-75.
Novina, C. D., Murray, M. F., Dykxhoorn, D. M., Beresford, P. J., Riess, J., Lee, S. K., Collman, R. G., Lieberman, J., Shankar, P., and Sharp, P. A. (2002). siRNA-directed inhibition of HIV-1 infection. Nat Med 8, 681-686.
Oliveira, A. C. R., Hydling, F., Olsson, E., Shi, T., Edwards, R. H., Fujiyama, F., Kaneko, T., Hökfelt, T., Cullheim, S., and Meister, B. (2003). Cellular Localization of Three Vesikular Glutamate Transporter mRNAs and Proteins in Rat Spinal Cord and Dorsal Root Ganglia. Synapse 50, 117-129.
Oset-Gasque, M. J., Castro, E., and Gonzalez, A. M. (1990). Mechanisms of 3H-GABA release by chromaffin cells in primary culture. J Neurosci Res 26, 181-187.
Ottersen, O. P., and Storm-Mathisen, J. (1984). Neurons containing or accumulating transmitter amino acids. In Handbook of Chemical Neuroanatomy, A. Björklund, H. T., and M. J. Kuhar, eds. (Amsterdam, Elsevier), pp. 141-246.
Ozkan, E. D., and Ueda, T. (1998). Glutamate transport and storage in synaptic vesicles. Jpn J Pharmacol 77, 1-10.
Paddison, P. J., and Hannon, G. J. (2002). RNA interference: the new somatic cell genetics? Cancer Cell 2, 17-23.
Palauqui, J. C. e. a. (1997). Systemic acquired silencing: transgenespecific post-transcriptional silencing is transmitted by grafting from silenced stocks to non-silenced scions. Embo J 16, 4738-4745.
Patapoutian, A., Peier, A. M., Story, G. M., and Viswanath, V. (2003). ThermoTRP channnels and beyond: mechanisms of temperature sensation. Nat Rev Neuroscience 4, 529-539.
Patapoutian, A., and Reichardt, L. F. (2001). Trk receptors: mediators of neurotrophin action. Curr Opin Neurobiol 11, 272-280.
Pines, G., Danbolt, N. C., Bjoras, M., Zhang, Y., Bendahan, A., Eide, L., Koepsell, H., Storm-Mathisen, J., Seeberg, E., and Kanner, B. I. (1992). Cloning and expression of a rat brain L-glutamate transporter. Nature 360, 464-467.
Plasterk, R. H. (2002). RNA silencing: the genome's immune system. Science 296, 1263-1265.
Platika, D., Baizer, L., and Fishman, M. C. (1985). Sensory neurons "immortalized" by fusion with neuroblastoma cells. Trans Assoc Am Physicians 98, 301-304.
Qin, X. F., An, D. S., Chen, I. S., and Baltimore, D. (2003). Inhibiting HIV-1 infection in human T cells by lentiviral-mediated delivery of small interfering RNA against CCR5. Proc Natl Acad Sci U S A 100, 183-188.
Ramachandran, B., Houben, K., Rozenberg, Y. Y., Haigh, J. R., Varpetian, A., and Howard, B. D. (1993). Differential expression of transporters for norepinephrine and glutamate in wild type, variant, and WNT1-expressing PC12 cells. J Biol Chem 268, 23891-23897.
Reimer, R. J., and Edwards, R. H. (2003). Organic anion transport is the primary function of the SLC17/type I phosphate transporter family. Pflugers Arch.
Ren, K. (1994). Wind up and the NMDA-receptor: from animal studies to humans. Pain 59, 157-158.
Romero, O., Figueroa, S., Vicente, S., Gonzalez, M. P., and Oset-Gasque, M. J. (2003). Molecular mechanisms of glutamate release by bovine chromaffin cells in primary culture. Neuroscience 116, 817-829.
Roseth, S., Fykse, E. M., and Fonnum, F. (1995). Uptake of L-glutamate into rat brain synaptic vesicles: effect of inhibitors that bind specifically to the glutamate transporter. J Neurochem 65, 96-103.
Roseth, S., Fykse, E. M., and Fonnum, F. (1998). Uptake of L-glutamate into synaptic vesicles: competitive inhibition by dyes with biphenyl and amino- and sulphonic acid-substituted naphthyl groups. Biochem Pharmacol 56, 1243-1249.
Rothstein, J. D., Dykes-Hoberg, M., Pardo, C. A., Bristol, L. A., Jin, L., Kuncl, R. W., Kanai, Y., Hediger, M. A., Wang, Y., Schielke, J. P., and Welty, D. F. (1996). Knockout of glutamate transporters reveals a major role for astroglial transport in excitotoxicity and clearance of glutamate. Neuron 16, 675-686.
Rozenberg, Y. Y., and Howard, B. D. (1994). Contrasting morphological changes in PC12 flat cells expressing two different forms of exogenous encogenic ras. Exp Cell Res 211, 59-67.
Rubinson, D. A., Dillon, C. P., Kwiatkowski, A. V., Sievers, C., Yang, L., Kopinja, J., Rooney, D. L., Ihrig, M. M., McManus, M. T., Gertler, F. B., et al. (2003). A lentivirus-based system to functionally silence genes in primary mammalian cells, stem cells and transgenic mice by RNA interference. Nat Genet 33, 401-406.
Sagne, C., El Mestikawy, S., Isambert, M.-F., Hamon, M., Henry, J.-P., Giros, B., and Gasnier, B. (1997). Cloning of a functional GABA and glycine transporter by screening of genome databases. FEBS Lett 417, 177-183.
Saiki, R. K., Gelfand, D. H., Stoffel, S., Scharf, S. J., Higuchi, R., Horn, G. T., Mullis, K. B., and Erlich, H. A. (1988). Primer-directed enzymatic amplification of DNA with a thermostable DNA polymerase. Science 239, 487-491.
Sassone-Corsi, P., Der, C. J., and Verma, I. M. (1989). ras-induced differentiation of PC12 cells: possible involment of fos and jun. Mol Cell Biol 9, 3174-3183.
Schafer, M. K., Varoqui, H., Defamie, N., Weihe, E., and Erickson, J. D. (2002). Molecular cloning and functional identification of mouse vesicular glutamate transporter 3 and its expression in subsets of novel excitatory neurons. J Biol Chem 277, 50734-50748.
Scholz, J., and Woolf, C. J. (2002). Can we conquer pain? Nat Neurosci 5, 1062-1067.
Schwarz, D. S., Hutvagner, G., Haley, B., and Zamore, P. D. (2002). Evidence that siRNAs function as guides, not primers, in the Drosophila and human RNAi pathways. Mol Cell 10, 537-548.
Shackleford, G. M., Willert, K., Wang, J., and Varmus, H. E. (1993). The Wnt-1 proto-oncogene induces changes in morphology, gene expression and growth factor responsivness in PC12 cells. Neuron 11, 865-875.
Shen, C., Buck, A. K., Liu, X., Winkler, M., and Reske, S. N. (2003). Gene silencing by adenovirusdelivered siRNA. FEBS Lett 539, 111-114.
Shi, Y. (2003). Mammalian RNAi for the masses. Trends Genet 19, 9-12.
Shioi, J., Naito, S., and Ueda, T. (1989). Glutamate uptake into synaptic vesicles from bovine cerebral cortex and electrochemical potential difference of proton across the membrane. Biochem J 258, 449-504.
Shuey, D. J., McCallus, D. E., and Giordano, T. (2002). RNAi: gene-silencing in therapeutic intervention. Drug Discov Today 7, 1040-1046.
Sijen, T., Fleenor, J., Simmer, F., Thijssen, K. L., Parrish, S., Timmons, L., Plasterk, R. H., and Fire, A. (2001). On the role of RNA amplification in dsRNA-triggered gene silencing. Cell 107, 465-476.
Sindrup, S. H., and Jensen, T. S. (1999). Efficiacy of pharmacological treatments of neuropathic pain: an update and effect related to mechanism of drug action. Pain 83, 389-400.
Skerry, T. M., and Genever, P. G. (2001). Glutamate signalling in non-neuronal tissues. Trends Pharmacol Sci 22, 174-181.
Smith, J. G., Walzem, R. L., and German, J. B. (1993). Liposomes as agents of DNA transfer. Biochim Biophys Acta 1154, 327-340.
Snider, W. D., and McMahon, S. B. (1998). Tackling pain at the source: new ideas about nociceptors. Neuron 20, 629-632.
Stark, G. (1998). How cells respond to interferons. Annu Rev Biochem 67, 227-264.
Stewart, S. A., Dykxhoorn, D. M., Paiiiser, D., Mizuno, H., Yu, E. Y., An, D. S., Sabatini, D. M., Chen, I. S., Hahn, W. C., Sharp, P. A., et al. (2003). Lentivirus-delivered stable gene silencing by RNAi in primary cells. Rna 9, 493-501.
Storck, T., Schulte, S., Hofmann, K., and Stoffel, W. (1992). Structure, expression, and functional analysis of a Na(+)-dependent glutamate/aspartate transporter from rat brain. Proc Natl Acad Sci U S A 89, 10955-10959.
Stucky, C. L., and Lewin, G. R. (1999). Isolectin B(4)-positive and -negative nociceptors are functionally distinct. J Neurosci 19, 6497-6505.
Sung, B., Lim, G., and Mao, J. (2003). Altered expression and uptake activity of spinal glutamate transporters after nerve injury contribute to the pathogenesis of neuropathic pain in rats. J Neurosci 23, 2899-2910.
Tabb, J., Kish, P., Vandyke, R., and Ueda, T. (1992). Glutamate transport into synaptic vesicles - role of membrane potential, pH gradient and intravesicular pH. J Biol Chem 267, 15412-15418.
Takamori, S., Malherbe, P., Broger, C., and Jahn, R. (2002). Molecular cloning and functional characterization of human vesicular glutamate transporter 3. EMBO Rep 3, 798-803.
Takamori, S., Rhee, J. S., Rosenmund, C., and Jahn, R. (2000). Identification of a vesicular glutamate transporter that defines a glutamatergic phenotype in neurons. Nature 407, 189-194.
Tijsterman, M., Ketting, R. F., Okihara, K. L., Sijen, T., and Plasterk, R. H. (2002). RNA helicase MUT-14-dependent gene silencing triggered in C. elegans by short antisense RNAs. Science 295, 694-697.
Timmons, L., Court, D. L., and Fire, A. (2001). Ingestion of bacterially expressed dsRNAs can produce specific and potent genetic interference in Caenorhabditis elegans. Gene 263, 103-112.
Todd, A. J., Hughes, D. I., Polgar, E., Nagy, G. G., Mackie, M., Ottersen, O. P., and Maxwell, D. J. (2003). The expression of vesicular glutamate transporters VGLUT1 and VGLUT2 in neurochemically defined axonal populations in the rat spinal cord with emphasis on the dorsal horn. Eur J Neurosci 17, 13-27.
Toll, L., and Howard, D. B. (1980). Evidence that an ATPase and a proton-motive force function in the transport of acetylcholine. J Biol Chem 255, 1787-1789.
Tölle, T. R. (1997). Chronischer Schmerz. In Klinische Neurobiologie: molekulare Pathogenese und Therapie von neurologischen Erkrankungen, T. Herdegen, T. R. Tölle, and M. Bähr, eds. (Heidelberg, Spektrum Akademischer Verlag), pp. 307-336.
Tominaga, M., Caterina, M. J., Malmberg, A. B., Rosen, T. A., Gilbert, H., Skinner, K., Raumann, B. E., Basbaum, A. I., and Julius, D. (1998). The cloned capsaicin receptor integrates multiple pain-producing stimuli. Neuron 21, 531-543.
Tuschl, T. (2002). Expanding small RNA interference. Nat Biotechnol 20, 446-448.
Vaistij, F. E., Jones, L., and Baulcombe, D. C. (2002). Spreading of RNA targeting and DNA methylation in RNA silencing requires transcription of the target gene and a putative RNA-dependent RNA polymerase. Plant Cell 14, 857-867.
Vandenberg, R. J. (1998). Molecular pharmacology and physiology of glutamate transporters in the central nervous system. Clin Exp Pharmacol Physiol 25, 393-400.
Varoqui, H., Schafer, M. K., Zhu, H., Weihe, E., and Erickson, J. D. (2002). Identification of the differentiation-associated Na+/PI transporter as a novel vesicular glutamate transporter expressed in a distinct set of glutamatergic synapses. J Neurosci 22, 142-155.
Wall, P. D., and Melzack, R. (1999). Textbook of pain (Edinburgh, Churchill Livingstone).
Wesley, S. V., Helliwell, C. A., Smith, N. A., Wang, M. B., Rouse, D. T., Liu, Q., Gooding, P. S., Singh, S. P., Abbott, D., Stoutjesdijk, P. A., et al. (2001). Construct design for efficient, effective and high-throughput gene silencing in plants. Plant J 27, 581-590.
Willis, W. D. (2001). Role of neurotransmitters in sensitization of pain responses. Ann N Y Acad Sci 933, 142-156.
Winston, W. M., Molodowitch, C., and Hunter, C. P. (2002). Systemic RNAi in C. elegans requires the putative transmembrane protein SID-1. Science 295, 2456-2459.
Wood, P. M. (1976). Separation of functional Schwann cells and neurons from normal peripheral nerve tissue. Brain Res 115, 361-375.
Yu, J. Y., DeRuiter, S. L., and Turner, D. L. (2002). RNA interference by expression of shortinterfering RNAs and hairpin RNAs in mammalian cells. Proc Natl Acad Sci U S A 99, 6047-6052.
Zamore, P. D., Tuschl, T., Sharp, P. A., and Bartel, D. P. (2000). RNAi: double-stranded RNA directs the ATP-dependent cleavage of mRNA at 21 to 23 nucleotide intervals. Cell 101, 25-33.
Zerangue, N., and Kavanaugh, M. P. (1996). Flux coupling in a neuronal glutamate transporter. Nature 383, 634-637.
Zheng, S., Ramachandran, B., Haigh, J. R., Palos, T. P., Steger, K., and Howard, B. D. (1996). The induction of ret by Wnt-1 in PC12 cells is atypically dependent on continual Wnt-1 expression. Oncogene 12, 555-562.
Zhuo, M. (2001). No pain, no gains. SclentificWorldJournal 1, 204-206.

**Konkordanztabelle**

| **SEQ ID NO:** | **Support** |
|---|---|
| 1-9 | S.6 und S. 7 |
| 10-13 | S.9 |
| 14-51 | Figur 26 |
| 52-69 | S. 12 |
| 70-72 | S. 17 |
| 73-83 | S. 36 |
| 84 | S.37 |
| 85 | S. 18 |
| 86-87 | S. 18 und S. 48 |
| 88-91 | S. 48 |
| 92-101 | S. 56 |
| 102-103 | S. 57 |
| 104 | Fig. 5 |
| 105 | Fig. 6 |
| 106 | Fig. 7 |
| 107 | Fig. 27a |
| 108 | Fig. 27b |
| 109 | Fig. 27c |
| 110 | Anspruch 21 |
| 111 | Anspruch 22 |
| 112 | Anspruch 22 |

## Patentansprüche

1. VGLUT-spezifische si-RNA-Moleküle mit einer Sequenz ausgewählt aus und

2. Isolierte Zelle, enthaltend mindestens ein si-RNA-Molekül nach Anspruch 1, ausgenommen eine humane embryonale Stammzelle.

3. Arzneimittel, enthaltend mindestens ein si-RNA-Molekül nach Anspruch 1 und/oder mindestens eine Zelle nach Anspruch 2 sowie gegebenenfalls geeignete Hilfs- und/oder Zusatzstoffe.

4. Diagnostikum, enthaltend mindestens ein si-RNA-Molekül nach Anspruch 1 und/oder mindestens eine Zelle gemäß Anspruch 2 sowie gegebenenfalls geeignete Zusatzstoffe.

5. Verwendung eines si-RNA-Moleküls nach Anspruch 1 und/oder einer Zelle gemäß Anspruch 2 zur Herstellung eines Arzneimittels bzw. eines Schmerzmittels zur Behandlung von Schmerz, insbesondere von chronischem Schmerz, taktiler Allodynie, thermisch ausgelöstem Schmerz und/oder Entzündungsschmerz.

6. Verwendung eines si-RNA-Moleküls nach Anspruch 1 und/oder einer Zelle nach Anspruch 2 zur Herstellung eines Arzneimittels zur Behandlung von Harninkontinenz, von neurogenem Blasensymptom, Pruritus, Tumoren, Entzündungen, Asthma.

7. si-RNA-Molekül nach Anspruch 1 und/oder Zelle gemäß Anspruch 2 zur Verwendung in der Gentherapie, vorzugsweise in der *in-vivo*- oder *in-vitro*-Gentherapie.

8. *In-vitro*-Verfahren zur Hemmung der Expression mindestens eines VGLUT-Familienmitglieds in einer Zelle, wobei ein si-RNA-Molekül nach Anspruch 1 in die Zelle eingeführt wird.

9. Verfahren nach Anspruch 8, wobei das si-RNA-Molekül in micellare Strukturen, vorzugsweise Liposomen, eingeschlossen wird.

10. Verfahren nach einem der Ansprüche 8 oder 9, wobei das si-RNA-Molekül in virale natürliche Kapside oder in auf chemischem oder enzymatischem Weg hergestellte Kapside oder davon abgeleitete Strukturen eingeschlossen wird.

## Claims

1. VGLUT-specific si-RNA-molecules having a sequence selected from and

2. Isolated cell comprising at least one si-RNA-molecule according to claim 1, except for a human embryonic stem cell.

3. Medicament comprising at least one si-RNA-molecule according to claim 1 and/or at least one cell according to claim 2 as well as appropriate excipients and/or additives, if necessary.

4. Diagnostic aid comprising at least one si-RNA-molecule according to claim 1 and/or at least one cell according to claim 2 as well as appropriate additives, if necessary.

5. Use of an si-RNA-molecule according to claim 1 and/or a cell according to claim 2 for producing a medicament and analgesic, respectively, for treating pain, in particular chronic pain, tactile allodynia, thermally induced pain and/or inflammatory pain.

6. Use of an si-RNA-molecule according to claim 1 and/or a cell according to claim 2 for producing a medicament for treating urinary incontinence, neurogenic bladder symptom, pruritus, tumors, inflammations, asthma.

7. Si-RNA-molecule according to claim 1 and/or a cell according to claim 2 for use in gene therapy, preferably for in-vivo or in-vitro gene therapy.

8. In-vitro process for inhibiting the expression of at least one VGLUT family member in a cell, wherein an si-RNA-molecule according to claim 1 is introduced into the cell.

9. The process according to claim 8, wherein the si-RNA-molecule is enclosed into micellar structures, preferably liposomes.

10. The process according to any of claims 8 or 9, wherein the si-RNA-molecule is enclosed into viral natural capsides or into chemically or enzymatically produced capsides or into structures derived therefrom.

## Revendications

1. Molécules de siARN spécifiques de VGLUT choisies parmi et

2. Cellule isolée contenant au moins une molécule de siARN selon la revendication 1 à l'exception d'une cellule souche humaine embryonnaire.

3. Médicament comprenant au moins une molécule de siARN selon la revendication 1 et/ou au moins une cellule selon la revendication 2 et le cas échéant des excipients et/ou additifs appropriés.

4. Agent diagnostique contenant au moins une molécule de siARN selon la revendication 1 et/ou au moins une cellule selon la revendication 2 et le cas échéant des additifs appropriés.

5. Utilisation d'une molécule de siARN selon la revendication 1 et/ou d'une cellule selon la revendication 2 pour la fabrication d'un médicament respectivement d'un antalgique destiné au traitement de la douleur en particulier de la douleur chronique, de l'allodynie tactile, de la douleur induite par chaleur et/ou de la douleur d'inflammation.

6. Utilisation d'une molécule de siARN selon la revendication 1 et/ou d'une cellule selon la revendication 2 pour la fabrication d'un médicament destiné au traitement de l'incontinence urinaire, des symptômes de la vessie neurogène, du prurit, des tumeurs, des inflammations, de l'asthme.

7. Molécule de siARN selon la revendication 1 et/ou la cellule selon la revendication 2 pour l'utilisation dans la génothérapie en particulier dans la génothérapie in vivo ou in vitro.

8. Procédé in vitro pour l'inhibition de l'expression d' au moins d'un membre de la famille de VGLUT dans une cellule dans lequel une molécule de siARN selon la revendication 1 est introduite dans la cellule.

9. Procédé selon la revendication 8, dans lequel la molécule de siARN est incluse dans des structures micellaires en particulier des liposomes.

10. Procédé selon l'une quelconque des revendications 8 ou 9, dans lequel la molécule de siARN est incluse dans capsides naturelles virales ou dans des capsides fabriquées par voie chimique ou enzymatique ou dans des structures dérivées de celles-ci.
